# EUROPEAN PATENT SPECIFICATION

(11) **EP 3 755 355 B1**
(45) Date of publication and mention of the grant of the patent: **07.10.2026**
(21) Application number: 19757891.7
(22) Date of filing: 20.02.2019
(51) Int. Cl.: A61K 35/545, A61K 35/15, A61K 35/17, A61K 35/28, A61P 31/12, A61P 37/04, C07D 487/04, C12N 5/071, C12N 5/0783, C12N 5/0784

(54) **EXPANSION OF NK AND DC CELLS IN VIVO MEDIATING IMMUNE RESPONSE**
IN-VIVO-EXPANSION VON NK- UND DC-ZELLEN ZUR VERMITTLUNG EINER IMMUNANTWORT
EXPANSION DE CELLULES NK ET DC IN VIVO MÉDIANT UNE RÉPONSE IMMUNITAIRE

(30) Priority: 20.02.2018 US 201862632733 P
(43) Date of publication of application: 30.12.2020
(73) Proprietor: UNIVERSITE DE MONTREAL, Montréal, Québec H3T 1J4 (CA)
(72) Inventor: SAUVAGEAU, Guy, Québec H3C 3J7 (CA); COHEN, Sandra, Montreal QC H3Y 2B9 (CA); ROY, Jean, Québec H3C 3J7 (CA); LACHANCE, Silvy, Québec H3C 3J7 (CA); DELISLE, Jean-Sébastien, Saint-Lambert QC J4P 3B3 (CA); CHAGRAOUI, Jalila, Québec H3C 3J7 (CA)
(74) Representative: Hoffmann Eitle
(86) International application number: PCT/CA2019/050208
(87) International publication number: WO 2019/161494

(56) References cited:
- WO-A1-2013/110198
- WO-A1-2016/109661
- WO-A1-2017/205977
- WO-A1-97/18297
- AU-A1- 2018 200 424
- CA-A1- 2 858 069
- CA-A1- 3 002 066
- KR-A- 20080 105 555
- US-A1- 2015 011 543
- US-B2- 8 440 177
- FARES ET AL.: "EPCR expression marks UM171-expanded CD 34+ cord blood stem cells", BLOOD, vol. 129, no. 25, 2017, pages 3344 - 3351, XP055631709, ISSN: 1528-0020
- FARES ET AL.: "Cord blood expansion. Pyrimidoindole derivatives are agonists of human hematopoietic stem cell self-renewal", SCIENCE, vol. 345, no. 6203, 19 September 2014 (2014-09-19), pages 1509 - 1512, XP055232763, ISSN: 1095-9203
- COHEN ET AL.: "201. Single UM171 Expanded Cord Blood Transplant is Feasible, Safe, and Permits Transplantation of Better HLA Matched Cords with Very Low Transplant Related Mortality", BIOLOGY OF BLOOD AND MARROW TRANSPLANTATION, vol. 24, March 2018 (2018-03-01), pages S156 - S157, XP055631706, ISSN: 1083-8791

## Description

### RELATED APPLICATIONS

The present application claims benefit of U.S. Provisional Application No. 62/632,733 filed February 20, 2018.

### TECHNICAL FIELD

It is provided a graft of expanded stem and/or progenitor cells cultured with at least one compound, for use in a method of reducing severe graft-versus-host disease (GVHD), relapse, and/or severe viral infections, wherein said graft leads to an expansion of dendritic (DC) cells, natural killer (NK) cells or a combination thereof in vivo in a patient, wherein said expansion of DC cells, NK cells or combination thereof stimulates an immune response in said patient. The invention is defined in the claims.

### BACKGROUND

Immunotherapy has been considered a major breakthrough in the field of anti-cancer therapy, since this approach demonstrated its efficacy against chemotherapy refractory cancers. Although many efforts focused on antigen-targeted approaches, harnessing innate immunity to fight cancer cells has also been proposed and natural killer (NK) cells are increasingly used to design anti-cancer immunotherapy.

NK cells recognize and kill infected or transformed cells without prior sensitization. Their cytotoxicity activity against cancer cells is highly regulated by the balance between activating and inhibitory signals as well as their education in order to distinguish self and untransformed cells from cancer and infected cells. Nonetheless, cancer cells can become resistant to NK cell-mediated lysis by down-regulating ligands for NK cell activating receptors. To circumvent this resistance, NK cell stimulation is required to increase the cytotoxic functions of NK cells. Interleukin (IL)-2 and IL-15 are the most frequently used cytokines to increase NK cell lytic functions, but their use in clinics is associated with high toxicity and side effects that can dampen the efficacy of NK cell mediated cytotoxicity against cancer. Natural killer cells play a crucial role in protection against cancer relapse and infections.

NK cell functions can also be stimulated by low numbers of activated dendritic cells. Dendritic cells (DCs) are the most potent antigen-presenting cells that stimulate both innate and acquired immune responses thereby conferring resistance to infection, protective anti-tumor immunity and tolerance to self. This unique intrinsic capacity to generate a large number of high avidity effector cells, such as cytotoxic T lymphocytes and Natural killer cells, designate DCs as very good candidates for cell based therapy against various hematological malignancies including cancer, infectious diseases, allergy and autoimmune diseases. DCs can shape their functions based on their immune states, which are crucial for the balance of immunity and tolerance to preserve homeostasis. In the immune response involved in stem cell transplantation, DCs are involved in inducing immune tolerance and antitumor immunity.

Several DCs subsets have been characterized expressing different repertoires of Toll like receptors (TLR) and surface molecules and different sets of cytokines/chemokines, all of which lead to distinct and specific humoral and/or cellular immune responses. The two major subsets are the myeloid DCs (mDCs) and the plasmacytoid DCs (pDCs).

mDC and pDC respond differently to pathogenic stimuli and each subset has a specialized function in directing immune responses. While mDC produces TNF-α and IL-12 in response to microbial stimuli through TLR, pDCs, are the key effectors in innate immunity because they produce large amounts of type I interferon (IFN) in response to bacterial or viral infections. Recent observations suggest that both pDCs and mDCs are important for the induction of antitumor responses and may act synergistically to induce stronger immunological outcome.

Allogeneic hematopoietic stem cell (HSC) transplant is the best available therapy to cure patients with blood cancers. The risks of graft rejection, tumor recurrence, and tumorigenicity are still present after stem cell transplantation. Unfortunately, 40% of patients will not have a human leucocyte antigen (HLA) matched donor (related or unrelated). Cord blood (CB) is the most attractive alternative donor source of stem cells due to its unique properties, which include permissive HLA mismatches, low incidence of chronic graft-versus-host disease (GVHD) and rapid availability. However, these advantages are offset by the limited cell dose (i.e. small cords in banks), which results in delayed- or non-engraftment, increased infections, prolonged hospitalization and early mortality.

Allogeneic transplantation consists of a conditioning regimen (chemotherapy +/- radiation) followed by the infusion of stem cells (the graft) to eradicate residual cancer cells.

Bone marrow and stimulated peripheral blood stem cells are obtained from HLA matched related or volunteer unrelated donors while umbilical CB is donated at birth. Duration of neutropenia correlates directly with the risk of severe infections and transplant related mortality: it is shortest with peripheral blood (14 days), followed by bone marrow (19 days), but the longest with CB (26 days).

Allogeneic HSC transplant is associated with transplant related mortality rates up to 40%. The most common complications include acute (~50%) and chronic (~60%) GVHD. GVHD is a donor driven immune reaction against recipient, which frequently damages mucous membranes (mouth, eyes), skin, liver, lungs and intestinal tract. Further, infections are very common and graft failure (the absence of engraftment, ~10%) is caused by insufficient stem cells in the graft and/or graft destruction by the recipient's immune system. These complications are modulated by 4 key factors: i) the intensity of the conditioning regimen; ii) the type of graft infused (bone marrow, peripheral blood or CB); iii) the degree of HLA mismatch between the donor and recipient; and iv) the patient's comorbidities. GVHD is treated with high dose immunosuppressive drugs, including corticosteroids and frequently requires an average of 4-5 years of treatment. This prolonged need for immunosuppressive therapy further increases the risk of infections, secondary cancers and medication-related toxicities, all of which contribute to dramatically affect patients' quality of life.

Transplant recipients suffer from prolonged immunodeficiency. Initial T-cell recovery relies on peripheral expansion of donor memory T cells. This is later followed by maturation of donor stem cell-derived lymphoid progenitors into naïve T cells in the thymus, essential for reconstitution of a polyclonal T-cell repertoire. Until robust thymic output can be achieved, CB graft recipients have only naïve T cells (without memory cells) to fight against pathogens, explaining the increased risk of viral infections in the first months. As functional CD4⁺ T cells are mandatory for production of mature memory B cells, the latter usually do not become completely reconstituted until 1-2 years following HSC transplant and humoral immunity is predominantly recipient-derived in the first year.

Currently, only 6% of available CB units have sufficient cell doses for adults. Therefore, double CB transplants have become routine in adults. Accessibility to transplant improves with 2 cords because minimal required TNC dose is lower at 1.5x107/kg/cord. Neutrophil engraftment is not improved but the risk of graft failure is reduced. Early after transplant, both cords are detected, but only one remains after 3 months. Increasing T lymphocyte and CD34⁺ cell doses determine the surviving cord as each CB mounts an immune response to reject the other. This immune response is responsible for a higher incidence of severe acute GVHD and HSC destruction explaining why engraftment is delayed despite infusing a higher cell dose. Furthermore, the high cost makes double CBs prohibitive.
Bernstein et al. (CA 2858069) relates to compositions and methods for enhanced generation of hematopoietic stem/progenitor cells. KR 2008 0105555 relates to a therapeutic agent or method for treating graft-versus-host disease using mesenchymal stem cells. De la Pena et al. (US 8440177) relates to a method of treating graft versus host disease using adipose derived mesenchymal stem cells. Long *et al.* (WO 9718297) relates to functional human hematopoietic cells. Zhang et al. (CA 3002066) relates to natural killer cells and ILC3 cells and uses thereof. Hariri et al. (AU 2018200424) relates to methods of generating natural killer cells. Fares *et al.* (doi: 10.1182/blood-2016-11-750729) relates to EPCR expression marking UM171-expanded CD34+ cord blood stem cells. Fares *et al.* (doi:10.1126/science.125633) relates to pyrimidoindole derivatives being agonists of human hematopoietic stem cell self-renewal. Murphy et al. (WO 2016109661) relates to natural killer cells and uses thereof. Sauvageau et al. (WO 2017205977) relates to selection of human hematopoetic stem cells using EPCR.

### SUMMARY

The present invention is directed at a graft of expanded stem and/or progenitor cells cultured with at least one compound, for use in a method of reducing severe graft-versus-host disease (GVHD) and/or severe viral infections, wherein said graft leads to an expansion of dendritic (DC) cells, natural killer (NK) cells or a combination thereof *in vivo* in a patient, wherein said expansion of DC cells, NK cells or combination thereof stimulates an immune response in said patient and wherein said at least one compound has formula I: 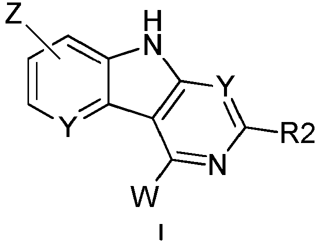 or a salt thereof,
wherein:
each Y is independently selected from N and CH;
Z is
   - CN
   - C(O)OR1,
   - C(O)N(R1)R3,
   - C(O)R1, or
   - heteroaryl optionally substituted with one or more RA or R4 substituents, wherein, when (R1) and R3 are attached to a nitrogen atom, optionally they join together with the nitrogen atom to form a 3 to 7-membered ring which optionally includes one or more other heteroatom selected from N, O and S, optionally the ring is substituted with one or more RA or R4;
W is
   - CN,
   - N(R1)R3,
   - C(O)OR1,
   - C(O)N(R1)R3,
   - NR1C(O)R1,
   - NR1C(O)OR1,
   - OC(O)N(R1)R3,
   - OC(O)R1,
   - C(O)R1,
   - NR1C(O)N(R1)R3,
   - NR1S(O)₂R1,
   - benzyl optionally substituted with 1, 2 or 3 RA or R1 substituents,
   - X-L-(X-L)n - N(R1)R3,
   - X-L-(X-L)n - heteroaryl optionally substituted with one or more RA or R4 substituents attached on either or both the L and heteroaryl groups,
   - X-L-(X-L)n - heterocyclyl optionally substituted with one or more RA or R4 substituents attached on either or both the L and heterocyclyl groups,
   - X-L-(X-L)n- aryl optionally substituted with one or more RA or R4 substituents,
   - X-L-(X-L)ₙ-NR1RA or
   - (N(R1)-L)ₙ - N⁺R1R3R5 R6⁻
wherein n is an integer equal to either 0, 1, 2, 3, 4, or 5,
and wherein, when R1 and R3 are attached to a nitrogen atom, optionally they join together with the nitrogen atom to form a 3 to 7-membered ring which optionally includes one or more other heteroatom selected from N, O and S, optionally the ring is substituted with one or more RA or R4;
each X is independently selected from C, O, S, and NR1;
each L is independently
   - C₁₋₆ alkylene,
   - C₂₋₆ alkenylene,
   - C₂₋₆ alkynylene,
   - C₃₋₇cycloalkylene, which optionally includes one or more other heteroatom selected from N, O and S or
   - C₃₋₇ cycloalkenylene, which optionally includes one or more other heteroatom selected from N, O and S
wherein the alkylene, the alkenylene, the alkynylene the cycloalkylene and the cycloalkenylene groups are each independently optionally substituted with one or two R4 or RA substituent;
R1 is each independently
   - H,
   - C₁₋₆ alkyl,
   - C₂₋₆ alkenyl,
   - C₂₋₆ alkynyl,
   - C₃₋₇ cycloalkyl,
   - C₃₋₇ cycloalkenyl,
   - C₁₋₅ perfluorinated,
   - heterocyclyl,
   - aryl,
   - heteroaryl,or
   - benzyl,
wherein the alkyl, the alkenyl, the alkynyl, the cycloalkenyl, the perfluorinated alkyl, the heterocyclyl, the aryl, the heteroaryl and the benzyl groups are each independently optionally substituted with 1, 2 or 3 RA or Rd substituents;
R2 is
   - H,
   - C₁₋₆ alkyl, optionally substituted with one more RA substituents
   - C(O)R4,
   - L-heteroaryl optionally substituted with one or more RA or R4 substituents
   - L-heterocyclyl optionally substituted with one or more RA or R4, or
   - L-aryl optionally substituted with one or more RA or R4 substituents;
R3 is each independently
   - H,
   - C₁₋₆ alkyl,
   - C₂₋₆ alkenyl,
   - C₂₋₆ alkynyl,
   - C₃₋₇ cycloalkyl,
   - C₃₋₇ cycloalkenyl,
   - C₁₋₅ perfluorinated,
   - heterocyclyl,
   - aryl,
   - heteroaryl,or
   - benzyl,
wherein the alkyl, the alkenyl, the alkynyl, the cycloalkyl, the cycloalkenyl, the perfluorinated alkyl, the heterocyclyl, the aryl, the heteroaryl and the benzyl groups are each independently optionally substituted with 1, 2 or 3 RA or Rd substituents;
R4 is each independently
   - H,
   - C₁₋₆ alkyl,
   - C₂₋₆ alkenyl,
   - C₂₋₆ alkynyl,
   - C₃₋₇ cycloalkyl,
   - C₃₋₇ cycloalkenyl,
   - C₁₋₅ perfluorinated,
   - heterocyclyl,
   - aryl,
   - heteroaryl, or
   - benzyl,
wherein the alkyl, the alkenyl, the alkynyl, the cycloalkyl, the cycloalkenyl, the perfluorinated alkyl, the heterocyclyl, the aryl, the heteroaryl and the benzyl groups are each independently optionally substituted with 1, 2 or 3 RA or Rd substituents;
R5 is each independently
   - C₁₋₆ alkyl,
   - C₁₋₆ alkylene-C₂₋₆ alkenyl which optionally includes one or more other heteroatom selected from N, O and S
   - C₁₋₆ alkylene-C₂₋₆ alkynyl which optionally includes one or more other heteroatom selected from N, O and S
   - L-aryl which optionally includes one or more RA or R4 substituents
   - L-heteroaryl which optionally includes one or more RA or R4 substituents
   - C₁₋₆ alkylene-C(O)O-
   - C₁₋₆ alkylene-C(O)OR1
   - C₁₋₆ alkylene-CN
   - C₁₋₆ alkylene-C(O)NR1R3, wherein R1 and R3 optionally they join together with the nitrogen atom to form a 3 to 7-membered ring which optionally includes one or more other heteroatom selected from N, O and S; or
   - C₁₋₆ alkylene-OH;
R6 is
   - Halogen
   - OC(O)CF₃ or
   - OC(O)R1;
RA is each independently
   - halogen,
   - CF₃,
   - OR1,
   - L-OR1,
   - OCF₃,
   - SR1,
   - CN,
   - NO₂,
   - NR1R3,
   - L-NR1R1,
   - C(O)OR1,
   - S(O)₂R4
   - C(O)N(R1)R3,
   - NR1C(O)R1,
   - NR1C(O)OR1,
   - OC(O)N(R1)R3,
   - OC(O)R1,
   - C(O)R4,
   - NHC(O)N(R1)R3,
   - NR1C(O)N(R1)R3, or
   - N₃; and
Rd is each independently
   - H,
   - C₁₋₆ alkyl,
   - C₂₋₆ alkenyl,
   - C₂₋₆ alkynyl,
   - C₃₋₇ cycloalkyl,
   - C₃₋₇ cycloalkenyl,
   - C₁₋₅ perfluorinated
   - benzyl or
   - heterocyclyl.

The invention is further defined in the claims.

In an embodiment, the stem and/or progenitor cells are human hematopoietic stem cells (HSC).

In another embodiment, the hematopoietic stem cells are from umbilical cord blood cells, mobilized peripheral blood cells, or bone marrow cells.

In a further embodiment, the hematopoietic stem cells are from human cord blood cells.

In an embodiment, the stem and/or progenitor cells are purified for CD34⁺, CD38⁺, CD90⁺, CD45RA⁺, CD133 and/or CD49f⁺ cells.

In another embodiment, the CD34+ cells are EPCR+ cells.

In a further embodiment, the NK cells are CD56⁺ or NKG2A⁺ cells.

In another embodiment, the DC cells are CD11c⁺.

In an embodiment, the stem and/or progenitor cells are cultured with at least one cell expanding factor.

In another embodiment, the at least one cell expanding factor is interleukin-3 (IL-3), granulocyte macrophage colony-stimulating factor (GM-CSF), thrombopoieting (TPO), FMS-like tyrosine kinase 3 ligand (FLT3-L), stem cell factor (SCF), interleukin-6 (IL-6) or a combination thereof.

In a further embodiment, the stem and/or progenitor cells are further cultured with an aryl hydrocarbon receptor (AHR) antagonist.

In an embodiment, the AHR antagonist is Stem Regenin 1 (SR1) or CH223191.

In a preferred embodiment, the compound of formula I is 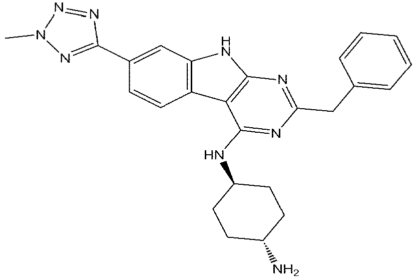 or a pharmaceutically acceptable salt thereof.

In another embodiment, the compound of formula I is a hydrobromide salt of

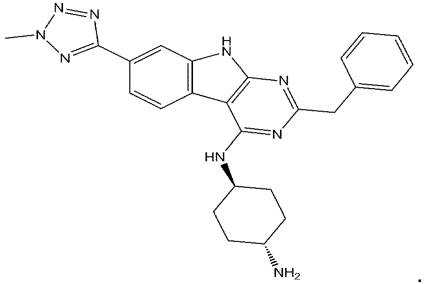

In a supplemental embodiment, the compound of formula I is 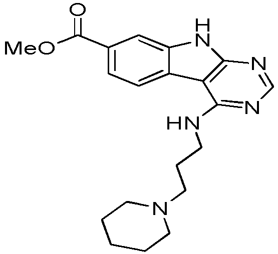 or a pharmaceutically acceptable salt thereof.

In another embodiment, the compound of formula I is:

| | |
|---|---|
| **1** | 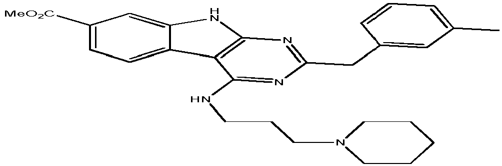 |
| **2** | 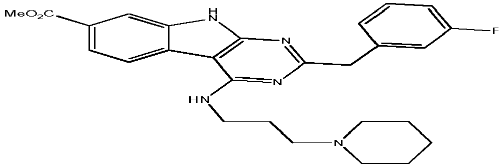 |
| **3** | 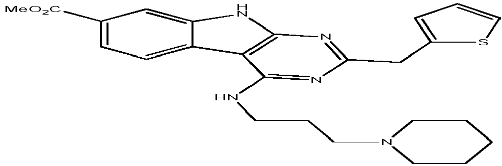 |
| **4** | 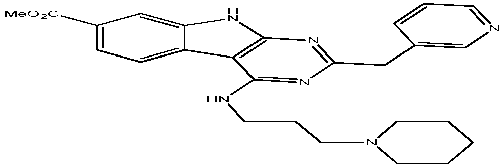 |
| **5** | 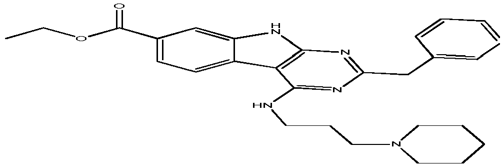 |
| **6** | 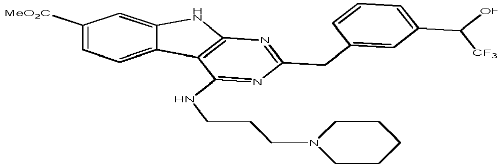 |
| **7** | 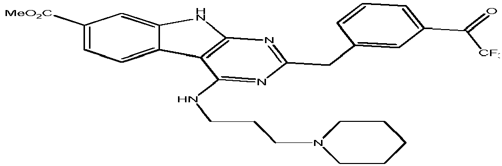 |
| **8** | 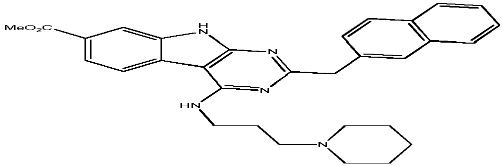 |
| **9** | 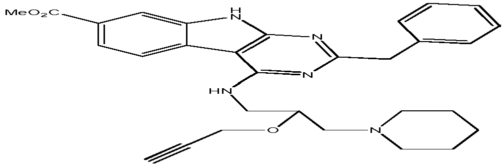 |
| **10** | 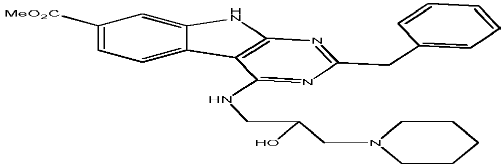 |
| **11** | 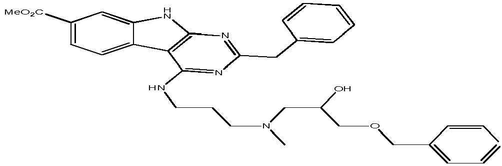 |
| **12** | 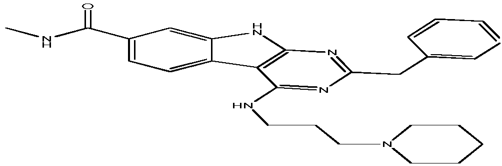 |
| **13** | 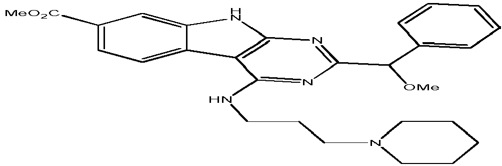 |
| **14** | 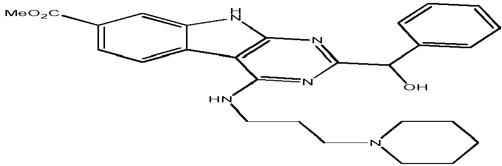 |
| **15** | 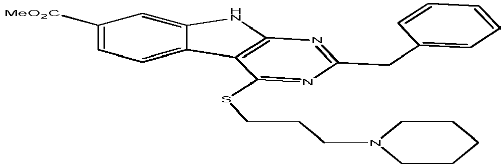 |
| **16** | 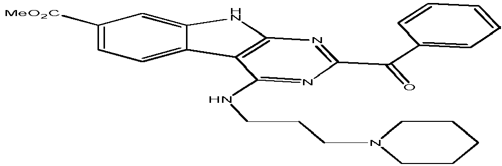 |
| **17** | 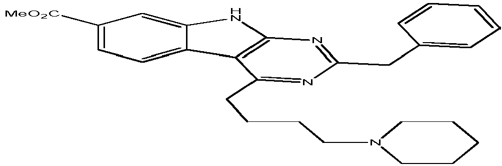 |
| **18** | 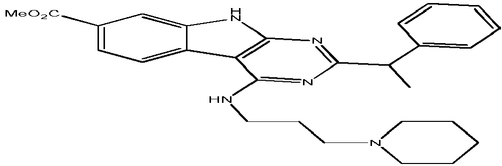 |
| **19** | 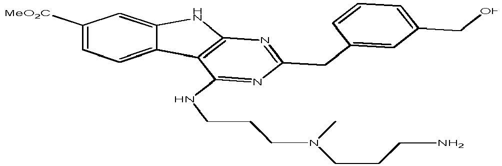 |
| **20** | 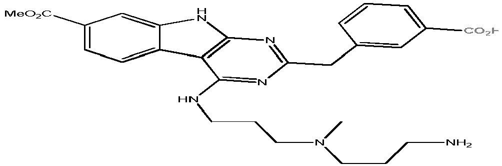 |
| **21** | 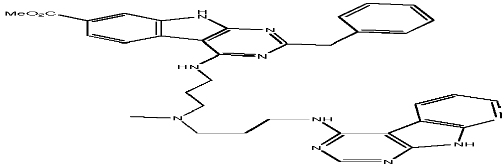 |
| **22** | 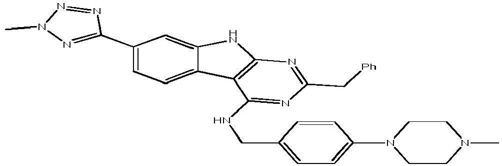 |
| **23** | 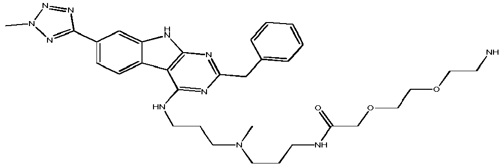 |
| **24** | 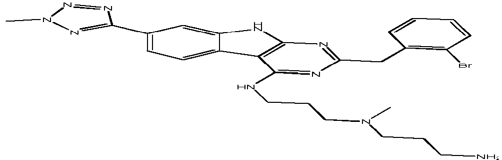 |
| **25** | 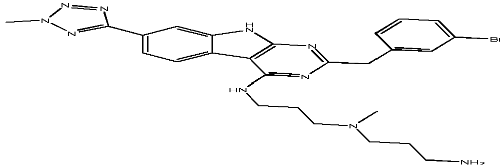 |
| **26** | 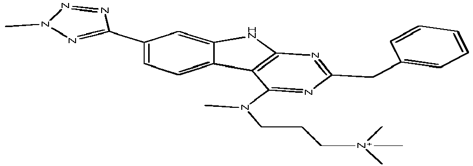 |
| **27** | 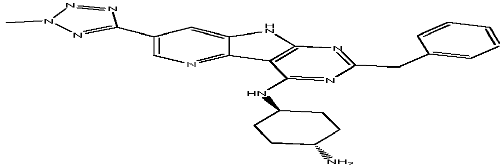 |
| **28** | 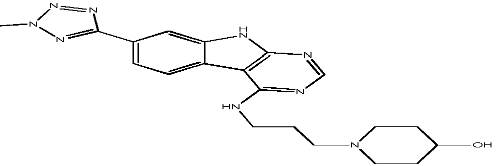 |
| **29** | 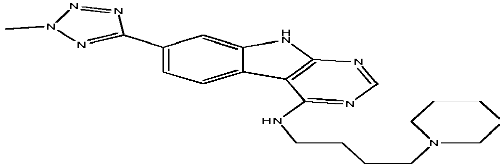 |
| **30** | 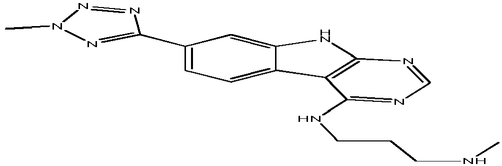 |
| **31** | 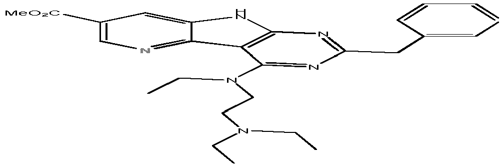 |
| **32** | 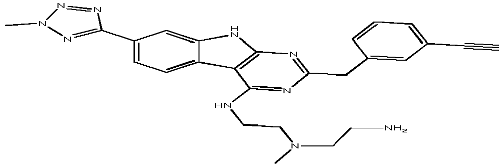 |
| **33** | 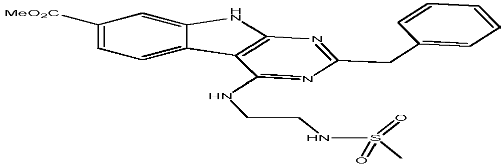 |
| **34** | 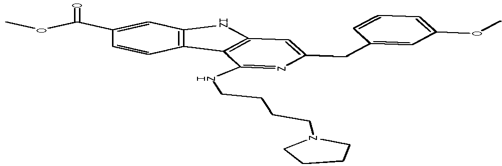 |
| **35** | 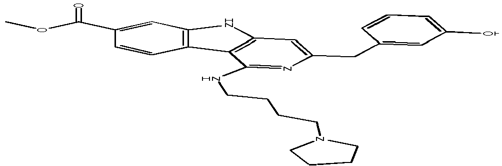 |
| **36** | 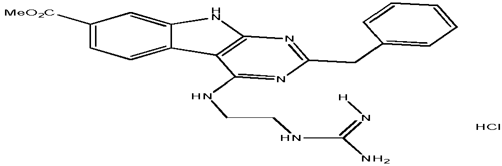 |
| **37** | 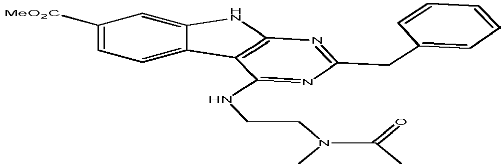 |
| **38** | 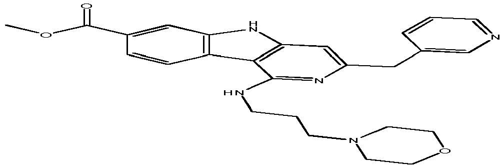 |
| **39** | 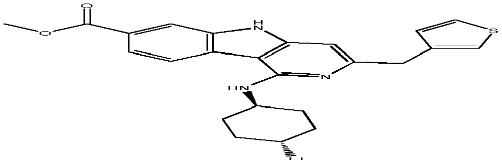 |
| **40** | 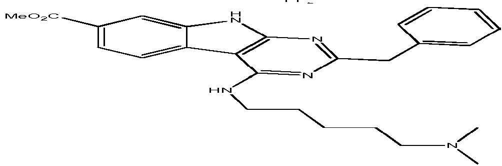 |
| **41** | 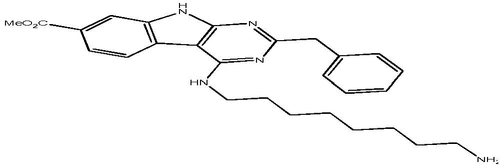 |
| **42** | 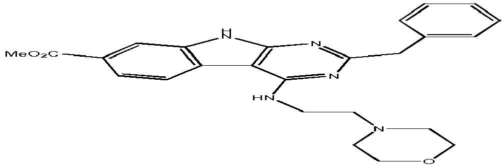 |
| **43** | 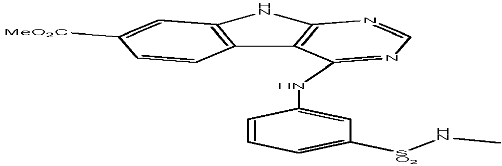 |
| **44** | 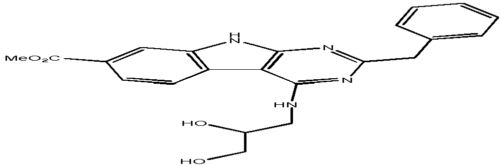 |
| **45** | 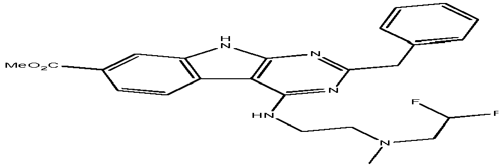 |
| **46** | 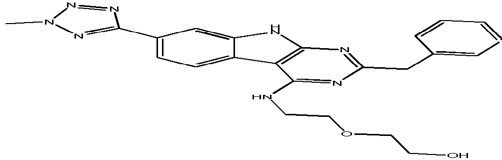 |
| **47** | 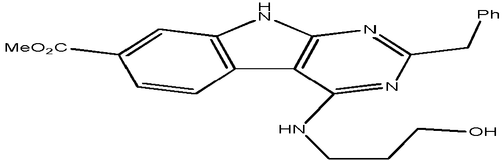 |
| **48** | 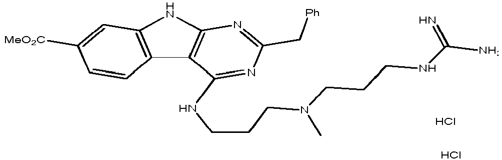 |
| **49** | 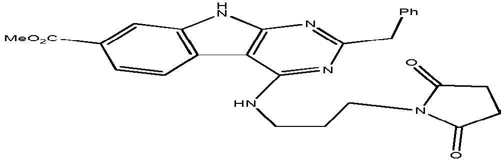 |
| **50** | 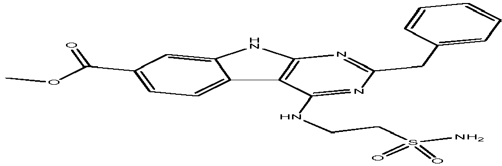 |
| **51** | 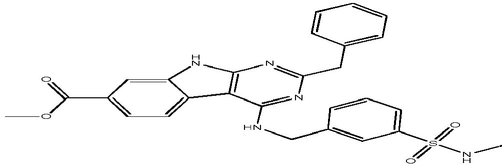 |
| **52** | 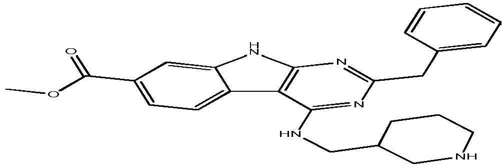 |
| **53** | 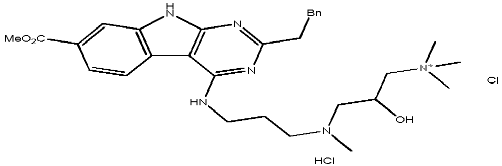 |
| **54** | 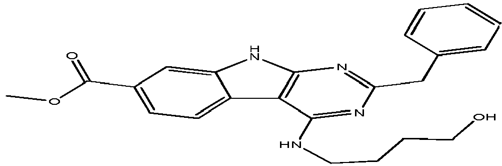 |
| **55** | 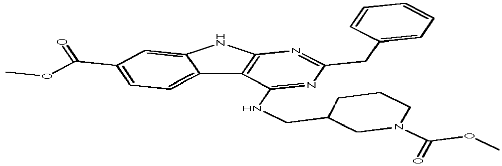 |
| **56** | 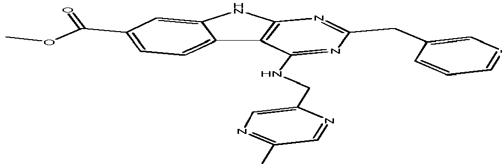 |
| **57** | 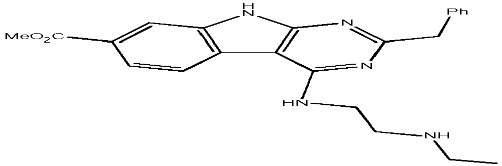 |
| **58** | 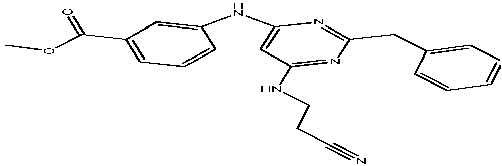 |
| **59** | 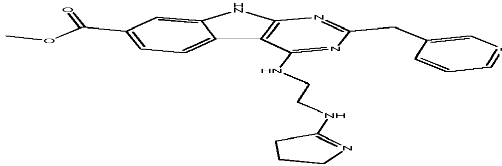 |
| **60** | 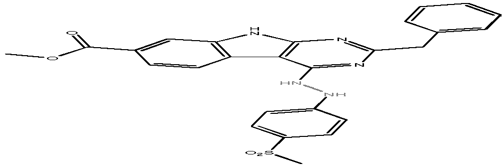 |
| **61** | 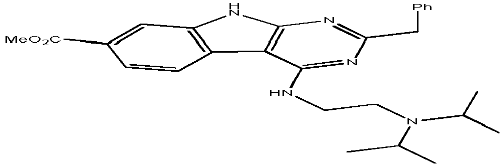 |

or a pharmaceutically acceptable salt thereof.

In another embodiment, the stem and/or progenitor cells are expanded in a bioreactor.

In an embodiment, the patient is a human or an animal.

In another embodiment, the animal is a mouse.

In an embodiment, the viral infection is a CMV infection, an EBV infection or an adenovirus cystitis.

In another embodiment, the DC cells and/or NK cells are expanded at 14 days, at 21 days, at 28 days, at 56 days, at 100 days, at 6 months, at 12 months or at 18 months in the patient.

In an embodiment, the inflammatory state is controlled in the patient.

In an embodiment, the graft comprises a dendritic cell population.

In another embodiment, the dendritic cell population are CD86⁺CD34⁺ cells.

In a further embodiment, the graft comprises 40-50% of dendritic cells.

In another embodiment, the graft comprises 1/3 of immature dendritic cells.

In an embodiment, the graft comprises mast cells.

In another embodiment, the graft comprises about 10% of mast cells.

In a preferred embodiment, the graft comprises FCER1⁺CD34⁺ cells, CD34⁺CD45RA⁺ cells, CD34⁺CD86⁺ cells, CD34⁺CD45RA⁻ cells and CD34⁻ cells.

### BRIEF DESCRIPTION OF THE DRAWINGS

Fig. 1 illustrates that UM171 promotes the *ex vivo* expansion of primitive dendritic cell progenitors, wherein cord blood derived CD34+ cells were cultured for 7 and 14 days in HSC expansion media supplemented with vehicle (DMSO 0.1%), SR1 (500nM) or UM171 (35nM), frequency of dendritic cells precursors (iDC: CD34+CD86+) was evaluated by flow.
Fig. 2 illustrates cell populations identified in cord blood samples expanded and cultured with DMSO, SR1 or UM171, showing the unique signature of the graft.
Fig. 3 illustrates in (A) the clinical trial design; in (B) the definition of patient cohort; cord blood accessibility using standard selection criteria for a 70 Kg patient in (C) or criteria used for cohort 2 patients in (D); and in (E) the historical data at Hôpital Maisonneuve-Rosemont showing relationship between CD34 cell dose (post thaw of CB unit) versus time to neutrophil engraftment.
Fig. 4 illustrates post-engraftment measurement of NK cells in UM171 CB patients wherein the arrow points to measurement at about 2 months post-transplant.
Fig. 5 illustrates engraftment and chimerism, showing in (A) time to 100 neutrophil engraftment; (B) resolution of fever and (C) duration of hospitalization for patients transplanted with UM171-expanded CB (blue) or unmanipulated CB cells (green). (D) Lineage chimerism at different time points in days in patients transplanted with UM171-expanded CB cells. (E) Comparison of CD4 counts at 3 and 12 months in patients transplanted with UM171 expanded CB and unmanipulated CB. (F) IgG levels in recipients of UM171 expanded CB. Ctrl CB: unmanipulated cord blood.
Fig. 6 illustrates HLA matching of CB blood units in UM171 patient cohort (left) and in contemporary CB cohort (right), showing 6-7/8 match between patient and CB unit: 4-5/8 match.
Fig. 7 illustrates cumulative incidence of (A) immunosuppressor withdrawal, (B) transplant-related mortality (TRM); and (C) Kaplan-Meier estimates of overall survival (OS) in UM171-expanded CB and unmanipulated CB cohorts.

### DETAILED DESCRIPTION

In accordance with the present disclosure, there is provided a graft of expanded stem and/or progenitor cells cultured with at least one compound for use in a method as defined in the claims.

It is thus provided a graft as defined in the claims for use in a method of expanding dendritic (DC) cells and/or natural killer (NK) cells *in vivo* in a patient comprising the steps of producing a graft of stem and progenitor cells cultured with UM171 or analogues therefrom and expanded before being administered to a patient. The expansion or increase in dendritic (DC) cells and natural killer (NK) cells population in the patient results in an increase immune response reducing transplant related mortality (TRM), severe graft-versus-host disease (GVHD), relapse, and/or severe viral infections.

Allogeneic hematopoietic stem cell (HSC) transplant is the best available therapy to cure patients with blood cancers. Cord blood (CB) is the most attractive source of stem cells.

It is known that a purine derivative, StemRegenin 1 (SR1), which promotes the *ex vivo* expansion of CD34⁺ cells, could expand HSCs but it was not possible not to reproduce expansion of long-term HSCs (Chen et al., 2012, Genes Dev., 26: 2499-2511). A major concern with SR1 is its ability to support leukemia stem cell growth *in vitro* (Pabst et al., 2014, Nature Methods, 11: 436-442).

Approximately 1% of CB cells express the CD34 surface antigen, which is made up of distinct subsets of stem and progenitor cells that have variable ability to provide short- or long-term hematopoiesis. These subsets contribute differently to early and long-term recovery of mature blood cell production. Only long-term HSCs can provide lifelong hematopoietic reconstitution. *In vitro* expansion of CB-derived short-term HSCs (progenitor cells) dramatically shortens time to neutrophil recovery post-transplant. However, most of the *ex vivo* cell expansion strategies described to date achieve this effect at the expense of long-term HSC loss, thereby compromising durable reconstitution with the risk of late graft failure. Accordingly, many of these strategies require the infusion of a 2^{nd} CB to ensure long-term engraftment. In sharp contrast, as described and encompassed herein, the molecule used in amplifying CB-derived short-term repopulating cells allow simultaneously expanding, not depleting, the long-term ones, paving the way for single expanded CB.

UM171 encompassed herein (see U.S. patent no. 9,409,906), has an activity on primitive cells which is rapidly reversible if the compound is washed out from culture. UM171 does not independently trigger cell proliferation in the absence of growth factors; it is not mitogenic but rather prevents cell differentiation. UM171 was studied in a fed-batch culture system. Several negative cytokine regulators are released by mature cells as they are generated in CD34⁺ CB cultures (Csaszar et al., 2012, Cell stem cell, 10: 218-229). The fed-batch encompassed herein leads to a reduction of endogenously produced negative regulators. This system requires much less media (<1 liter culture vs. 10 liters in most studies) and better supports the maintenance of CB-derived HSCs. A 7-day culture is optimal to maximize the quality of the cells. Importantly, the fed-batch culture is a closed system without the need for cell manipulation, minimizing contamination risk and facilitating the transition to cell product manufacturing.

As encompassed herein, the expansion of stem and/or progenitor cells can be conducted in a bioreactor consisting of any manufactured or engineered device or system that supports a biologically active environment such as cultured cells for expansion of said cells.

Accordingly, it is encompassed a graft as defined in the claims for use in a method expanding *in vivo* natural killer cells, dendritic cells or a combination thereof after transplant with a cord blood graft using a compound of general formula I as defined herein: 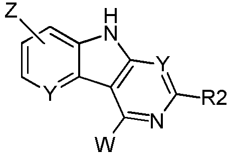 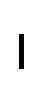 or a salt thereof,
wherein:
each Y is independently selected from N and CH;
Z is -CN; -C(O)OR1; -C(O)N(R1)R3; -C(O)R1; or -heteroaryl optionally substituted with one or more RA or R4 substituents, wherein, when (R1) and R3 are attached to a nitrogen atom, optionally they join together with the nitrogen atom to form a 3 to 7-membered ring which optionally includes one or more other heteroatom selected from N, O and S, optionally the ring is substituted with one or more RA or R4;
W is -CN; -N(R1)R3; -C(O)OR1; -C(O)N(R1)R3; -NR1C(O)R1; -NR1C(O)OR1; - OC(O)N(R1)R3; -OC(O)R1; -C(O)R1; -NR1C(O)N(R1)R3; -NR1S(O)₂R1; -benzyl optionally substituted with 1, 2 or 3 RA or R1 substituents; -X-L-(X-L)n; -N(R1)RS3; -X-L-(X-L)n - heteroaryl optionally substituted with one or more RA or R4 substituents attached on either or both the L and heteroaryl groups; -X-L-(X-L)n - heterocyclyl optionally substituted with one or more RA or R4 substituents attached on either or both the L and heterocyclyl groups; -X-L-(X-L)n- aryl optionally substituted with one or more RA or R4 substituents; -X-L-(X-L)ₙ-NR1RA or -(N(R1)-L)ₙ - N⁺R1R3R5 R6⁻, wherein n is an integer equal to either 0, 1, 2, 3, 4, or 5,
and wherein, when R1 and R3 are attached to a nitrogen atom, optionally they join together with the nitrogen atom to form a 3 to 7-membered ring which optionally includes one or more other heteroatom selected from N, O and S, optionally the ring is substituted with one or more RA or R4;
each X is independently selected from C, O, S, and NR1;
L is each independently -C₁₋₆ alkylene; -C₂₋₆ alkenylene; -C₂₋₆ alkynylene; -C₃₋₇ cycloalkylene, which optionally includes one or more other heteroatom selected from N, O and S; or -C₃₋₇ cycloalkenylene, which optionally includes one or more other heteroatom selected from N, O and S, wherein the alkylene, the alkenylene, the alkynylene, the cycloalkylene and the cycloalkenylene groups are each independently optionally substituted with one or two R4 or RA substituent;
R1 is each independently -H; -C₁₋₆ alkyl; -C₂₋₆ alkenyl; -C₂₋₆ alkynyl; -C₃₋₇ cycloalkyl; -C₃₋₇ cycloalkenyl; -C₁₋₅ perfluorinated; -heterocyclyl; -aryl; -heteroaryl; or -benzyl, wherein the alkyl, the alkenyl, the alkynyl, the cycloalkenyl, the perfluorinated alkyl, the heterocyclyl, the aryl, the heteroaryl and the benzyl groups are each independently optionally substituted with 1, 2 or 3 RA or Rd substituents;
R2 is -H; -C₁₋₆ alkyl, optionally substituted with one more RA substituents; -C(O)R4; -L-heteroaryl optionally substituted with one or more RA or R4 substituents; -L-heterocyclyl optionally substituted with one or more RA or R4; or -L-aryl optionally substituted with one or more RA or R4 substituents;
R3 is each independently -H; -C₁₋₆ alkyl; -C₂₋₆ alkenyl; -C₂₋₆ alkynyl; -C₃₋₇ cycloalkyl; -C₃₋₇ cycloalkenyl; -C₁₋₅ perfluorinated; -heterocyclyl; -aryl; -heteroaryl; or -benzyl, wherein the alkyl, the alkenyl, the alkynyl, the cycloalkyl, the cycloalkenyl, the perfluorinated alkyl, the heterocyclyl, the aryl, the heteroaryl and the benzyl groups are each independently optionally substituted with 1, 2 or 3 RA or Rd substituents;
R4 is each independently -H; -C₁₋₆ alkyl; -C₂₋₆ alkenyl; -C₂₋₆ alkynyl; -C₃₋₇ cycloalkyl; -C₃₋₇ cycloalkenyl; -C₁₋₅ perfluorinated; -heterocyclyl; -aryl; -heteroaryl, or -benzyl; wherein the alkyl, the alkenyl, the alkynyl, the cycloalkyl, the cycloalkenyl, the perfluorinated alkyl, the heterocyclyl, the aryl, the heteroaryl and the benzyl groups are each independently optionally substituted with 1, 2 or 3 RA or Rd substituents;
R5 is each independently -C₁₋₆ alkyl; -C₁₋₆ alkylene-C₂₋₆ alkenyl which optionally includes one or more other heteroatom selected from N, O and S; -C₁₋₆ alkylene-C₂₋₆ alkynyl which optionally includes one or more other heteroatom selected from N, O and S; -L-aryl which optionally includes one or more RA or R4 substituents; -L-heteroaryl which optionally includes one or more RA or R4 substituents; -C₁₋₆ alkylene-C(O)O-; -C₁₋₆ alkylene-C(O)OR1; -C₁₋₆ alkylene-CN; -C₁₋₆ alkylene-C(O)NR1R3, wherein R1 and R3 optionally they join together with the nitrogen atom to form a 3 to 7-membered ring which optionally includes one or more other heteroatom selected from N, O and S; or - C₁₋₆ alkylene-OH;
R6 is halogen; -OC(O)CF₃; or -OC(O)R1;
RA is each independently -halogen; -CF₃; -OR1; -L-OR1; -OCF₃; -SR1; -CN; -NO₂; - NR1R3; -L-NR1R1; -C(O)OR1; -S(O)₂R4; -C(O)N(R1)R3; -NR1C(O)R1; - NR1C(O)OR1; -OC(O)N(R1)R3; -OC(O)R1; -C(O)R4; -NHC(O)N(R1)R3; - NR1C(O)N(R1)R3; or -N₃; and
Rd is each independently -H; -C₁₋₆ alkyl; -C₂₋₆ alkenyl; -C₂₋₆ alkynyl; -C₃₋₇ cycloalkyl; -C₃₋₇ cycloalkenyl; -C₁₋₅ perfluorinated; -benzyl; or -heterocyclyl.

In one embodiment, Z is -C(O)OR1, or -heteroaryl optionally substituted with one or more RA or R1 substituents, R2 is H, -C₁₋₆ alkyl optionally substituted with one or more RA substituents or -L-aryl optionally substituted with one or more RA or R4 substituents, W is -N(R1)R3 wherein R1 is C₃₋₇ cycloalkyl substituted by RA and R3 is H.

In one embodiment, Z is -C(O)O-C₁₋₄ alkyl or 5-membered ring heteroaryl, the heteroaryl comprising 2-4 heteroatoms (N or O), R2 is H, or -L-aryl optionally substituted by halogen, OR1, C₁₋₆ alkyl optionally substituted by RA, C(O)R4, - heterocyclyl, C(O)OR4 OR C₂₋₆ alkynyl, W is -N(R1)R3 wherein R1 is cyclohexyl substituted by RA, and R3 is H.

In one embodiment, Z is COOMe, COOEt, tetrazole or oxadiazole.

In one embodiment, R2 is = H, or -CH2-aryl optionally substituted by substituted by halogen, OR1, C₁₋₆ alkyl optionally substituted by RA, C(O)R4, - heterocyclyl, C(O)OR4 OR C₂₋₆ alkynyl, wherein the aryl is phenyl.

In one embodiment, R2 is H, -C₁₋₆ alkylene-heteroaryl or -C₁₋₆ alkylene-aryl, optionally substituted with one or more RA or R4 substituents.

In accordance with another embodiment, the compound is of Formula I, IA or IIA wherein Z is CO₂Me or 2-methyl-2H-tetrazol-5-yl;
R2 is benzyl, or H; and
W is NH-L-N(R1)R3 wherein L is C₂₋₄ alkylene or C₃₋₇ cycloalkylene and R1 and R3 is C₁₋₄ alkyl or H; or R1 and R3 join together with the nitrogen atom to which they are attached to form a 3 to 7-membered ring, which optionally includes one or more other heteroatom selected from N, O and S, optionally the ring is substituted with one or more RA or R4.

In accordance with another embodiment, the compound is of Formula I wherein W is

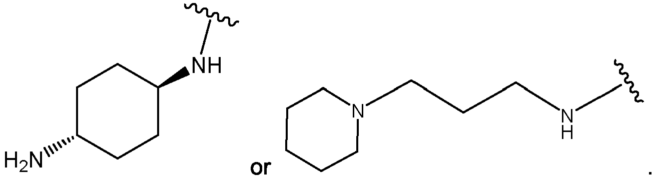

The compounds of formula I (including the representative compounds set forth below) disclosed herein, including the preparation and characterization thereof, are described in PCT publication No. WO 2013/110198 as well as in the synthetic methodology section found below.

As used herein, the term "alkyl" is intended to include both branched and straight chain saturated aliphatic hydrocarbon groups having the specified number of carbon atoms, for example, C1-C6 in C1-C6 alkyl is defined as including groups having 1, 2, 3, 4, 5 or 6 carbons in a linear or branched saturated arrangement. Examples of C1-C6 alkyl as defined above include, but are not limited to, methyl, ethyl, n-propyl, i-propyl, n-butyl, t-butyl, i-butyl, pentyl, and hexyl.

As used herein, the term "cycloalkyl" is intended to mean a monocyclic saturated aliphatic hydrocarbon group having the specified number of carbon atoms therein, for example, C3-C7 in C3-C7 cycloalkyl is defined as including groups having 3, 4, 5, 6 or 7 carbons in a monocyclic saturated arrangement. Examples of C3-C7 cycloalkyl as defined above include, but are not limited to, cyclopropyl, cyclobutyl, cyclopentyl, cyclohexyl and cycloheptyl.

As used herein, the term, "alkenyl" is intended to mean unsaturated straight or branched chain hydrocarbon groups having the specified number of carbon atoms therein, and in which at least two of the carbon atoms are bonded to each other by a double bond, and having either E or Z regiochemistry and combinations thereof. For example, C2-C6 in C2-C6 alkenyl is defined as including groups having 2, 3, 4, 5 or 6 carbons in a linear or branched arrangement, at least two of the carbon atoms being bonded together by a double bond. Examples of C2-C6 alkenyl include, but are not limited to, ethenyl (vinyl), 1-propenyl, 2-propenyl, 1-butenyl and the like.

As used herein, the term "alkynyl" is intended to mean unsaturated, straight chain hydrocarbon groups having the specified number of carbon atoms therein and in which at least two carbon atoms are bonded together by a triple bond. For example C2-C4 alkynyl is defined as including groups having 2, 3 or 4 carbon atoms in a chain, at least two of the carbon atoms being bonded together by a triple bond. Examples of such alkynyl include, but are not limited to, ethynyl, 1-propynyl, 2-propynyl and the like.

As used herein, the term "cycloalkenyl" is intended to mean a monocyclic saturated aliphatic hydrocarbon group having the specified number of carbon atoms therein, for example, C3-C7 in C3-C7 cycloalkenyl is defined as including groups having 3, 4, 5, 6 or 7 carbons in a monocyclic arrangement. Examples of C3-C7 cycloalkenyl as defined above include, but are not limited to, cyclopentenyl, cyclohexenyl and the like.

As used herein, the term "halo" or "halogen" is intended to mean fluorine, chlorine, bromine or iodine.

As used herein, the term "haloalkyl" is intended to mean an alkyl as defined above, in which each hydrogen atom may be successively replaced by a halogen atom. Examples of haloalkyl include, but are not limited to, CH₂F, CHF₂ and CF₃.

As used herein, the term "aryl," either alone or in combination with another radical, means a carbocyclic aromatic monocyclic group containing 6 carbon atoms which may be further fused to a second 5- or 6-membered carbocyclic group which may be aromatic, saturated or unsaturated. Examples of aryl include, but are not limited to, phenyl, indanyl, 1-naphthyl, 2-naphthyl, tetrahydronaphthyl and the like. The aryl may be connected to another group either at a suitable position on the cycloalkyl ring or the aromatic ring.

As used herein, the term "heteroaryl" is intended to mean a monocyclic or bicyclic ring system of up to 10 atoms, wherein at least one ring is aromatic, and contains from 1 to 4 hetero atoms selected from the group consisting of O, N, and S. The heteroaryl may be attached either via a ring carbon atom or one of the heteroatoms. Examples of heteroaryl include, but are not limited to, thienyl, benzimidazolyl, benzo[b]thienyl, furyl, benzofuranyl, pyranyl, isobenzofuranyl, chromenyl, xanthenyl, 2H-pyrrolyl, pyrrolyl, imidazolyl, pyrazolyl, pyridyl, pyrazinyl, pyrimidinyl, pyridazinyl, indolizinyl, isoindolyl, 3H-indolyl, indolyl, indazolyl, purinyl, 4H-quinolizinyl, isoquinolyl, quinolyl, phthalazinyl, napthyridinyl, quinoxalinyl, quinazolinyl, cinnolinyl, pteridinyl, isothiazolyl, isochromanyl, chromanyl, isoxazolyl, furazanyl, indolinyl, isoindolinyl, thiazolo[4,5-b]-pyridine, tetrazolyl, oxadiazolyl, thiadiazolyl, thienyl, pyrimido-indolyl, pyrido-indolyl, pyrido-pyrrolo-pyrimidinyl, pyrrolo-dipyridinyl and fluoroscein derivatives.

As used herein, the term "heterocycle," "heterocyclic" or "heterocyclyl" is intended to mean a 3, 4, 5, 6, or 7 membered non-aromatic ring system containing from 1 to 4 heteroatoms selected from the group consisting of O, N and S. Examples of heterocycles include, but are not limited to, pyrrolidinyl, tetrahydrofuranyl, piperidyl, 3,5-dimethylpiperidyl, pyrrolinyl, piperazinyl, imidazolidinyl, morpholinyl, imidazolinyl, pyrazolidinyl, pyrazolinyl, tetrahydro-1H-thieno[3,4-d]imidazole-2(3H)-one, diazirinyl, and the like, where the attachment to the ring can be on either the nitrogen atom or a carbon atom of the ring such as described hereafter:

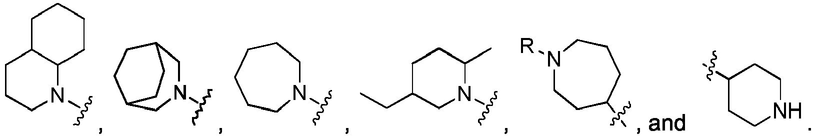

As used herein, the term "optionally substituted with one or more substituents" or its equivalent term "optionally substituted with at least one substituent" is intended to mean that the subsequently described event of circumstances may or may not occur, and that the description includes instances where the event or circumstance occurs and instances in which it does not. The definition is intended to mean from zero to five substituents.

As used herein, the term "subject" or "patient" is intended to mean humans and non-human mammals such as primates, cats, dogs, swine, cattle, sheep, goats, horses, rabbits, rats, mice and the like.

If the substituents themselves are incompatible with the synthetic methods described herein, the substituent may be protected with a suitable protecting group (PG) that is stable to the reaction conditions used in these methods. The protecting group may be removed at a suitable point in the reaction sequence of the method to provide a desired intermediate or target compound. Suitable protecting groups and the methods for protecting and de-protecting different substituents using such suitable protecting groups are well known to those skilled in the art; examples of which may be found in T. Greene and P. Wuts, "Protecting Groups in Chemical Synthesis" (4th ed.), John Wiley & Sons, NY (2007). Examples of protecting groups used throughout include, but are not limited to, Fmoc, Bn, Boc, CBz and COCF₃. In some instances, a substituent may be specifically selected to be reactive under the reaction conditions used in the methods described herein. Under these circumstances, the reaction conditions convert the selected substituent into another substituent that is either useful in an intermediate compound in the methods described herein or is a desired substituent in a target compound.

As used herein, the term "pharmaceutically acceptable salt" is intended to mean both acid and base addition salts.

As used herein, the term "pharmaceutically acceptable acid addition salt" is intended to mean those salts which retain the biological effectiveness and properties of the free bases, which are not biologically or otherwise undesirable, and which are formed with inorganic acids such as hydrochloric acid, hydrobromic acid, sulfuric acid, nitric acid, phosphoric acid and the like, and organic acids such as acetic acid, trifluoroacetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like.

As used herein, the term "pharmaceutically acceptable base addition salt" is intended to mean those salts which retain the biological effectiveness and properties of the free acids, which are not biologically or otherwise undesirable. These salts are prepared from addition of an inorganic base or an organic base to the free acid. Salts derived from inorganic bases include, but are not limited to, the sodium, potassium, lithium, ammonium, calcium, magnesium, iron, zinc, copper, manganese, aluminum salts and the like. Salts derived from organic bases include, but are not limited to, salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethylaminoethanol, 2-diethylaminoethanol, dicyclohexylamine, lysine, arginine, histidine, caffeine, procaine, hydrabamine, choline, betaine, ethylenediamine, glucosamine, methylglucamine, theobromine, purines, piperazine, piperidine, N-ethylpiperidine, polyamine resins and the like.

The compounds encompassed herein or their pharmaceutically acceptable salts may contain one or more asymmetric centers, chiral axes and chiral planes and may thus give rise to enantiomers, diastereomers, and other stereoisomeric forms and may be defined in terms of absolute stereochemistry, such as (R)- or (S)- or, as (D)- or (L)- for amino acids. The present is intended to include all such possible isomers, as well as, their racemic and optically pure forms. Optically active (+) and (-), (R)- and (S)-, or (D)- and (L)-isomers may be prepared using chiral synthons or chiral reagents, or resolved using conventional techniques, such as reverse phase HPLC. The racemic mixtures may be prepared and thereafter separated into individual optical isomers or these optical isomers may be prepared by chiral synthesis. The enantiomers may be resolved by methods known to those skilled in the art, for example by formation of diastereoisomeric salts which may then be separated by crystallization, gas-liquid or liquid chromatography, selective reaction of one enantiomer with an enantiomer specific reagent. It will also be appreciated by those skilled in the art that where the desired enantiomer is converted into another chemical entity by a separation technique, an additional step is then required to form the desired enantiomeric form. Alternatively specific enantiomers may be synthesized by asymmetric synthesis using optically active reagents, substrates, catalysts, or solvents or by converting one enantiomer to another by asymmetric transformation.

Certain compounds encompassed herein may exist as a mix of epimers. Epimers means diastereoisomers that have the opposite configuration at only one of two or more stereogenic centers present in the respective compound.

Compounds encompassed herein may exist in Zwitterionic form and the present includes Zwitterionic forms of these compounds and mixtures thereof.

In addition, the compounds encompassed herein also may exist in hydrated and anhydrous forms. Hydrates of the compound of any of the formulas described herein are included. In a further embodiment, the compound according to any of the formulas described herein is a monohydrate. In embodiments, the compounds described herein comprise about 10% or less, about 9 % or less, about 8% or less, about 7% or less, about 6% or less, about 5% or less, about 4% or less, about 3% or less, about 2% or less, about 1% or less, about 0.5% or less, about 0.1% or less by weight of water. In other embodiments, the compounds described herein comprise, about 0.1% or more, about 0.5% or more, about 1% or more, about 2% or more, about 3% or more, about 4% or more, about 5% or more, or about 6% or more by weight of water.

In an embodiment, a compound of formula I, more specifically UM171 or derivatives can be used alone or in combination with a AHR for the differentiation of monocytic derived AML cell lines into immature and mature functional DCs.

UM171 as the following structure:

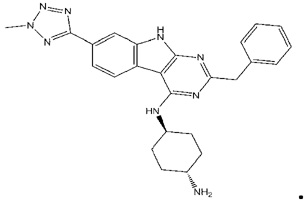

Accordingly, also encompassed are the following compounds:

| a | Structure | *EC₅₀ (nM) |
|---|---|---|
| 1 | 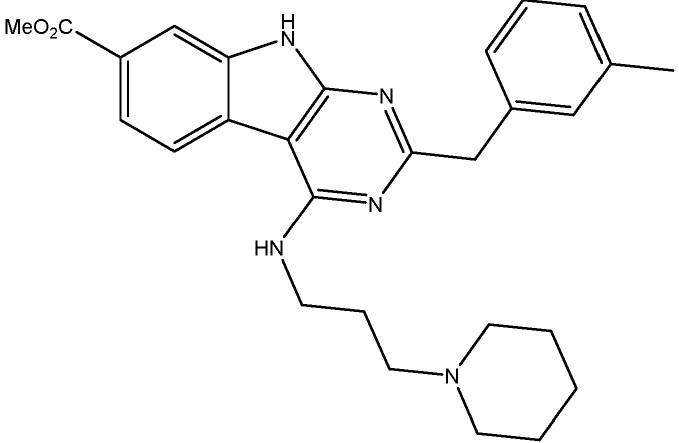 | 38 |
| 2 | 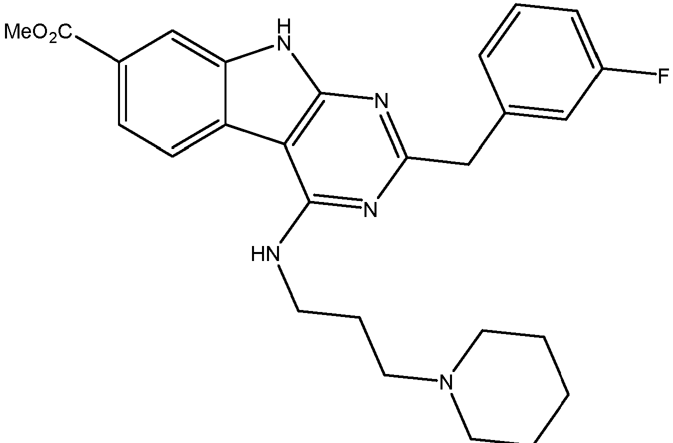 | 20 |
| 3 | 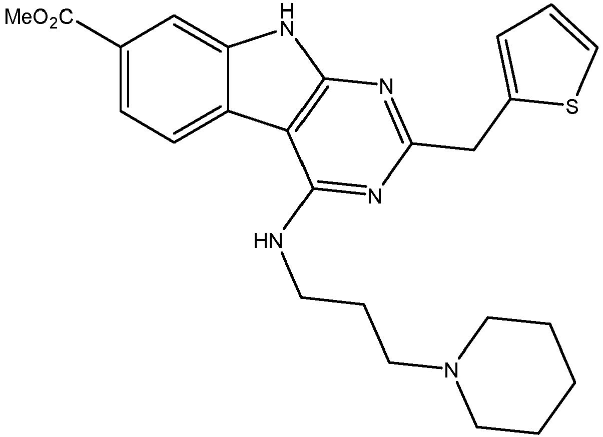 | 8,0 |
| 4 | 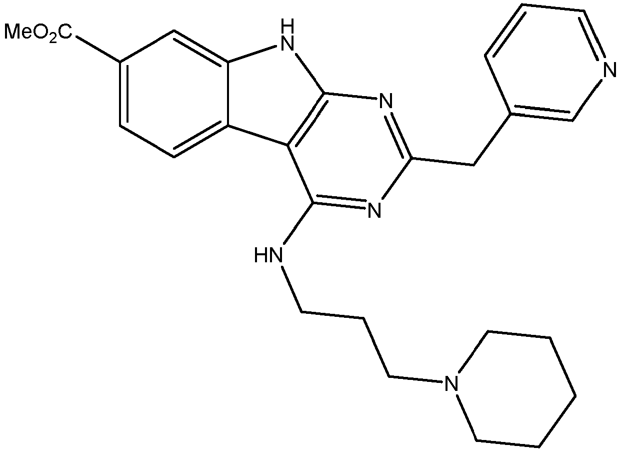 | 14 |
| 5 | 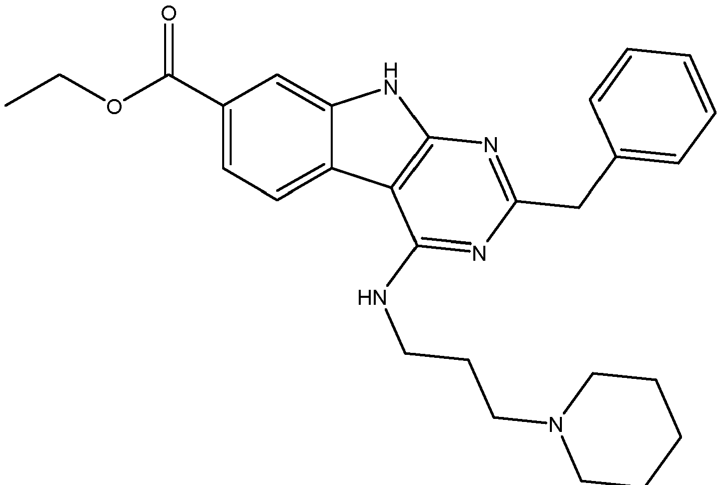 | 154 |
| 6 | 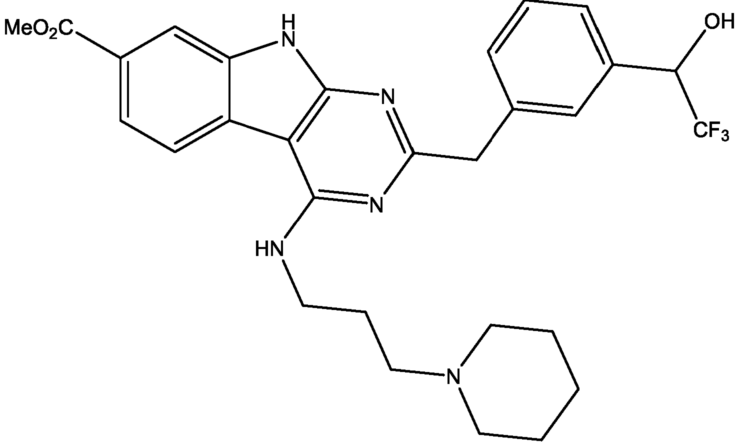 | 16 |
| 7 | 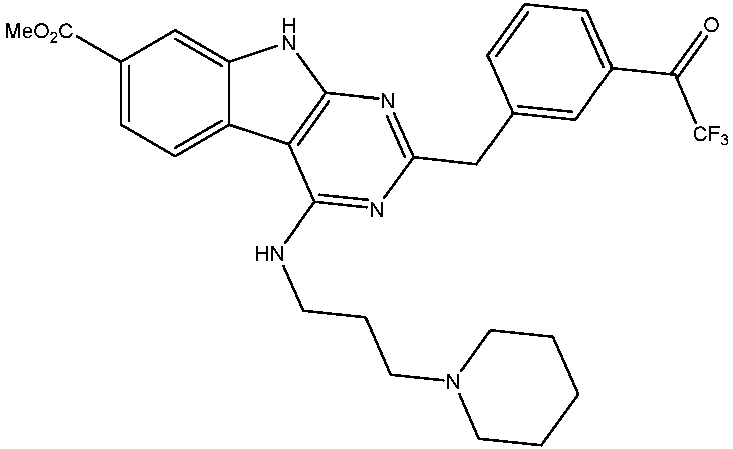 | 31 |
| 8 | 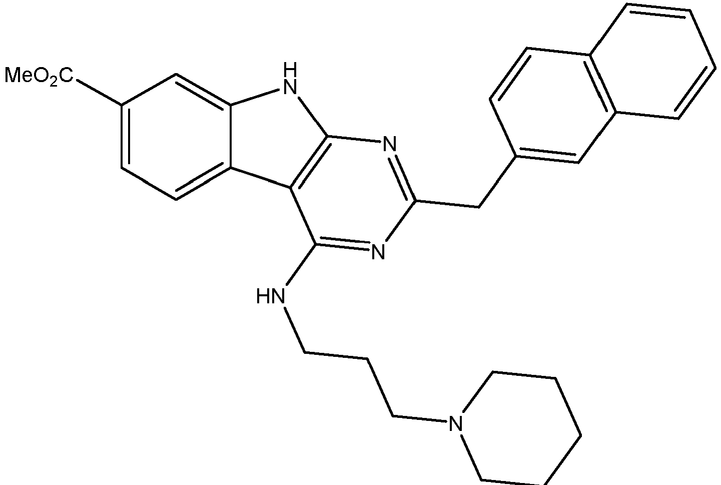 | 150 |
| 9 | 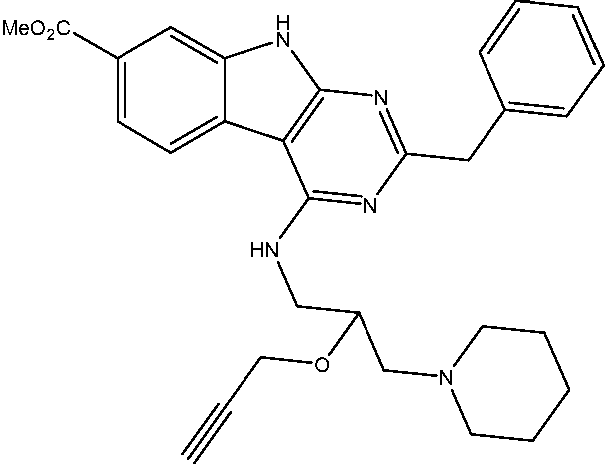 | 24 |
| 10 | 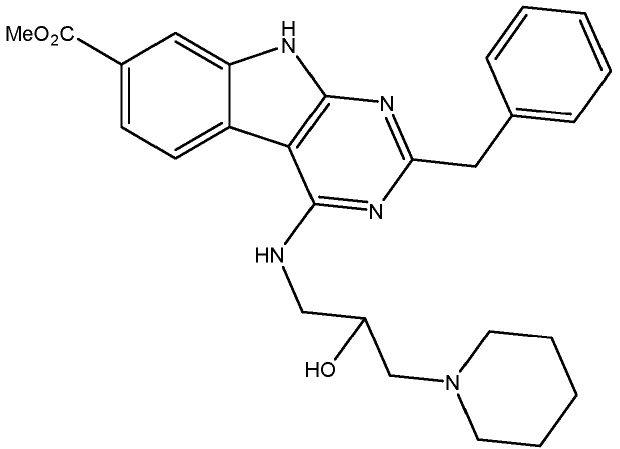 | 7,0 |
| 11 | 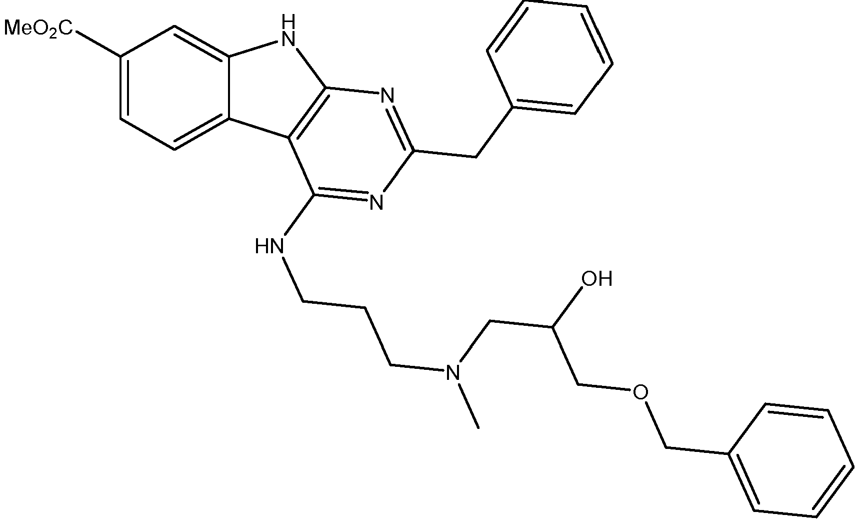 | 101 |
| 12 | 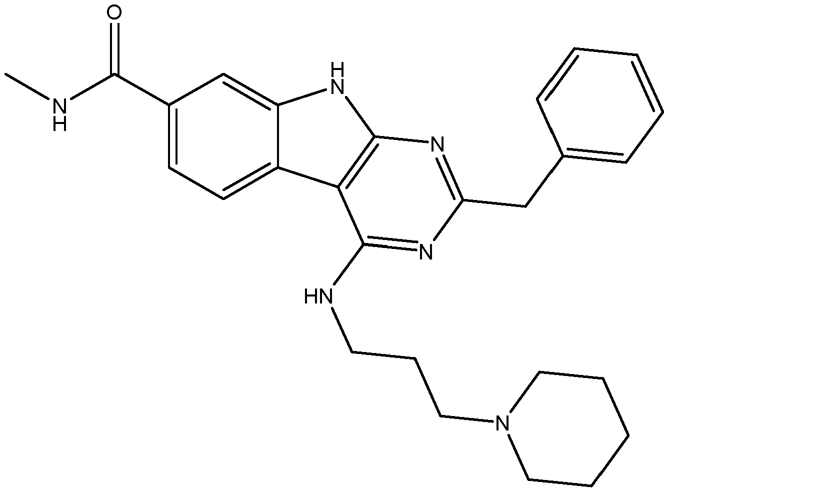 | 2445 |
| 13 | 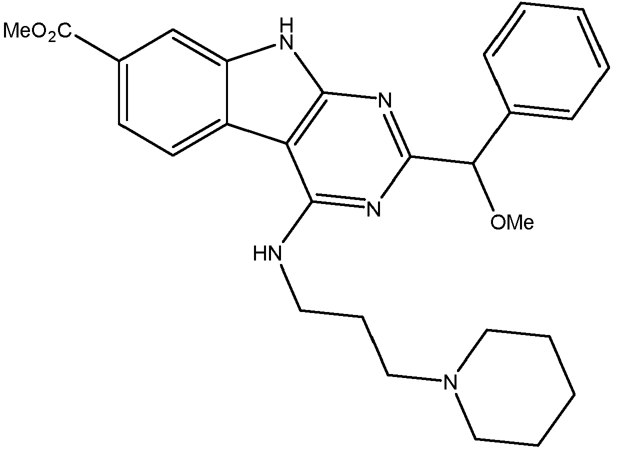 | 68 |
| 14 | 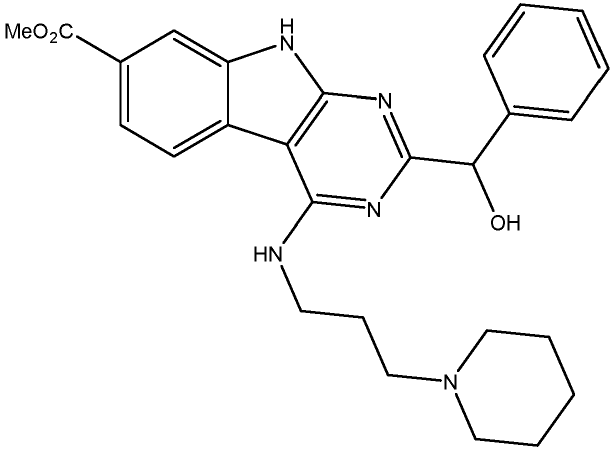 | 4,0 |
| 15 | 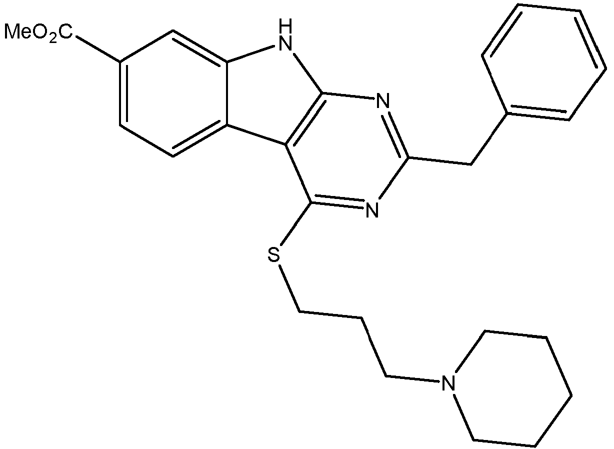 | 327 |
| 16 | 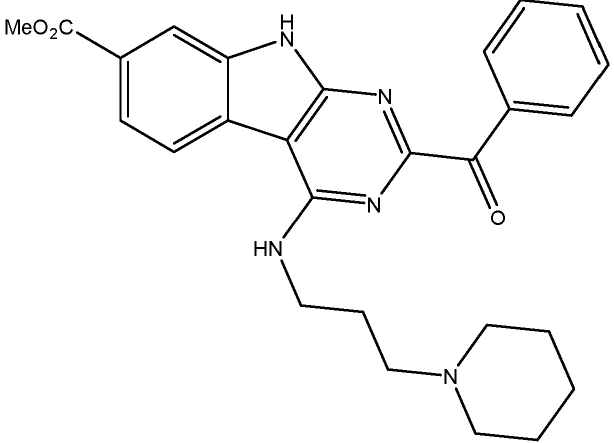 | 5,0 |
| 17 | 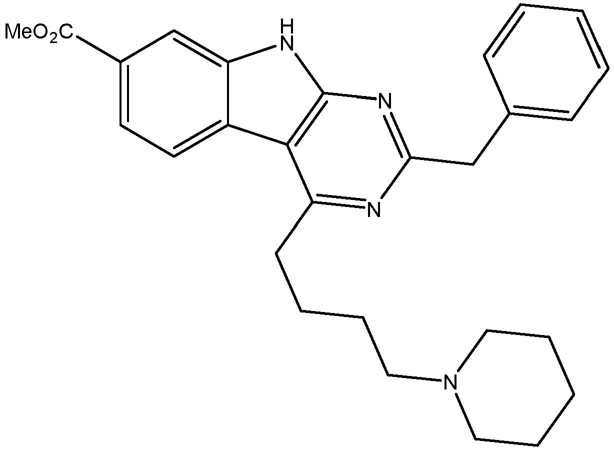 | 15 |
| 18 | 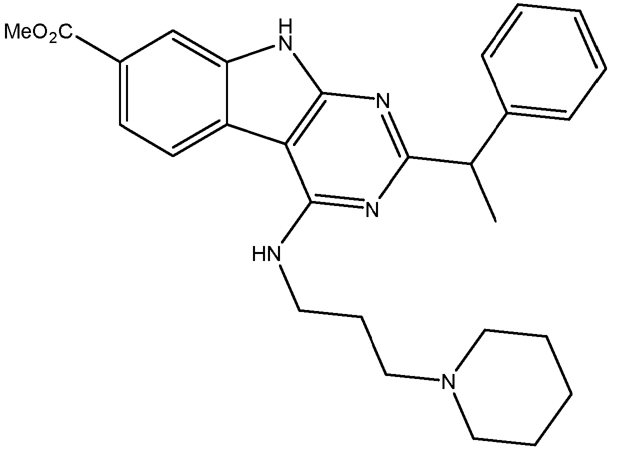 | 67 |
| 19 | 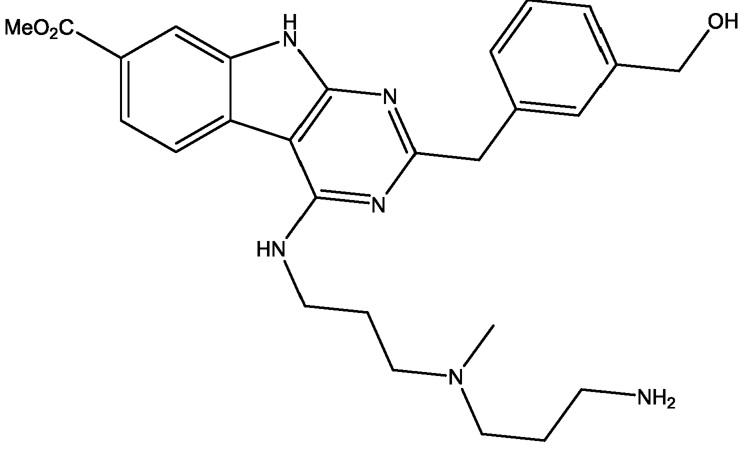 | 38 |
| 20 | 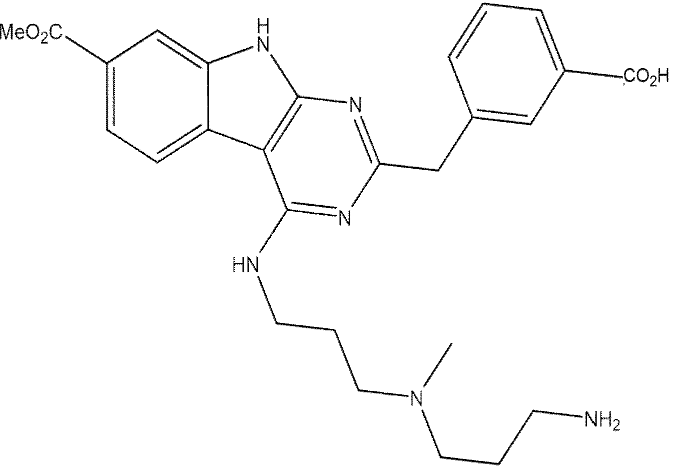 | 113 |
| 21 | 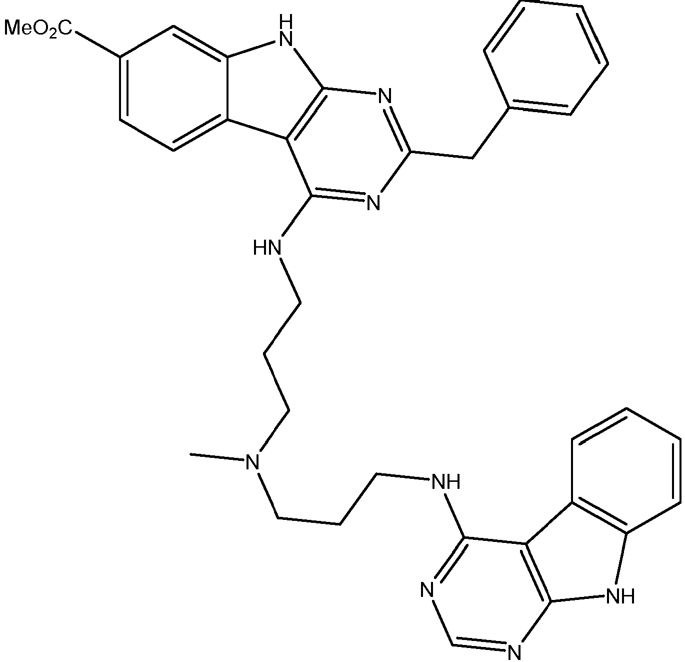 | 19 |
| 22 | 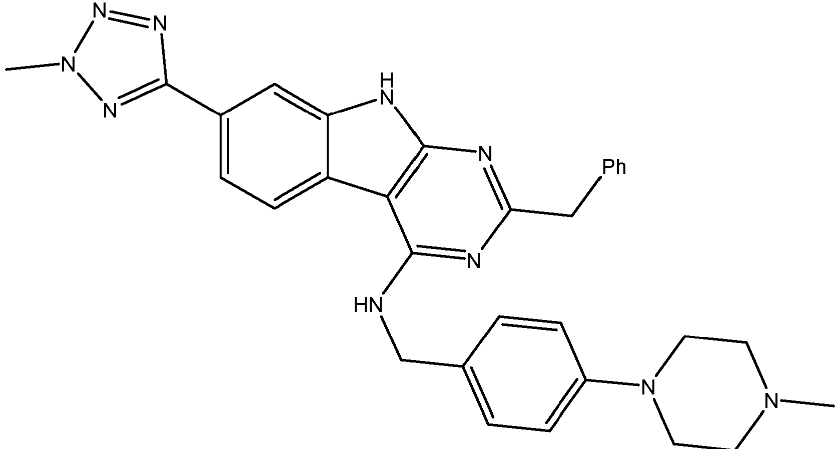 | 100 |
| 23 | 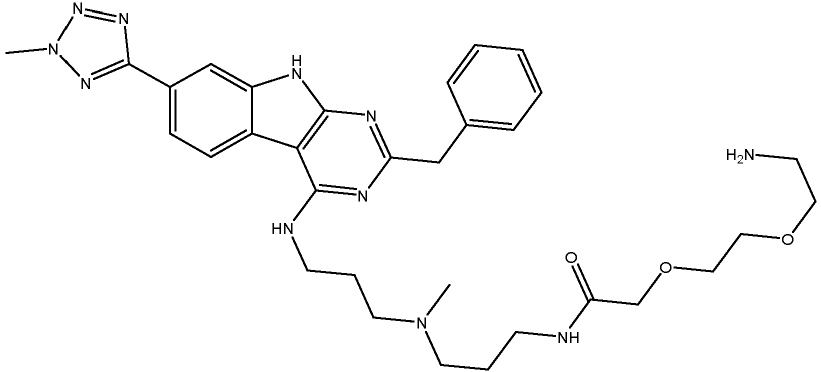 | 249 |
| 24 | 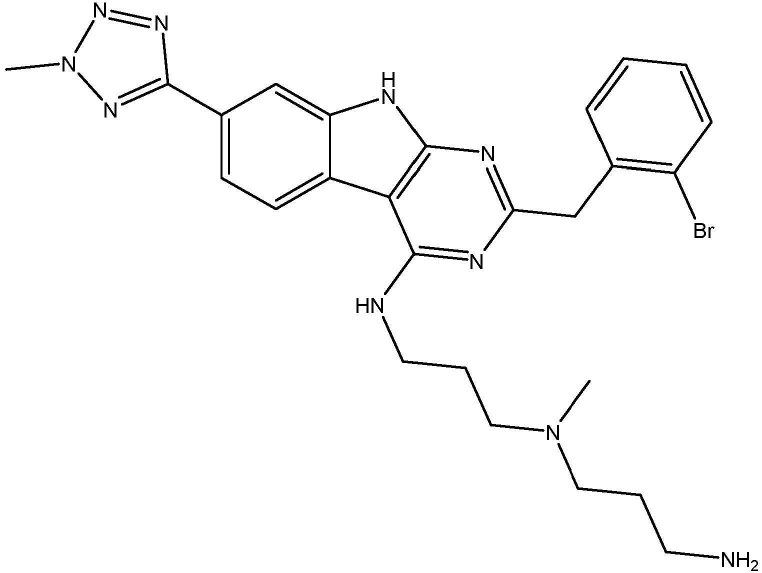 | 51 |
| 25 | 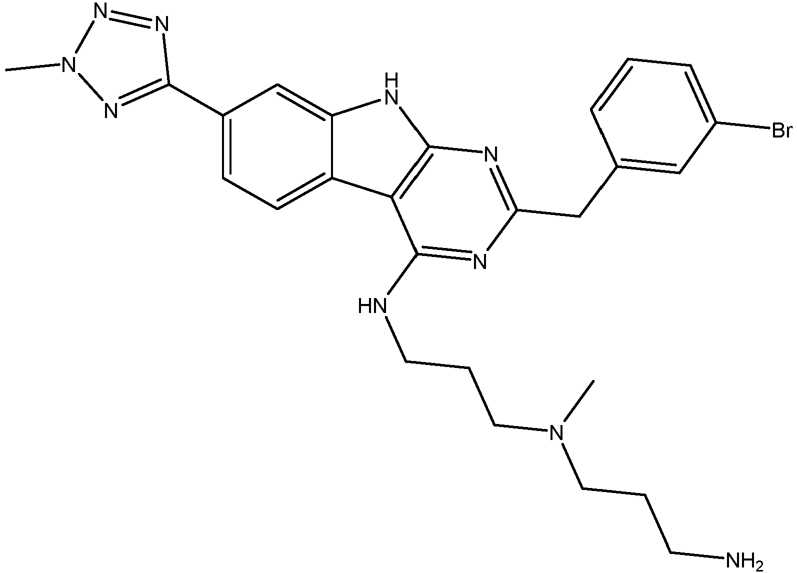 | 115 |
| 26 | 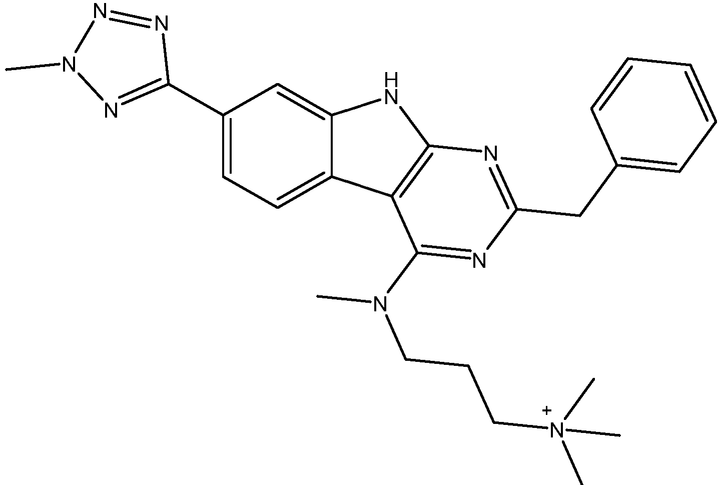 | 484 |
| 27 | 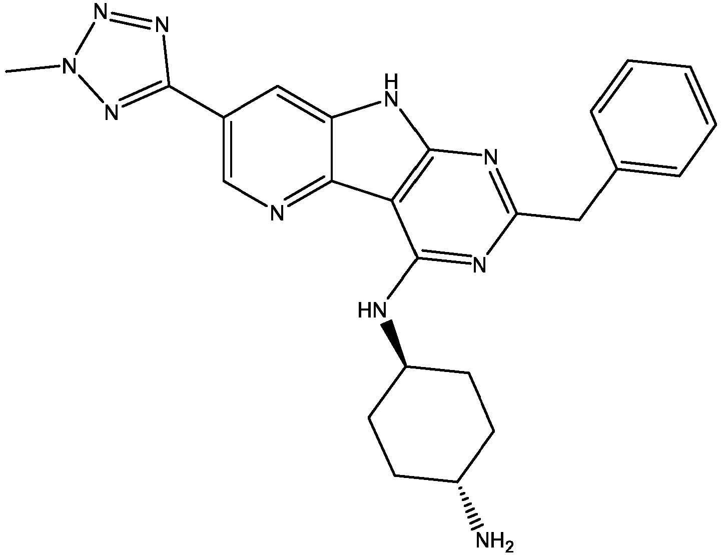 | 367 |
| 28 | 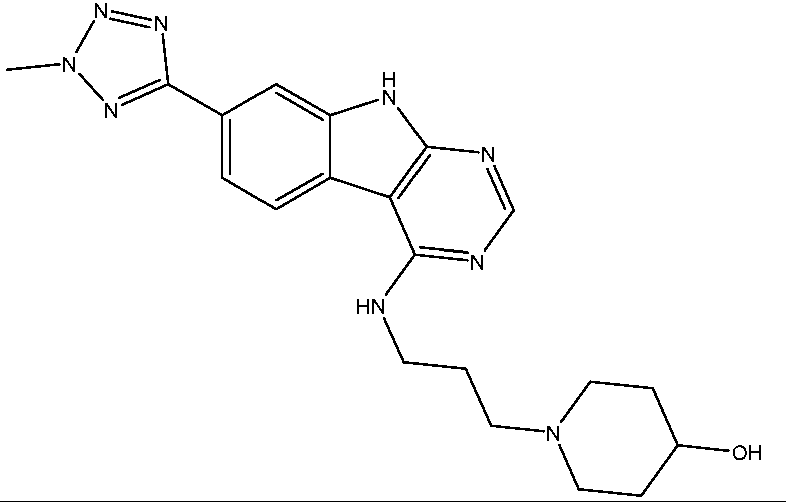 | 277 |
| 29 | 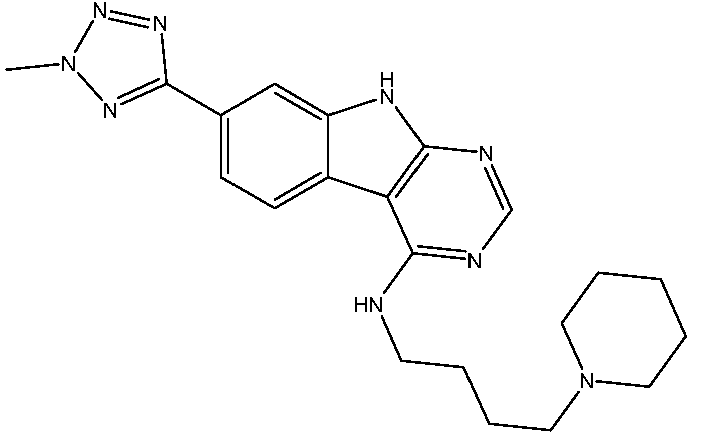 | 36 |
| 30 | 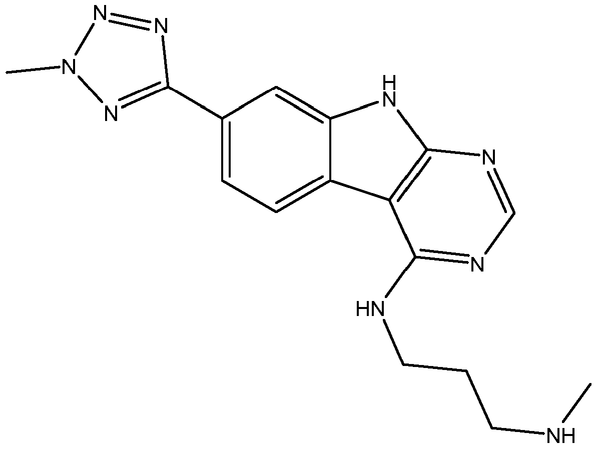 | 135 |
| 31 | 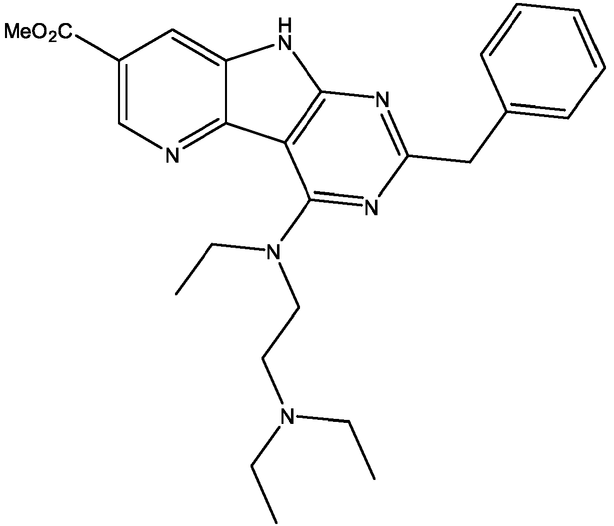 | 394 |
| 32 | 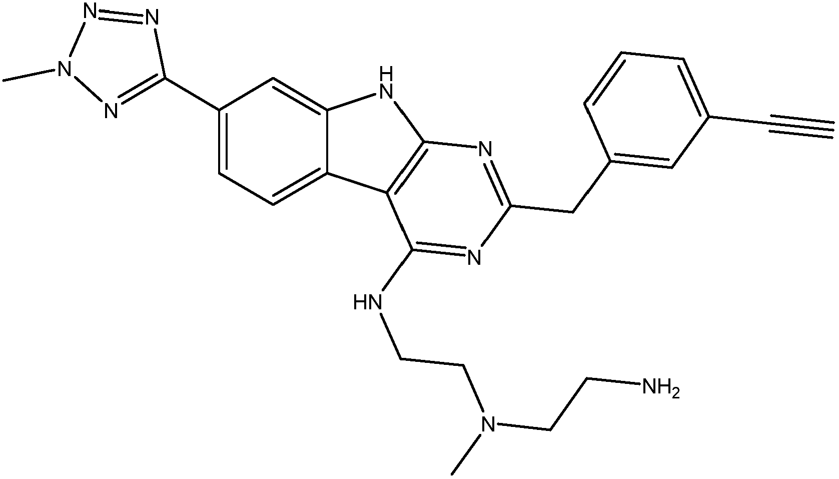 | 104 |
| 33 | 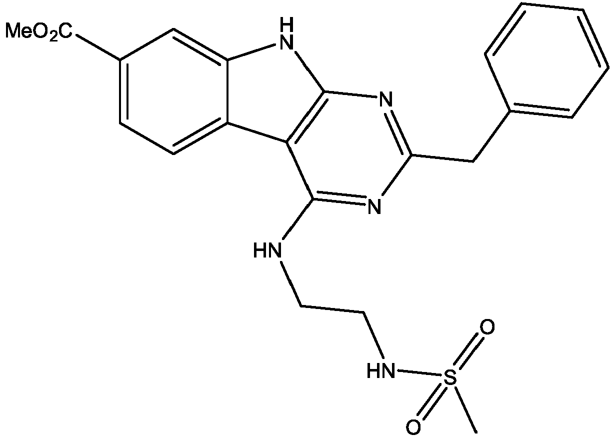 | 33 |
| 34 | 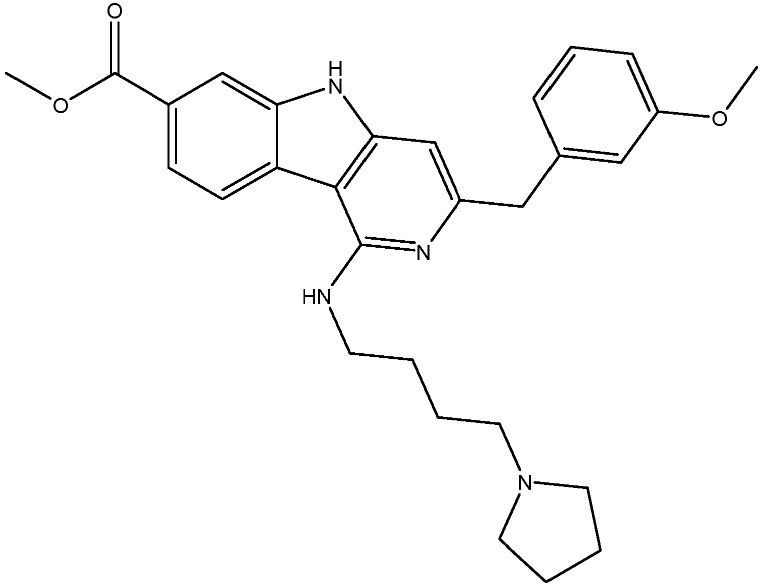 | 76 |
| 35 | 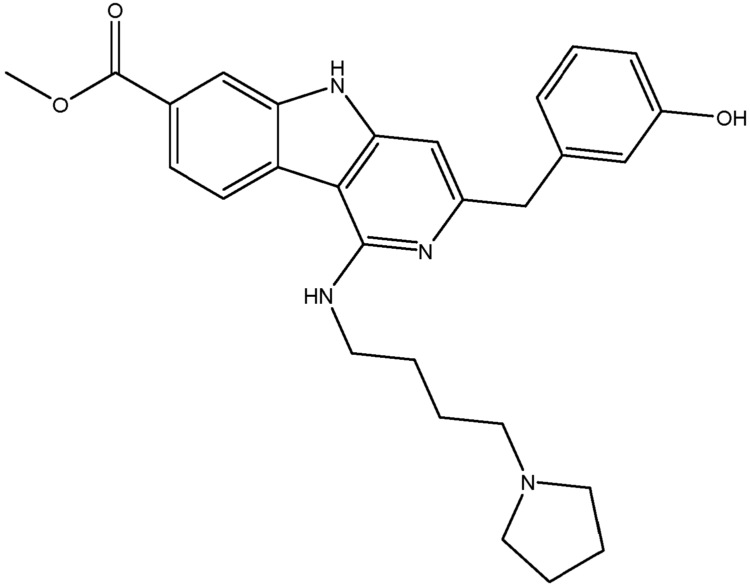 | 44 |
| 36 | 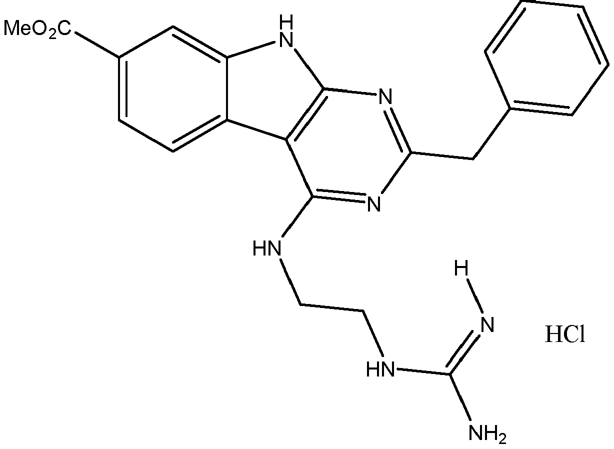 | 68 |
| 37 | 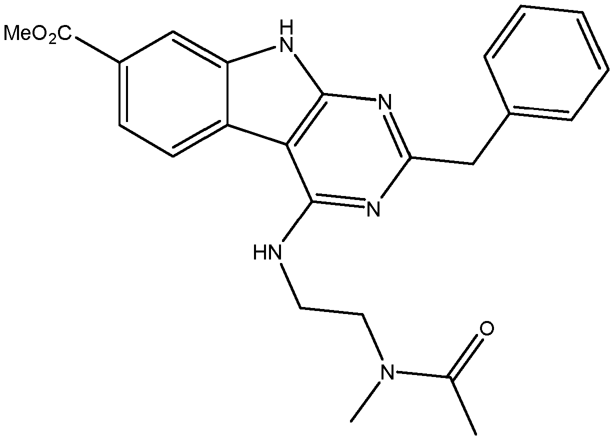 | 83 |
| 38 | 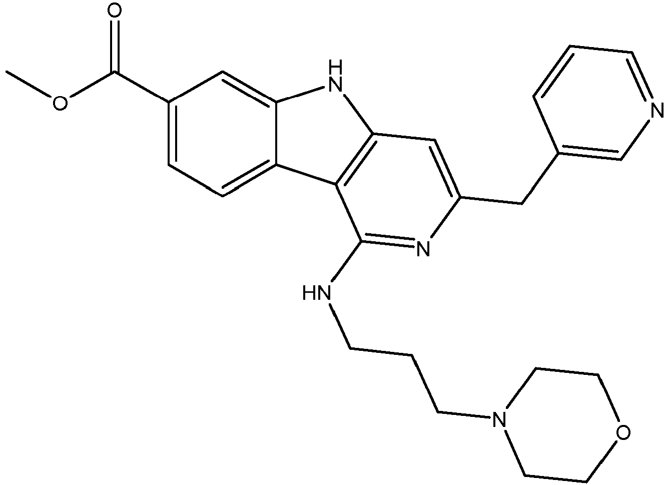 | 704 |
| 39 | 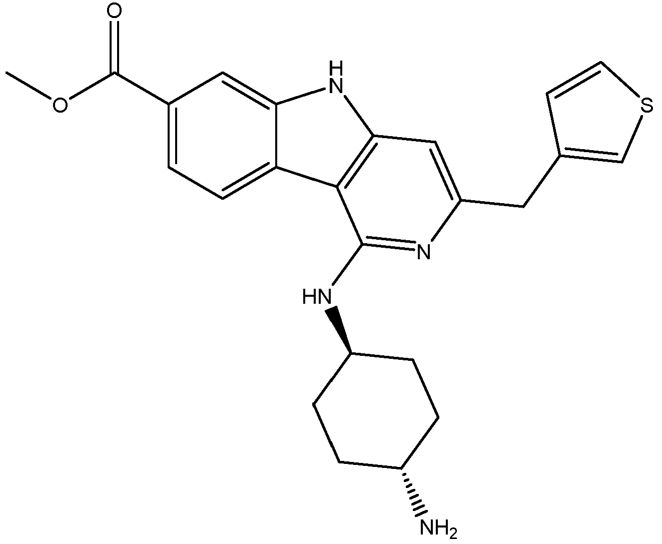 | 114 |
| 40 | 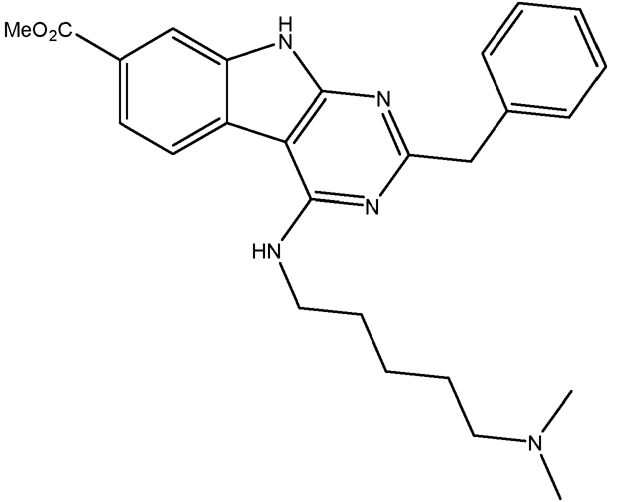 | 2,0 |
| 41 | 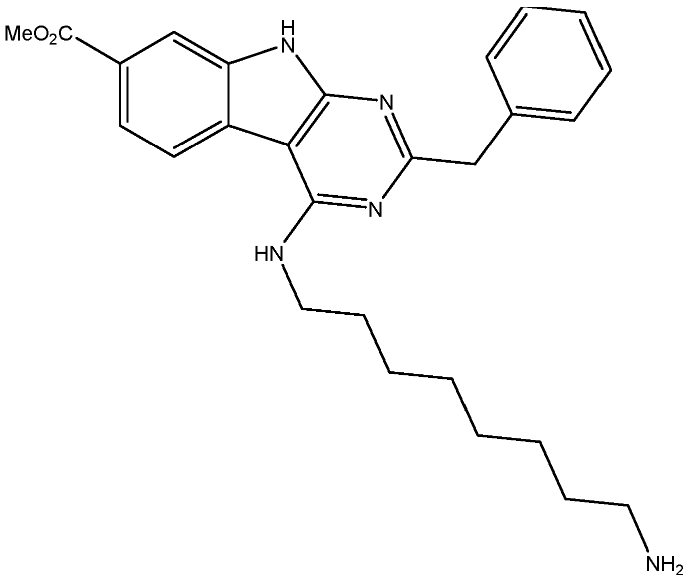 | 39 |
| 42 | 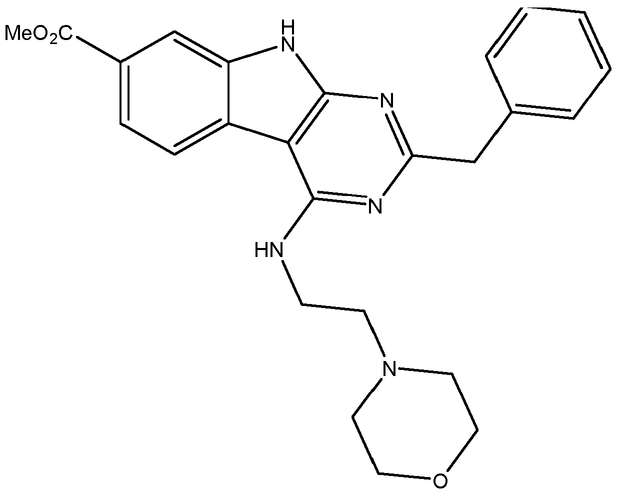 | 12,0 |
| 43 | 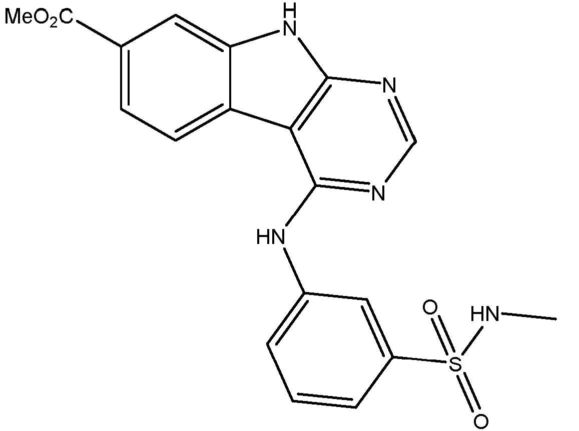 | 263 |
| 44 | 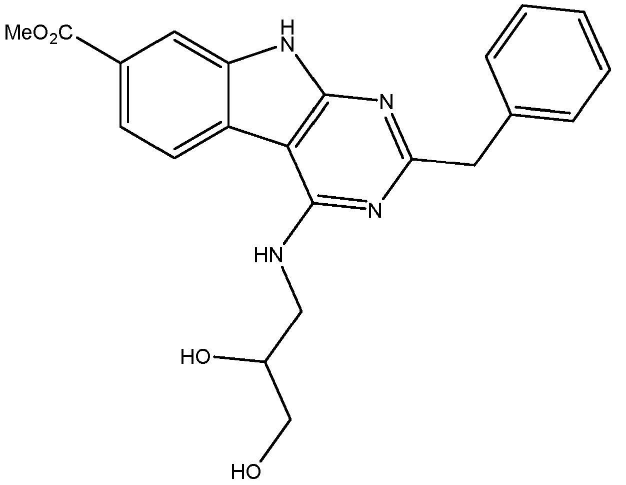 | 59 |
| 45 | 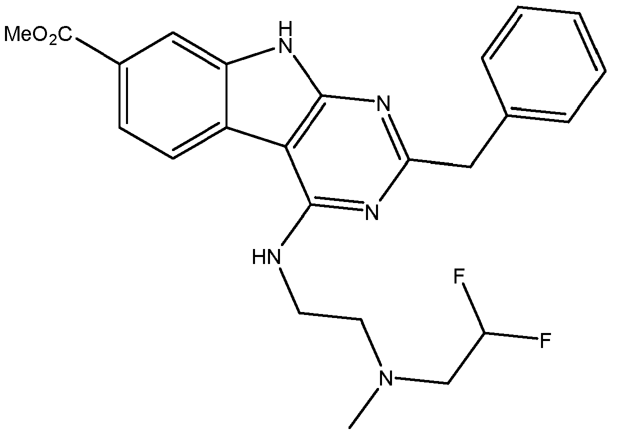 | 336 |
| 46 | 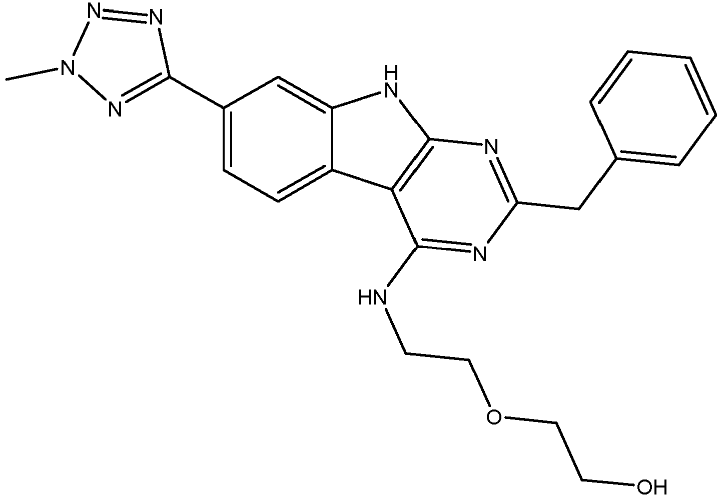 | 141 |
| 47 | 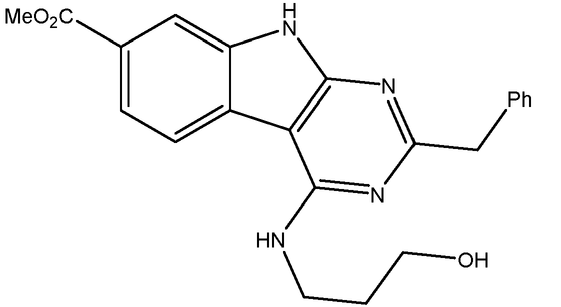 | 26 |
| 48 | 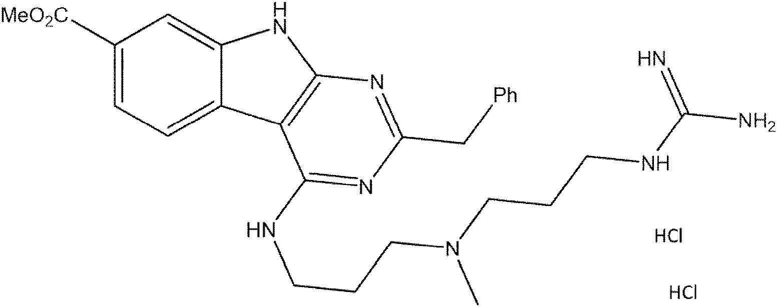 | 61 |
| 49 | 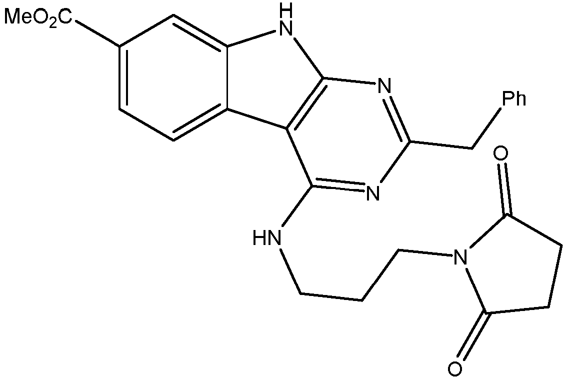 | 41 |
| 50 | 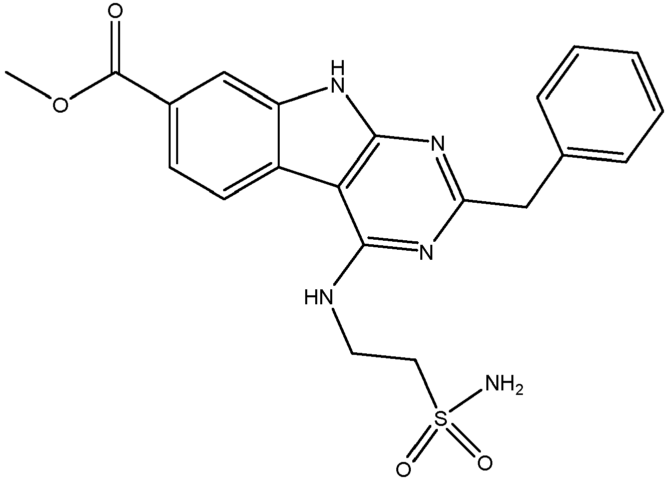 | 67 |
| 51 | 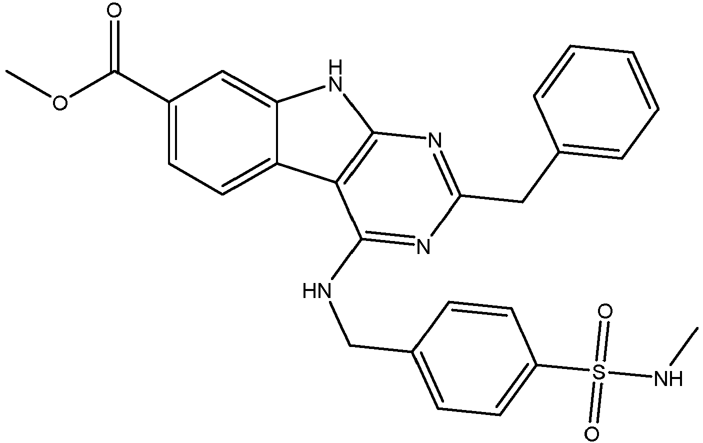 | 62 |
| 52 | 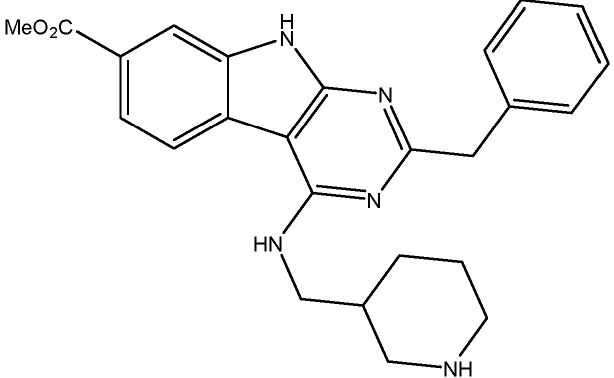 | 7,0 |
| 53 | 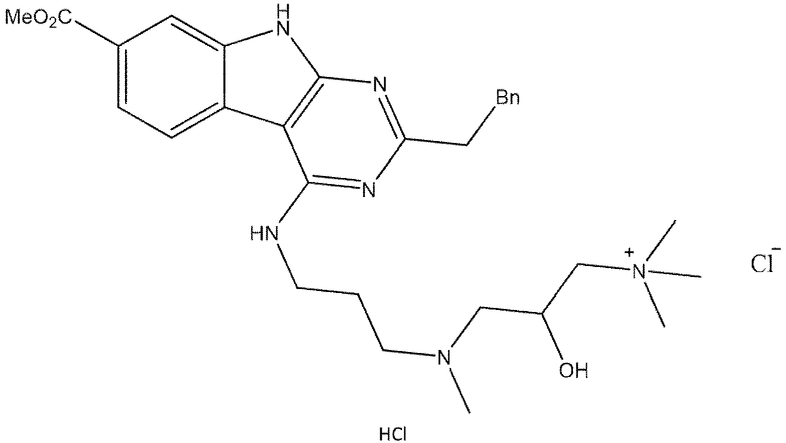 | 117 |
| 54 | 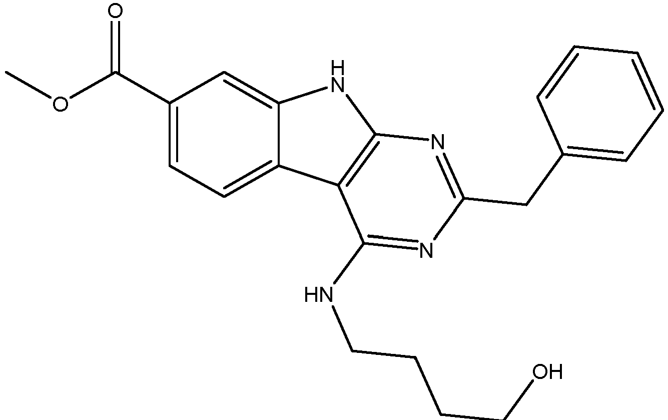 | 118 |
| 55 | 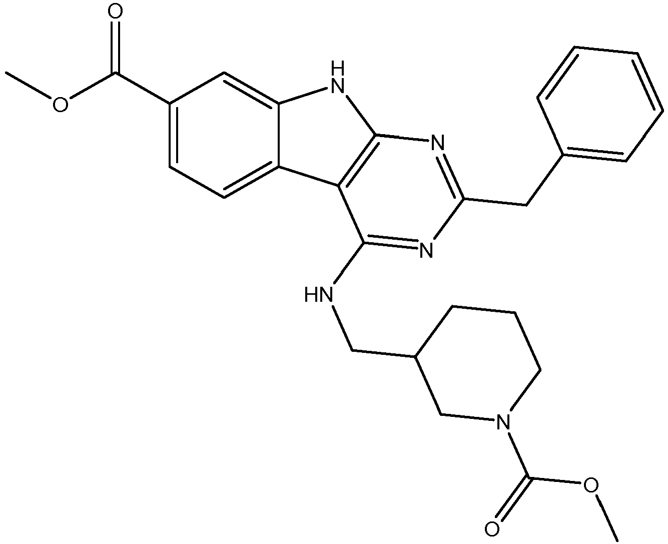 | 79,0 |
| 56 | 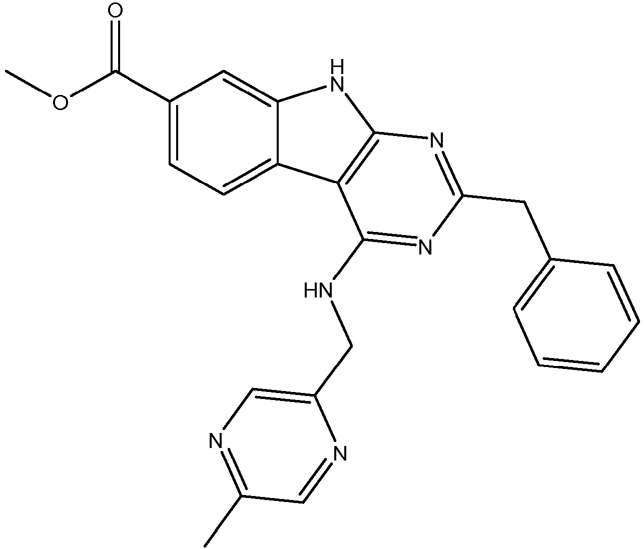 | 48 |
| 57 | 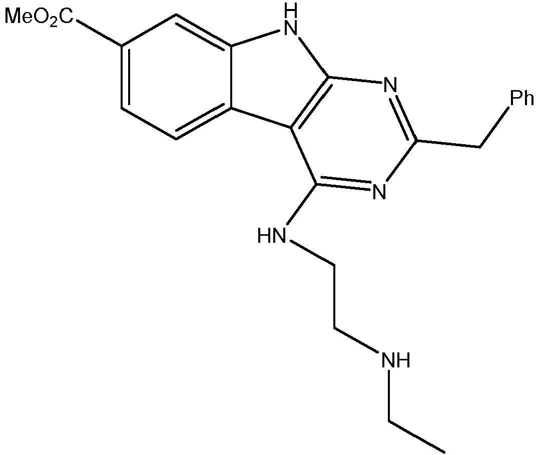 | 4,8 |
| 58 | 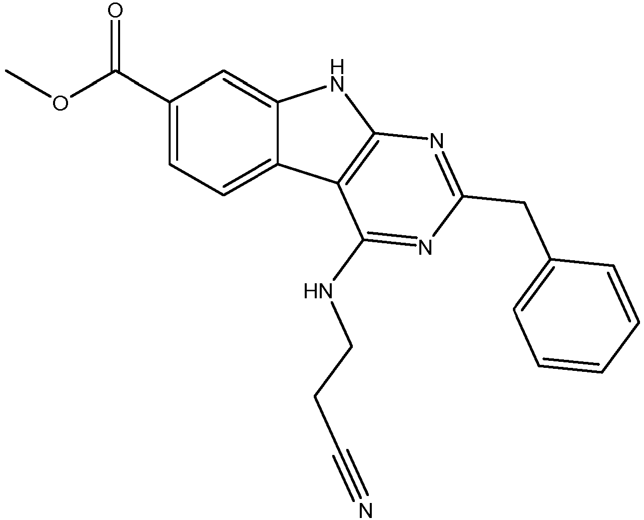 | 79 |
| 59 | 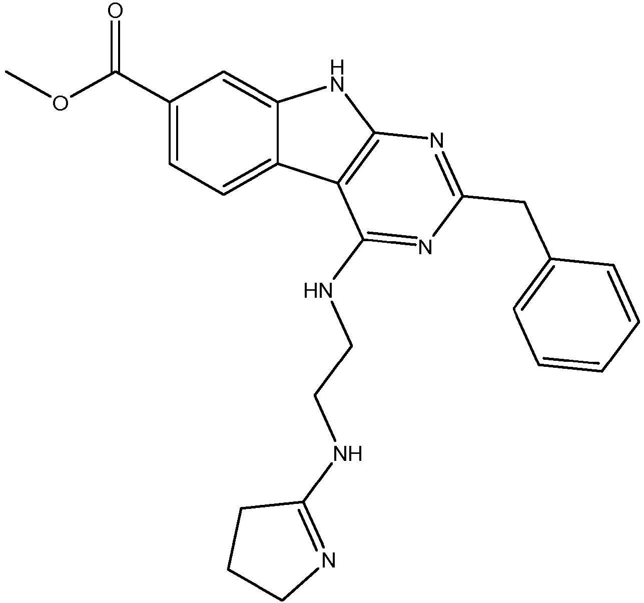 | 75 |
| 60 | 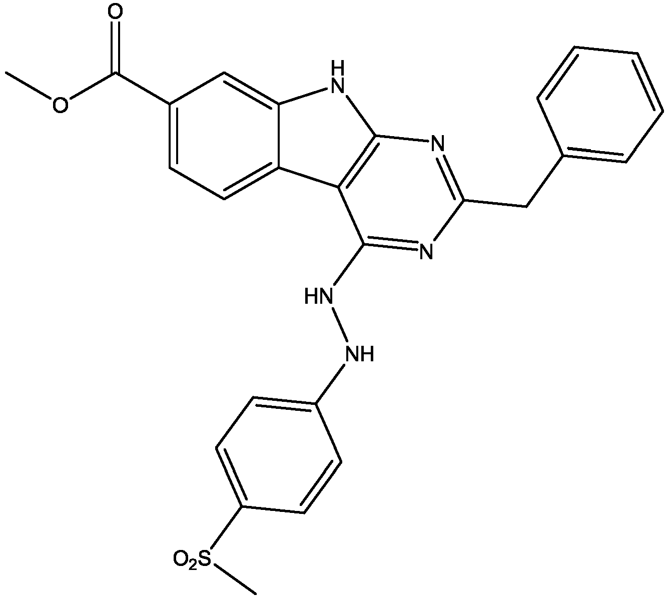 | 23 |
| 61 | 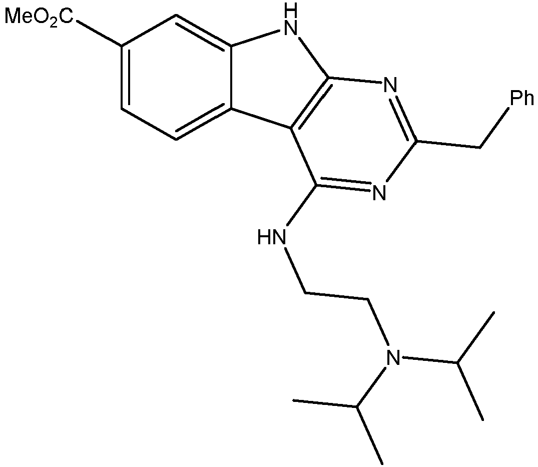 | 10,1 |

| | | |
|---|---|---|
| *EC₅₀ refers to the concentration of compound necessary to expand 50% more of CD34+CD45RA- cells as compared to cells treated with DMSO. | | |

UM171 in the fed-batch system led to significant expansion of CD34⁺CD45RA⁻ cells, an important subpopulation which encompasses all HSCs, including long-term (determined by their capacity to reconstitute NSG mice 20 to 30 weeks after transplantation) and short-term repopulating cells such as the CFU-GEMM (colony forming unit-granulocyte, erythrocyte, monocyte and megakaryocyte) progenitors which determine time to neutrophil engraftment. The CD34⁺CD45RA⁻ and not CD34⁺ phenotype represents the best surrogate parameter to monitor HSC expansion. Cells undergoing expansion in the fed-batch system with DMSO differentiate into mature cells losing CD34⁺ expression from 100% to less than 10% after 12 days.

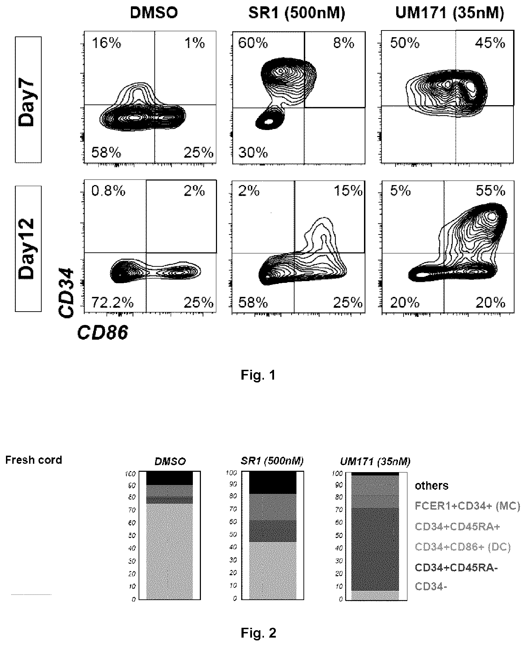

As seen in Fig. 1, when human CD34+ progenitors were purified from cord blood samples and cultured for two weeks with UM171, said compounds promotes the *ex vivo* enrichment of primitive dendritic cell progenitors and compared to SR1 for example, results in a new graft (Fig. 2). The cell populations expanded and generated upon exposure to other molecules (like SR1) are different than those obtained with UM171. The dendritic cells population (CD86⁺CD34⁺) represent 40-50% of the graft in UM171 expanded cord blood while it represent less than 5% in SR1 expanded cord blood, and are not detectable in fresh cord blood. UM171 treatment dramatically changed graft composition leading to a 500-fold increase in immature dendritic cells. Mast cells expressing the FceR1A and c-Kit were also preferentially amplified by UM171 exposure and represented approximately 10% of the graft (>8000-fold expansion).

The ability to expand short- and long-term HSCs to unprecedented levels (>1000x expansion) with UM171/fed-batch lead to prompt and durable engraftment. The safety, feasibility, engraftment and immune reconstitution in patients is described herein.

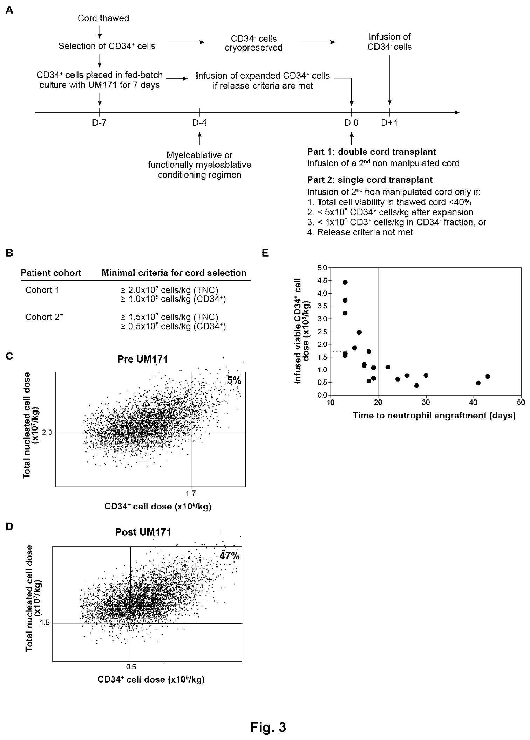

In a 22 patients cohort, the capacity of expanded CB units using UM171/fed-batch system to result in durable engraftment with neutrophil recovery <21 days was tested. Dose reduction proceeded according to Wald test based on Cox modeling which takes into consideration the strong correlation between infused CD34⁺ dose and neutrophil recovery within the clinically acceptable 21-day time point. For safety reasons, a minimum of i) 5x10⁵/kg viable CD34⁺ cells and ii) 1x10⁶/kg viable CD3⁺ T cells must be infused. Failure to achieve these values or to meet release criteria resulted in the injection of a second CB (Fig.3A).

Cohort description and pre expansion CB selection criteria are as follows:
Cohort 1 : TNC ≥ 2.0x10⁷/kg and CD34⁺ ≥ 1.0x10⁵/kg;
Cohort 2: TNC ≥ 1.5x10⁷/kg and CD34⁺ ≥ 0.5x10⁵/kg; and
Cohort 3: TNC ≥1.25x10⁷/kg and CD34⁺≥ 0.25 x10⁵/kg.
To move from cohort 1 to cohort 2, a minimum of 3 patients had to engraft within 18 days following infusion of a single UM171-expanded CB that had <2.0 x 10⁵ CD34+ cells/kg at thaw. To move from cohort 2 to cohort 3, a minimum of 3 patients had to engraft within 18 days following infusion of a single UM171-expanded CB that had <1.0 x 10⁵ CD34+ cells/kg at thaw (Fig. 3B). Cohort 2 made 47% of banked CB units available, compared to only 5% when unexpanded CB are used (Fig.3C and 3D).

Based on the strong relationship between neutrophil engraftment and infused viable CD34⁺ dose, one can extrapolate within a small cohort of patients if CD34⁺ cell dose reduction is safely achievable clinically. For example, viable CD34⁺ cell dose above 3x10⁵/kg predicts very rapid engraftment, whereas levels below 0.5x10⁵/kg lead to late engraftment (Fig. 3E).

Henceforth, 22 patients (numbered 1-22) received a single UM171 cord (N=22). Median follow-up is 18 months (range 1-28 months for patients 1-22). Results were compared to same institution continuous transplanted patients with unmanipulated cord blood (Ctrl CB). Infusion of ≥ 1x10⁵ viable CD34⁺ cells/kg was effected as this ensured neutrophil engraftment within 21 days (Fig. 3E). In addition, only CB controls who received the same conditioning regimen without ATG as patients in the current trial were included in the control group.

The CB selection process was standardized to avoid any variability. The CB selected for the study were compared to CB(s) selected if patient were not on protocol. This allowed demonstration of improvement in HLA match and decrease in need for double CBs.

CB for expansion were purchased and shipped to manufacturing center (FHCRC for 1^{st} 10 patients and CETC for the next 15 patients). The CB were thawed and underwent CD34⁺ cell selection; the CD34⁺ cells were cultured in the fed-batch for 7 days with growth factors, then washed and cryopreserved were shipped to the transplant center. The CD34⁻ cells were cryopreserved and infused to the patient at transplant. All cell products were assessed for viability and phenotype at every step and release criteria include sterility, mycoplasma, endotoxin, minimal 10 fold CD34⁺ expansion and viability >70%.

Patients received a predefined myeloablative conditioning regimen (Barker et al., 2005, Blood, 105: 1343-1347; Oran et al., 2001, Biology of blood and marrow transplantation: Journal of the American Society for Blood and Marrow Transplantation, 17: 1327-1334). On the day of transplant, expanded CB were infused, while CD4-product was infused the next day. GVHD prophylaxis consisted of mycophenolate mofetil and cyclosporine. Patients are followed for a minimum of 3 years to ensure the absence of any unexpected complication. Patients are receiving standardized supportive care.

As can been seen in Table 1 below, cells population were measured at day 14, 21, 28, 56, 100, at 6 months and 12 months post transplantation of the graft. Antibodies panels were chosen to be measured to allow the evaluation of both myeloid and lymphoid lineages engraftment. Particularly:
- CD45⁺CD3⁺ are lymphoid T cells
- CD56⁺NKP46⁺ or NKG2A⁺ are known NK cells.

Table 1 express the number of cells measured per µl of blood µl/blood ± 5 %.

**Table 1**

| Expansion of NK cells measured in patient after cord blood transplant expanded with UM171 | | | | | | | |
|---|---|---|---|---|---|---|---|
| **Average of all Patients** (µl/blood ± 5 %) | **J14** | **J21** | **J28** | **J56** | **J100** | **M6** | **M12** |
| CD45+ CD3+ | 0,1159 | 0,0773 | 0,1208 | 0,8568 | 0,9113 | 1,0792 | 2,5205 |
| Activated CD3+ | 0,0014 | 0,0023 | 0,0021 | 0,0631 | 0,0261 | 0,0102 | 0,0247 |
| CD3+ CD117+ | 0,0157 | 0,0010 | 0,0019 | 0,0163 | 0,0111 | 0,0082 | 0,0098 |
| CD3+ FCER1+ | 0,0257 | 0,0003 | 0,0006 | 0,0094 | 0,0031 | 0,0027 | 0,0033 |
| CD3+ NKG2A+ | 0,0307 | 0,0045 | 0,0040 | 0,0283 | 0,0164 | 0,0248 | 0,0681 |
| CD45+ CD3- | 0,6191 | 0,9127 | 2,5961 | 9,0394 | 7,3954 | 5,6145 | 3,5695 |
| Activated CD45+ CD3- | 0,0025 | 0,0133 | 0,0344 | 0,1120 | 0,0676 | 0,1011 | 0,0560 |
| CD16/56- NKP46- | 0,3336 | 0,3959 | 1,0043 | 5,9586 | 4,4629 | 2,9746 | 2,4824 |
| CD16/56+ NKP46+ | 0,0672 | 0,1912 | 0,6216 | 1,4386 | 1,0450 | 0,5541 | 0,3086 |
| CD16/CD56- NKP46+ | 0,0168 | 0,0610 | 0,0892 | 0,1516 | 0,0476 | 0,0289 | 0,0190 |
| NK cells | 0,0643 | 0,1938 | 0,5967 | 1,2330 | 0,8180 | 0,4652 | 0,2640 |
| Immature cells (Prog. CD117+) | 0,0737 | 0,0650 | 0,0972 | 0,8225 | 0,8097 | 0,9186 | 0,0647 |
| Inflammation APC (FCER1+) | 0,0080 | 0,0034 | 0,0116 | 0,0671 | 0,0719 | 0,0898 | 0,0557 |
| Neutrophil/macrophage/mastocyte | 0,2136 | 0,3059 | 0,9172 | 1,5991 | 1,8399 | 2,0569 | 0,7595 |
| CD45+ CD3- NKG2A+ | 0,0895 | 0,2119 | 0,6372 | 1,3776 | 0,9513 | 0,7242 | 0,5500 |
| Nb CD45+ acquired | 94438 | 112353 | 117663 | 80936 | 74282 | 142387 | 162494 |
| TOTAL µl/blood | 0,1567 | 0,4031 | 1,2588 | 2,8162 | 1,9962 | 1,2783 | 0,8586 |

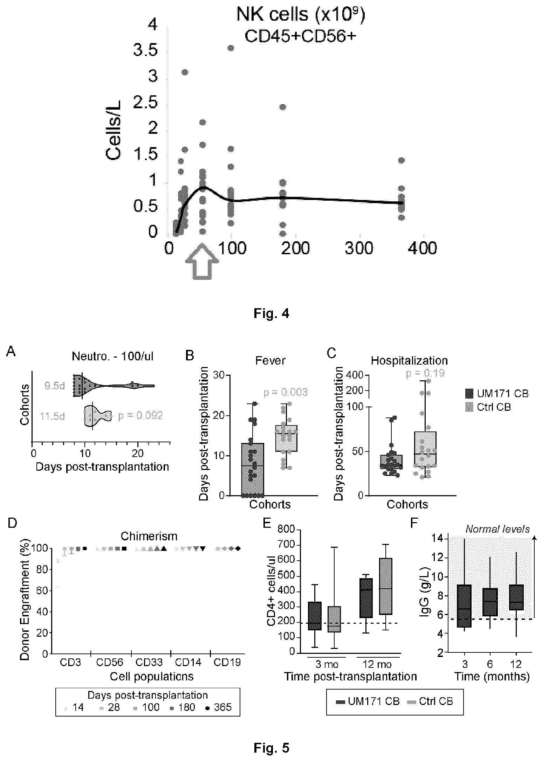

As seen, a flare or expansion of NK cells was measured *in vivo* in patients between day 21 and coming back to initial numbers at 12 months post transplantation. As seen in Fig. 4, the expansion of NK cells measured in patients is significantly higher at two months post-engraftment (see arrow in Fig. 4) than what has been reported (see Lucchini et al., 2015, Cytotherapy, 17: 711-722).

When CD11c⁺CD14⁺ and CD11c⁺CD14⁻ cells levels were measured, which are two subsets of dendritic cells, a similar flare or expansion is observed (Table 2).

**Table 2**

| Expansion of DC cells measured in patients after cord blood transplant expanded with UM171 | | | | | | | |
|---|---|---|---|---|---|---|---|
| **Average of all Patients** (µVblood ± 5 %) | J14 | J21 | J28 | J56 | J100 | M6 | M12 |
| CD45+ / singlet2 | - | - | - | - | - | - | - |
| CD45+ CD3+ | 0,14 | 0,07 | 0,12 | 0,72 | 0,87 | 1,33 | 2,67 |
| CD45+ CD3- | 0,60 | 0,92 | 2,59 | 9,18 | 7,44 | 5,37 | 3,42 |
| CD11c- CD14- | 0,21 | 0,35 | 0,92 | 3,88 | 4,12 | 3,00 | 2,42 |
| CD11c- CD14+ | 0,30 | 0,36 | 1,00 | 3,01 | 0,43 | 0,55 | 0,17 |
| CD11c+ CD14- | 0,01 | 0,04 | 0,06 | 0,29 | 0,55 | 0,63 | 0,31 |
| CD11c+ CD14+ | 0,0819 | 0,1611 | 0,6136 | 1,9942 | 2,3338 | 1,3920 | 0,5140 |
| HLA-DR+ | 0,01 | 0,02 | 0,03 | 1,04 | 1,44 | 1,10 | 1,57 |
| pDC (CD123+ BDCA2+) | 0,00 | 0,00 | 0,01 | 0,03 | 0,01 | 0,01 | 0,02 |
| pDC CD86 low/int | 0,00 | 0,00 | 0,00 | 0,01 | 0,00 | 0,00 | 0,00 |

The *in vivo* flare in natural killer cells and dendritic cells measured in patients transplanted with UM171 expanded cells have an immunosuppressive activity and prevent GVH reaction and allow graft tolerance. The immune cell recovery is faster with UM171 expanded cord, which is important for decreasing post-hematopoietic cell transplant (post-HCT) infections and relapse. Accordingly, UM171 promotes the expansion of HSCs and of progenitor cells by coordinating pro- and anti-inflammatory responses and help controlling the inflammatory state.

Cumulative incidence of neutrophil recovery was 100% with a median time to 100 and 500 neutrophils of 9.5 (8-23) and 18 (10-30) days, respectively (Fig. 5A). Median time to 100 neutrophils for Ctrl CB was 11.5 days, while time to 500 neutrophils was 19 days. Interestingly, achieving 100 neutrophils was independent of CD34 cell dose. No late graft failure developed and almost all patients had platelets ≥100, neutrophils ≥1500 and hemoglobin ≥100 at 6 and 12 months post-transplant. Prompt engraftment resulted in faster resolution of fever post-transplantation and shorter hospitalization time for UM171 patients (Fig. 5B and 5C).

Chimerism analysis of different cell populations, notably CD3, CD56, CD19, CD14, and CD33 revealed that patients rapidly achieved 100% donor engraftment in all cell lines (Fig. 5D).

After more than 10 months of follow-up, it is reported that out of 16 patients, only 2 patients with grade III acute GVHD was observed (which promptly responded to steroids) and no case of moderate to severe chronic GVHD was observed. Interestingly, few viral infectious complications (CMV, EBV, adenovirus cystitis for example) was observed indicating a robust immune response in these patient. A spontaneous resolution of EBV viremia was observed in 2 out of 3 patients which were infected prior to the transplantation (Table 3).

**Table 3**

| Spontaneous resolution of EBV viremia in patients | | | |
|---|---|---|---|
| | Patient 6 | Patient 9 | Patient 12 |
| 1^{st} positive EBV viremia | 30,000 | 15,488 | 33,113 |
| Day of 1^{st} dose rituximab | 0 | 4,266 | 190,546 |
| Day of 2nd dose rituximab | 0 | 427 | 0 |

| | | | |
|---|---|---|---|
| Threshold of rituximab therapy is 10,000 copies | | | |

Accordingly, 11 patients were positive for CMV serology and 6 developed viremia requiring therapy but no case of CMV disease occurred. Two cases of early onset adenovirus cystitis occurring prior to D+25 required therapy with intravenous cidofovir. Three patients received rituximab for EBV viremia. There was no case of PTLD. Two cases of Pneumocystis jiroveci pneumonia (PJP) occurred, the first patient was non-compliant with prophylaxis for > 2 months and the second was on atovoquone prophylaxis in an institution where atovaquone resistant PJP is present. There were 2 cases of dermatomal shingles, both while off prophylaxis. No case of invasive fungal infection, toxoplasmosis, or HHV6 infection was ascertained. Median CD4/µL at 3, 6 and 12 months were 196, 301 and 413, respectively (Fig. 5E). Only 1 patient did not achieve a CD4 count of 50 by D+100. Median IgG at 3, 6, 12 months were 5.9, 7.3 and 7.3 g/L, respectively (Normal 5.5-16.3) (Fig. 5F).

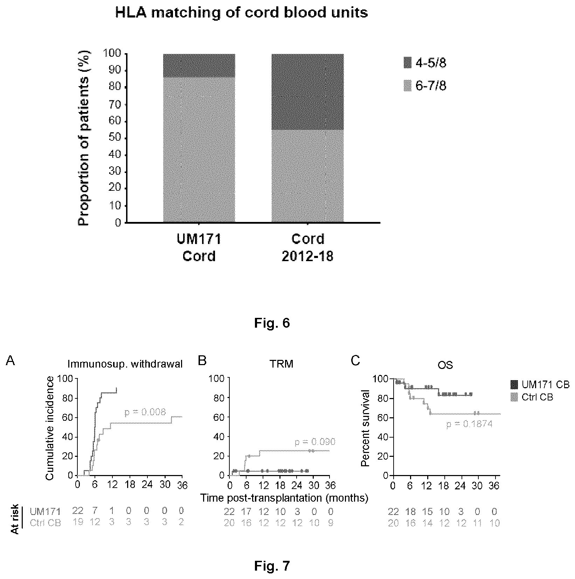

When compared to standard selection criteria for cord blood (TNC ≥2.0x10⁷/kg and CD34 ≥1.7x10⁵/kg), 12/22 patients got a better HLA matched cord as minimal cell dose criteria was lower. Thus >75% of the patients were transplanted with a CB HLA matched 6-7/8 (5 5/8, 15 6/8, 2 7/8) (Fig. 6). No patient was excluded from the trial because a HLA matched CB with sufficient cell dose could not be identified.

At 12 months post-transplant, immunosuppressive therapy had been discontinued in 85% of UM171 CB vs 72% of Ctrl CB patients (Fig. 7A). Overall, 1 patient has died of diffuse alveolar hemorrhage, for a cumulative incidences of TRM at 1 one year of 5% (95%CI: 1-31%) in UM171 CB cohort compared to 25% (95%CI: 12-54) in Ctrl CB cohort (Fig. 7B). OS at 12 months was 90% (95%CI: 67-98), compared to 75% (95%CI: 49-87) in Ctrl CB (Fig. 7C).

It is demonstrated herein that transplanting patients with a single, smaller, better HLA matched UM171 expanded CB would lower the risk of morbidity and mortality of CB transplantation. To achieve this goal, a phase I-II clinical trial was conducted and primary endpoints of feasibility (expansion failure 1 cord with suspicion of inherent underlying abnormality), safety with 5% TRM, and capability of using smaller, better HLA matched cords with prompt engraftment was demonstrated.

Furthermore, prompt (<D+18), robust and durable neutrophilic engraftment was ensured without any graft failure. Furthermore, very early recovery of 100 neutrophils (D+10) was achieved leading to rapid resolution of febrile neutropenia (D+7.5) and shorter hospitalization (35 days for UM171 CB vs 47 days for CB controls), similar to that seen with conventional allogeneic transplants (29.5 days for BM and 33 days for PB). One would expect an earlier discharge for UM171 patients compared to BM-PB because of prompter 500 neutrophil recovery.

Cord blood is traditionally thought to be associated with poor and delayed T cell immune reconstitution at least in part due to the naivety of the infused T cells and lack of memory T cells contributing to an increased risk of viral infections. Despite lack of transfer of memory T cells and an average T cell loss of 33% with the described expansion procedure, CD4 recovery is at least as fast compared to unmanipulated CB transplant (Fig.4E). CD4 recovery has been shown to be important not only for prevention of infectious complications but also TRM and relapse. In pediatric CB transplantation, Admiraal et al. (2016, Blood, 128: 2734-2741) reported that if a CD4 count of 50 is not achieved by D+100, the risk of relapse was 100% for AML (vs. 24%), TRM 31% (vs. 11%), and OS 56% (vs. 73%). In the cohort of 22 patients described herein, only one had not achieved a CD4 of 50 by D+100. Prompt immune recovery in the trial translated in absence of severe viral infections with no CMV disease or PTLD. Furthermore, 2/3 patients who were treated for EBV viremia had already improved EBV titers below threshold before receiving rituximab.

Expected TRM in CB transplantation is higher than with conventional transplants because of slower neutrophil and immune reconstitution, and higher risk of graft failure. The high TRM and the prolonged hospitalizations are the main reasons for the declining interest in CB and the rise of use of haploidentical transplants. The HCT-CI is the best validated index to predict TRM in transplantation. TRM with CB is reported at 26% and 50% if HCTCI is < 3 and ≥ 3, respectively. Median HCT-CI in the trial was 2 with 8 (38%) patients having a score ≥3. The TRM thus appears to be very low at 5%. There are multiple reasons to explain and corroborate a low TRM: i) rapid neutrophil recovery at D+18 with very rapid attainment of 100 neutrophils, ii) better HLA match with >75% of patients receiving a ≥6/8 allele matched CB, iii) very low risk of graft failure, iv) absence of ATG use, v) prompt immune reconstitution leading to low risk of severe viral infections, and vi) absence of steroid refractory GVHD. It is unusual to have TRM with CB transplantation beyond 12 months because of the low incidence of chronic GVHD and therefore it is unlikely that the TRM described herein will increase dramatically with longer follow up.

In summary, excellent results were obtained with single UM171 expanded cord blood transplants in patients with high-risk hematologic malignancies and multiple comorbidities. Findings provided herein confirm UM171 expanded single CB as an acceptable graft when no HLA identical donor is available.

## Claims

1. A graft of expanded stem and/or progenitor cells cultured with at least one compound, for use in a method of reducing severe graft-versus-host disease (GVHD) and/or severe viral infections, wherein said graft leads to an expansion of dendritic (DC) cells, natural killer (NK) cells or a combination thereof *in vivo* in a patient, wherein said expansion of DC cells, NK cells or combination thereof stimulates an immune response in said patient and wherein said at least one compound has formula I: 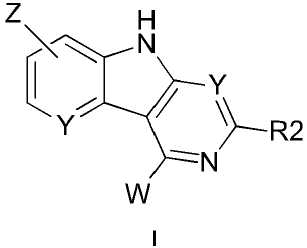 or a salt thereof,
wherein:
each Y is independently selected from N and CH;
Z is
- CN
- C(O)OR1,
- C(O)N(R1)R3,
- C(O)R1, or
- heteroaryl optionally substituted with one or more RA or R4 substituents,
wherein, when (R1) and R3 are attached to a nitrogen atom, optionally they join together with the nitrogen atom to form a 3 to 7-membered ring which optionally includes one or more other heteroatom selected from N, O and S, optionally the ring is substituted with one or more RA or R4;
W is
- CN,
- N(R1)R3,
- C(O)OR1,
- C(O)N(R1)R3,
- NR1C(O)R1,
- NR1C(O)OR1,
- OC(O)N(R1)R3,
- OC(O)R1,
- C(O)R1,
- NR1C(O)N(R1)R3,
- NR1S(O)₂R1,
- benzyl optionally substituted with 1, 2 or 3 RA or R1 substituents,
- X-L-(X-L)n - N(R1)R3,
- X-L-(X-L)n - heteroaryl optionally substituted with one or more RA or R4 substituents attached on either or both the L and heteroaryl groups,
- X-L-(X-L)n - heterocyclyl optionally substituted with one or more RA or R4 substituents attached on either or both the L and heterocyclyl groups,
- X-L-(X-L)n- aryl optionally substituted with one or more RA or R4 substituents,
- X-L-(X-L)ₙ-NR1RA or
- (N(R1)-L)ₙ - N⁺R1R3R5 R6⁻
wherein n is an integer equal to either 0, 1, 2, 3, 4, or 5,
and wherein, when R1 and R3 are attached to a nitrogen atom, optionally they join together with the nitrogen atom to form a 3 to 7-membered ring which optionally includes one or more other heteroatom selected from N, O and S, optionally the ring is substituted with one or more RA or R4;
each X is independently selected from C, O, S, and NR1;
each L is independently
- C₁₋₆ alkylene,
- C₂₋₆ alkenylene,
- C₂₋₆ alkynylene,
- C₃₋₇cycloalkylene, which optionally includes one or more other heteroatom selected from N, O and S or
- C₃₋₇ cycloalkenylene, which optionally includes one or more other heteroatom selected from N, O and S
wherein the alkylene, the alkenylene, the alkynylene the cycloalkylene and the cycloalkenylene groups are each independently optionally substituted with one or two R4 or RA substituent;
R1 is each independently
- H,
- C₁₋₆ alkyl,
- C₂₋₆ alkenyl,
- C₂₋₆ alkynyl,
- C₃₋₇ cycloalkyl,
- C₃₋₇ cycloalkenyl,
- C₁₋₅ perfluorinated,
- heterocyclyl,
- aryl,
- heteroaryl,or
- benzyl,
wherein the alkyl, the alkenyl, the alkynyl, the cycloalkenyl, the perfluorinated alkyl, the heterocyclyl, the aryl, the heteroaryl and the benzyl groups are each independently optionally substituted with 1, 2 or 3 RA or Rd substituents;
R2 is
- H,
- C₁₋₆ alkyl, optionally substituted with one more RA substituents
- C(O)R4,
- L-heteroaryl optionally substituted with one or more RA or R4 substituents
- L-heterocyclyl optionally substituted with one or more RA or R4, or
- L-aryl optionally substituted with one or more RA or R4 substituents;
R3 is each independently
- H,
- C₁₋₅ alkyl,
- C₂₋₆ alkenyl,
- C₂₋₆ alkynyl,
- C₃₋₇ cycloalkyl,
- C₃₋₇ cycloalkenyl,
- C₁₋₅ perfluorinated,
- heterocyclyl,
- aryl,
- heteroaryl,or
- benzyl,
wherein the alkyl, the alkenyl, the alkynyl, the cycloalkyl, the cycloalkenyl, the perfluorinated alkyl, the heterocyclyl, the aryl, the heteroaryl and the benzyl groups are each independently optionally substituted with 1, 2 or 3 RA or Rd substituents;
R4 is each independently
- H,
- C₁₋₅ alkyl,
- C₂₋₆ alkenyl,
- C₂₋₆ alkynyl,
- C₃₋₇ cycloalkyl,
- C₃₋₇ cycloalkenyl,
- C₁₋₅ perfluorinated,
- heterocyclyl,
- aryl,
- heteroaryl, or
- benzyl,
wherein the alkyl, the alkenyl, the alkynyl, the cycloalkyl, the cycloalkenyl, the perfluorinated alkyl, the heterocyclyl, the aryl, the heteroaryl and the benzyl groups are each independently optionally substituted with 1, 2 or 3 RA or Rd substituents;
R5 is each independently
- C₁₋₆ alkyl,
- C₁₋₆ alkylene-C₂₋₆ alkenyl which optionally includes one or more other heteroatom selected from N, O and S
- C₁₋₆ alkylene-C₂₋₆ alkynyl which optionally includes one or more other heteroatom selected from N, O and S
- L-aryl which optionally includes one or more RA or R4 substituents
- L-heteroaryl which optionally includes one or more RA or R4 substituents
- C₁₋₆alkylene-C(O)O-
- C₁₋₆ alkylene-C(O)OR1
- C₁₋₆ alkylene-CN
- C₁₋₆ alkylene-C(O)NR1R3, wherein R1 and R3 optionally they join together with the nitrogen atom to form a 3 to 7-membered ring which optionally includes one or more other heteroatom selected from N, O and S; or
- C₁₋₆ alkylene-OH;
R6 is
- Halogen
- OC(O)CF₃ or
- OC(O)R1;
RA is each independently
- halogen,
- CF₃,
- OR1,
- L-OR1,
- OCF₃,
- SR1,
- CN,
- NO₂,
- NR1R3,
- L-NR1R1,
- C(O)OR1,
- S(O)₂R4
- C(O)N(R1)R3,
- NR1C(O)R1,
- NR1C(O)OR1,
- OC(O)N(R1)R3,
- OC(O)R1,
- C(O)R4,
- NHC(O)N(R1)R3,
- NR1C(O)N(R1)R3, or
- N₃; and
Rd is each independently
- H,
- C₁₋₆ alkyl,
- C₂₋₆ alkenyl,
- C₂₋₆ alkynyl,
- C₃₋₇ cycloalkyl,
- C₃₋₇ cycloalkenyl,
- C₁₋₅ perfluorinated
- benzyl or
- heterocyclyl.

2. The graft of expanded stem and/or progenitor cells cultured with at least one compound, said at least one compound having formula I, for use of claim 1, wherein the stem and/or progenitor cells are human hematopoietic stem cells (HSC).

3. The graft of expanded stem and/or progenitor cells cultured with at least one compound, said at least one compound having formula I, for use of claim 2, wherein the hematopoietic stem cells are from umbilical cord blood cells, mobilized peripheral blood cells, or bone marrow cells.

4. The graft of expanded stem and/or progenitor cells cultured with at least one compound, said at least one compound having formula I, for use of any one of claims 1-3, wherein said NK cells are CD56⁺ or NKG2A⁺ cells and said DC cells are CD11c+ cells.

5. The graft of expanded stem and/or progenitor cells cultured with at least one compound, said at least one compound having formula I, for use of any one of claims 1-4, wherein said stem and progenitor cells are further cultured with an aryl hydrocarbon receptor (AHR) antagonist; preferably the AHR antagonist is Stem Regenin 1 (SR1) or CH223191.

6. The graft of expanded stem and/or progenitor cells cultured with at least one compound, said at least one compound having formula I, for use of any one of claims 1-5, wherein the compound of formula I is UM171 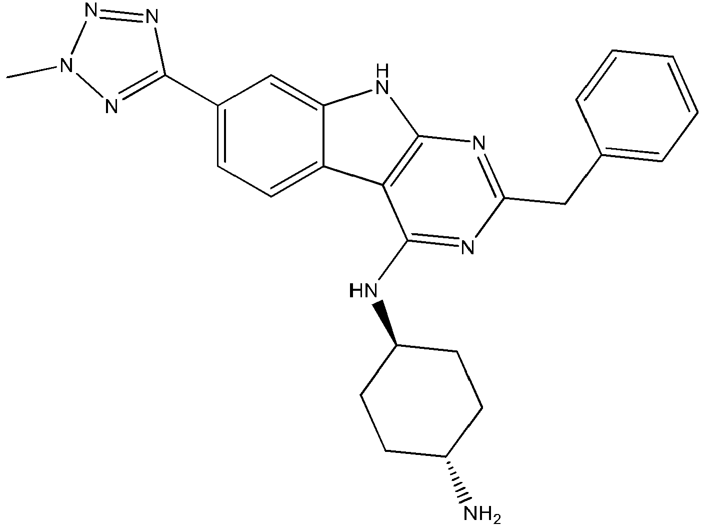 or a pharmaceutically acceptable salt thereof.

7. The graft of expanded stem and/or progenitor cells cultured with at least one compound, said at least one compound having formula I, for use of any one of claims 1-5, wherein the compound of formula I is a hydrobromide salt of

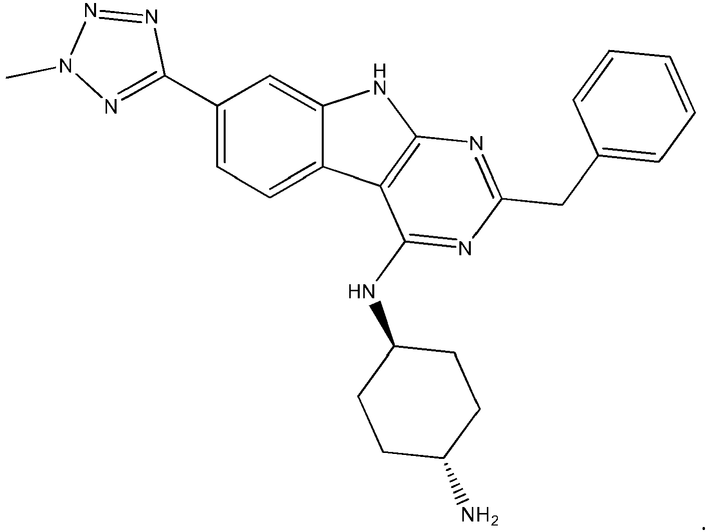

8. The graft of expanded stem and/or progenitor cells cultured with at least one compound, said at least one compound having formula I, for use of any one of claims 1-5, wherein the compound of formula I is 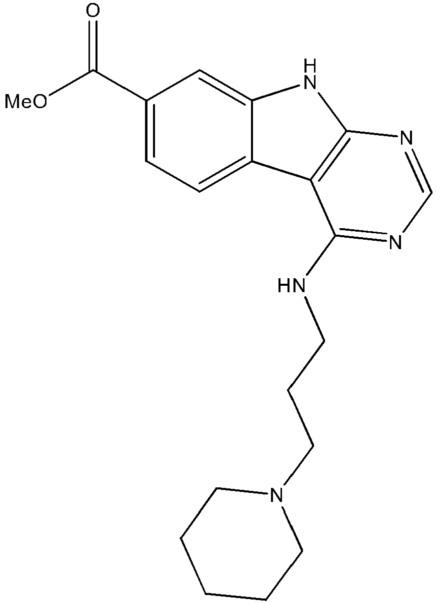 or a pharmaceutically acceptable salt thereof.

9. The graft of expanded stem and/or progenitor cells cultured with at least one compound, said at least one compound having formula I, for use of any one of claims 1-5, wherein the compound of formula I is:
| | |
|---|---|
| 1 | 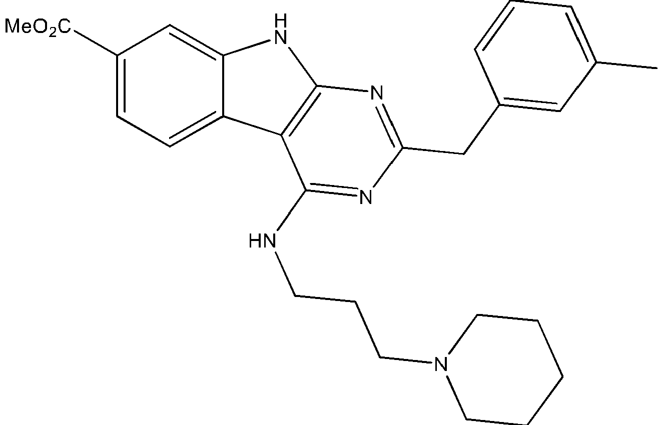 |
| 2 | 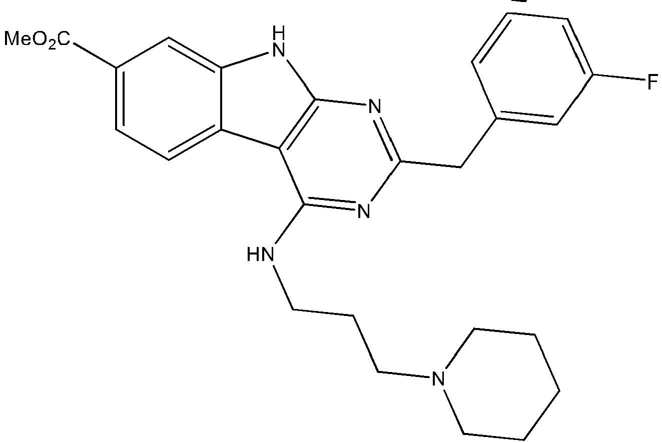 |
| 3 | 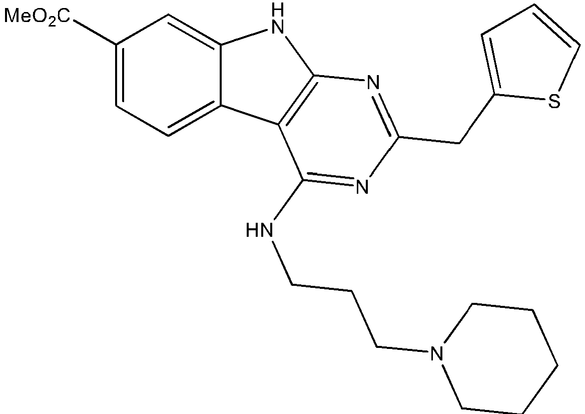 |
| 4 | 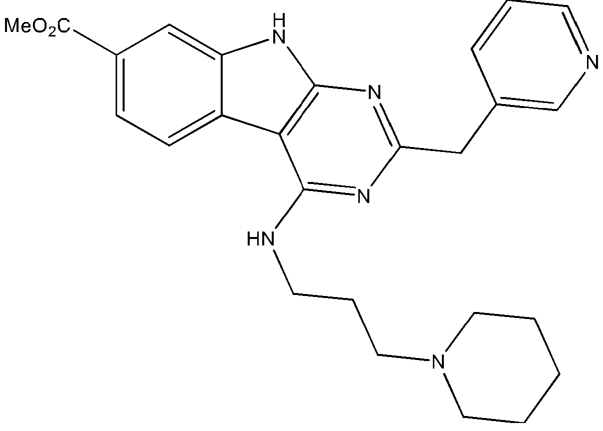 |
| 5 | 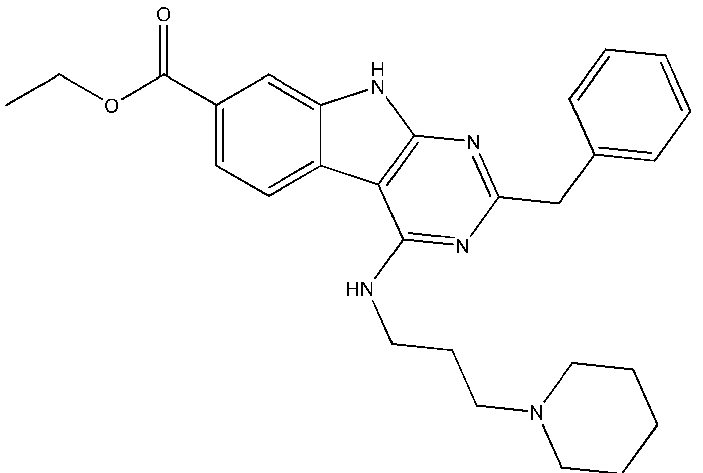 |
| 6 | 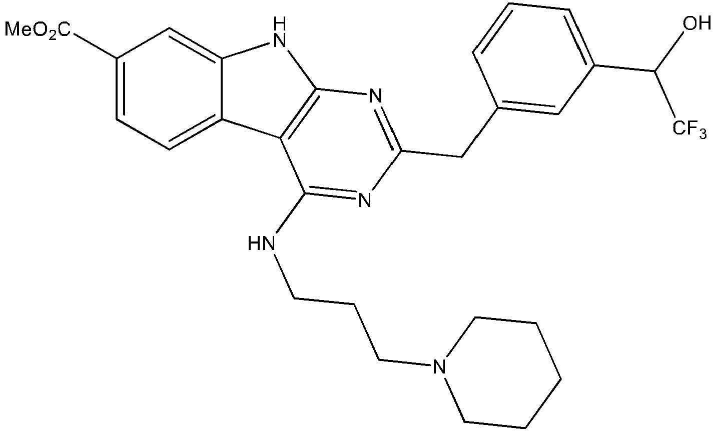 |
| 7 | 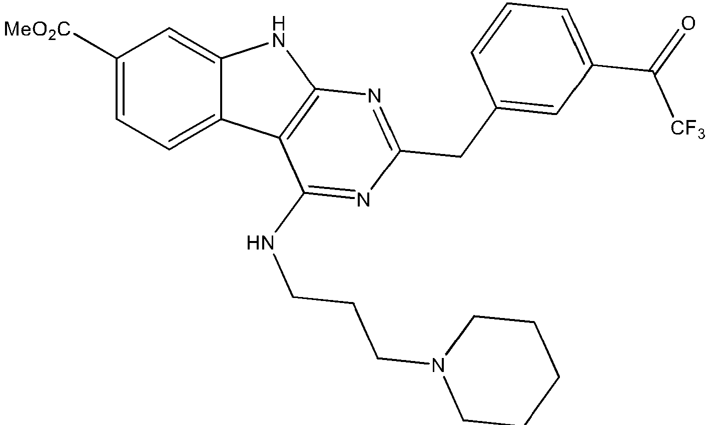 |
| 8 | 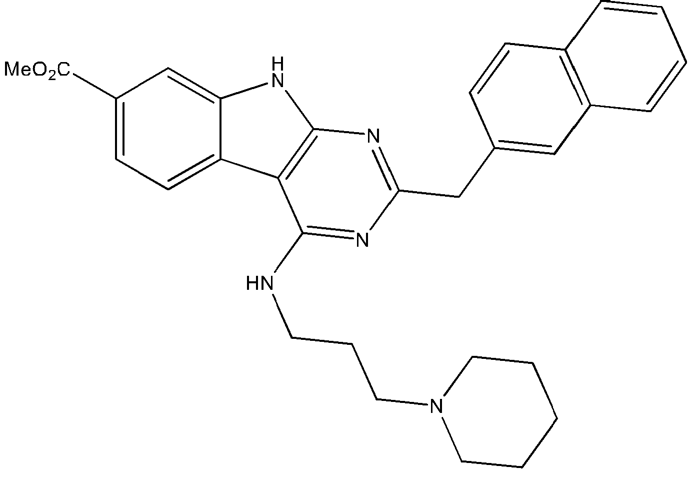 |
| 9 | 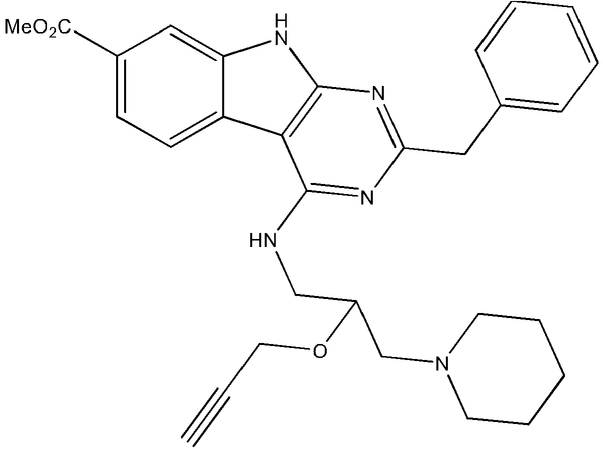 |
| 10 | 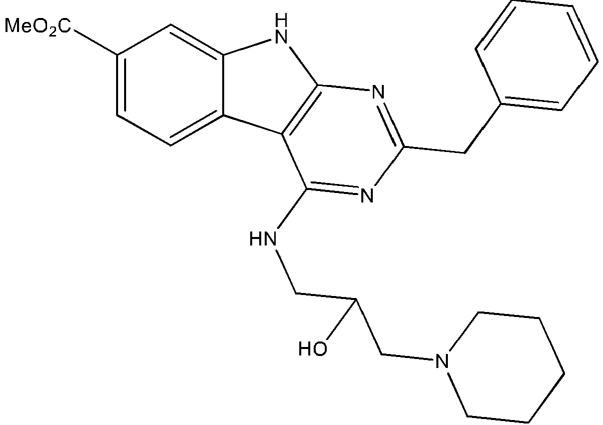 |
| 11 | 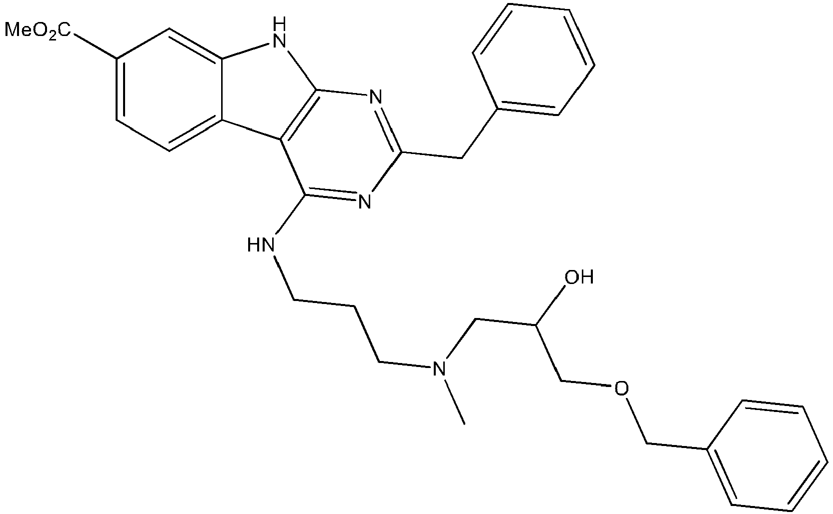 |
| 12 | 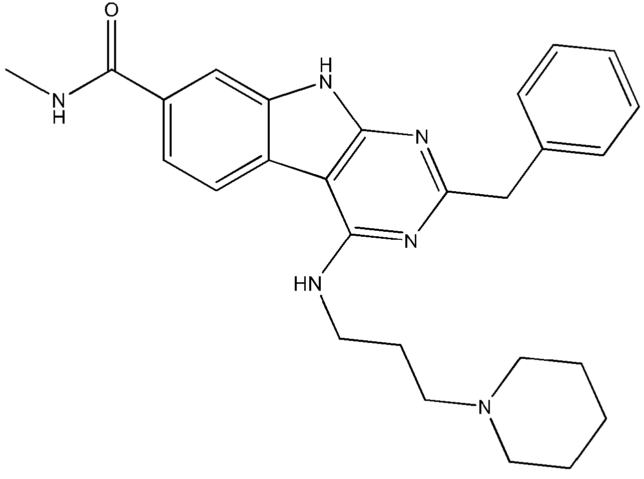 |
| 13 | 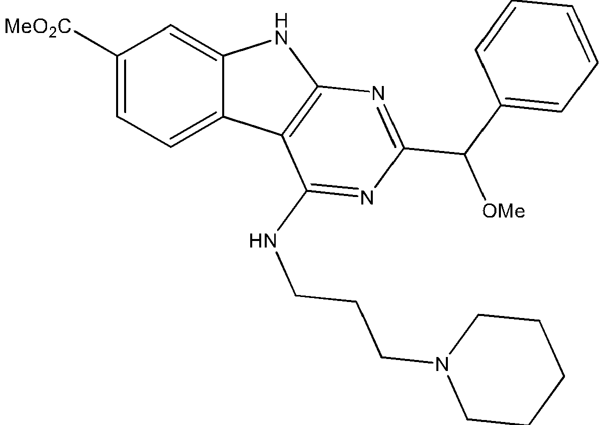 |
| 14 | 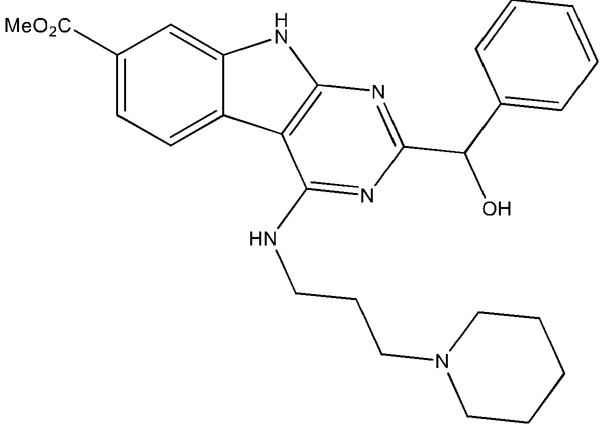 |
| 15 | 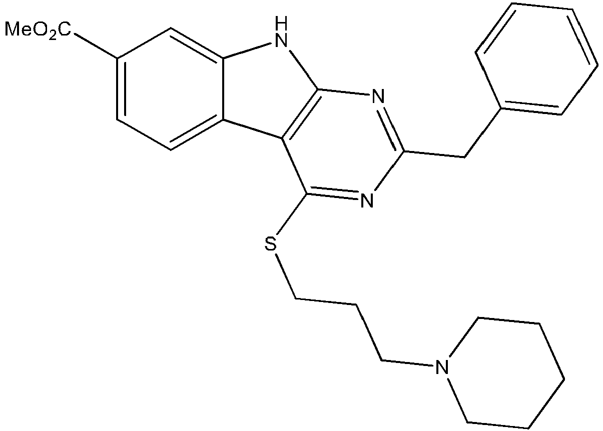 |
| 16 | 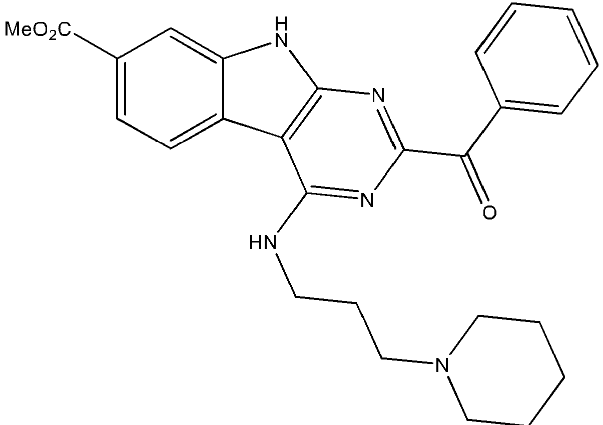 |
| 17 | 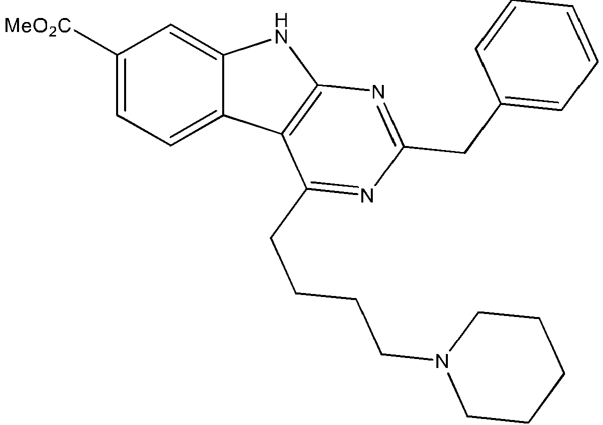 |
| 18 | 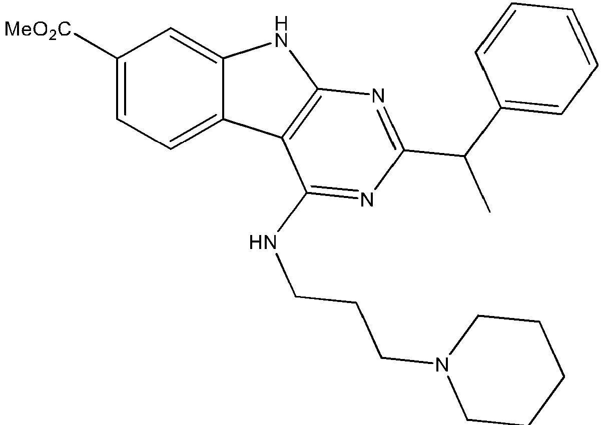 |
| 19 | 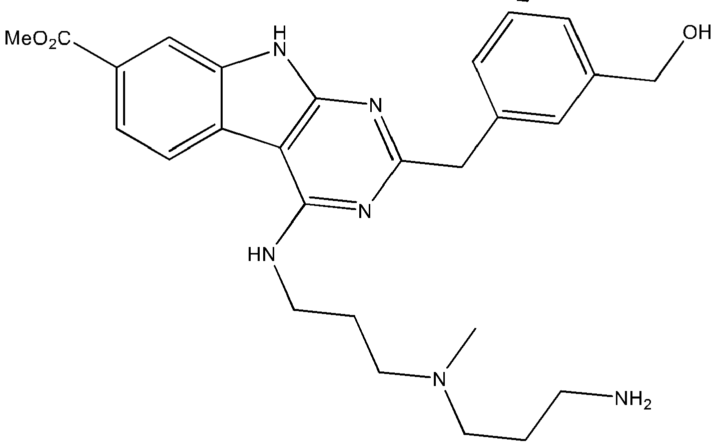 |
| 20 | 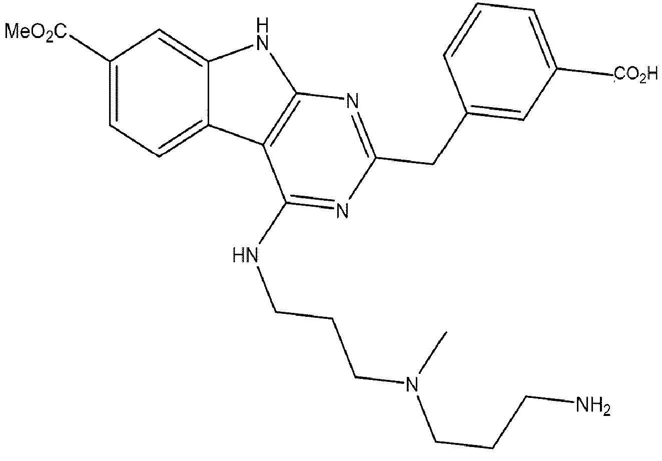 |
| 21 | 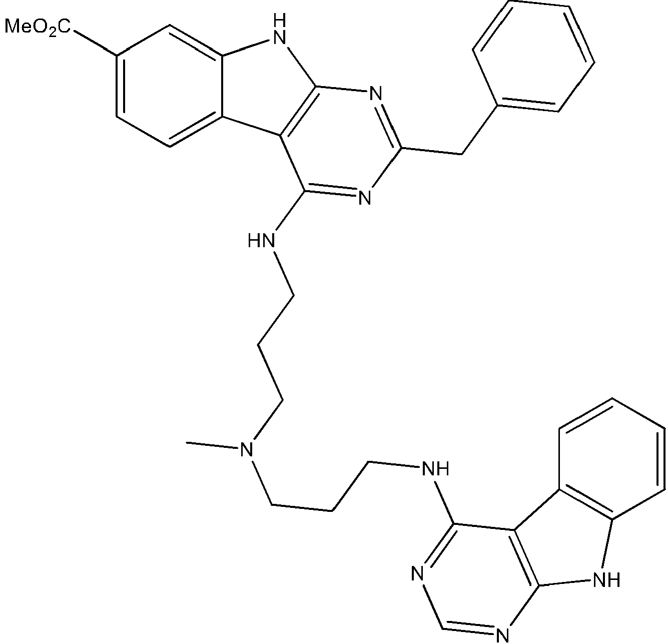 |
| 22 | 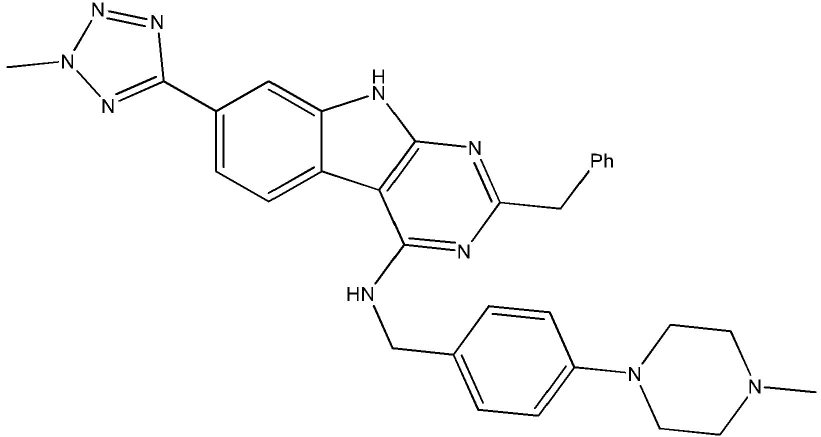 |
| 23 | 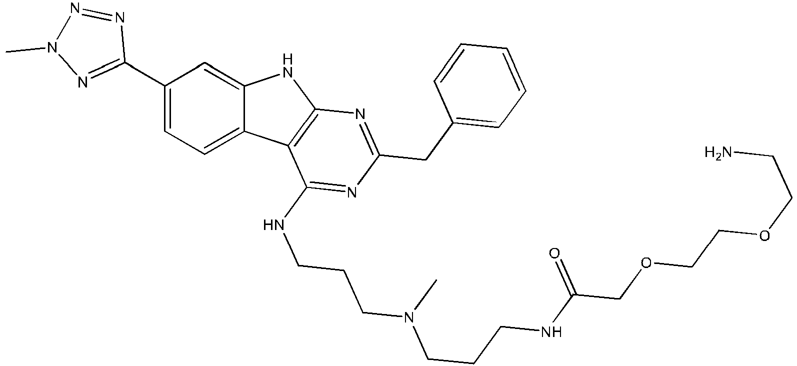 |
| 24 | 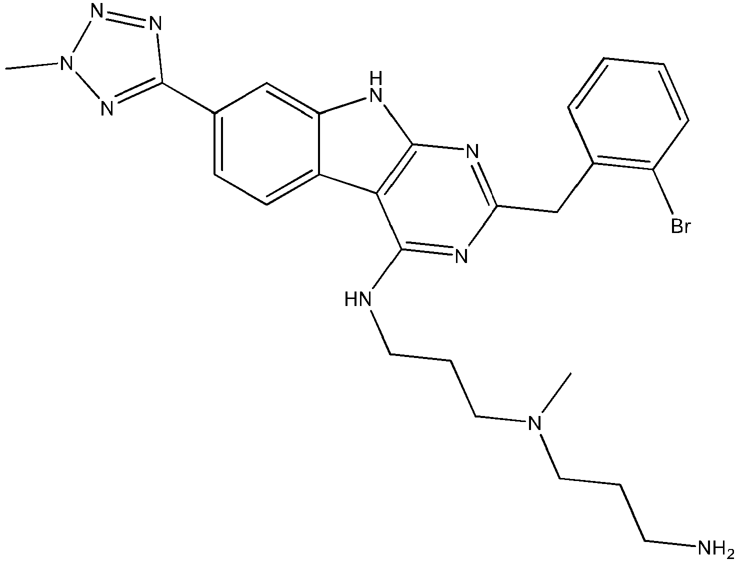 |
| 25 | 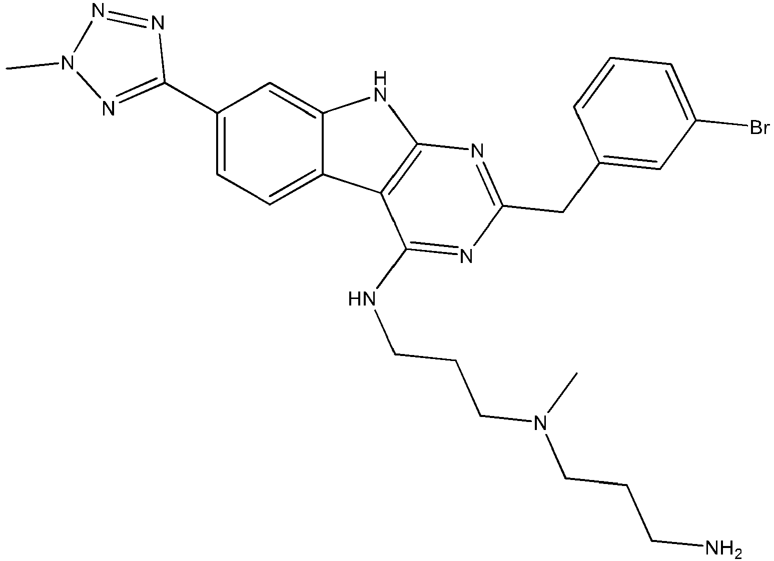 |
| 26 | 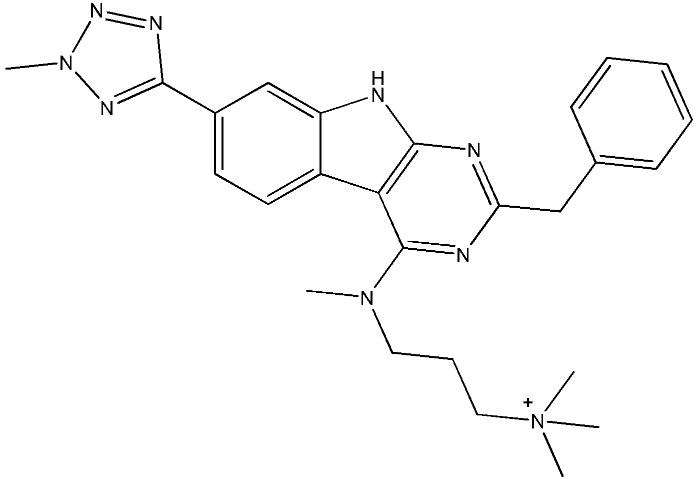 |
| 27 | 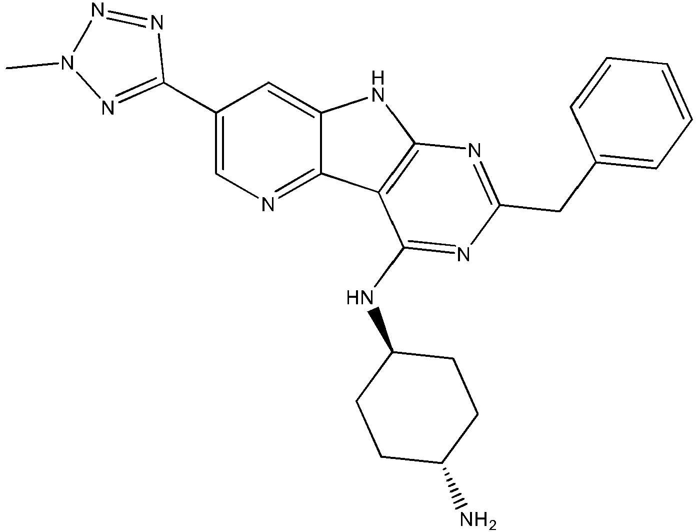 |
| 28 | 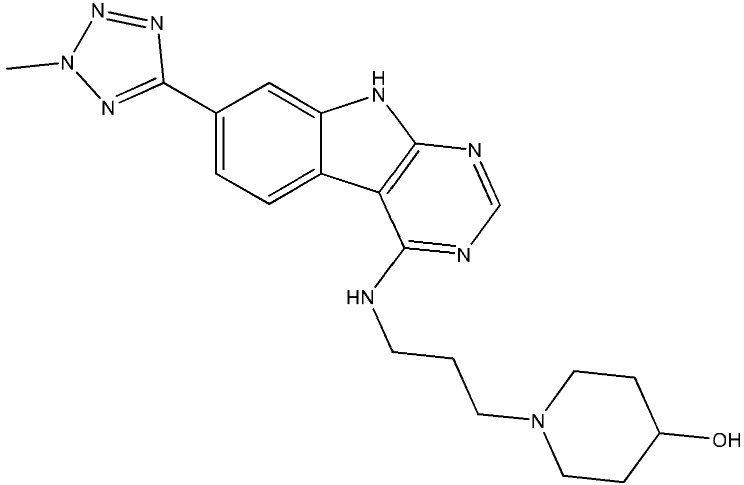 |
| 29 | 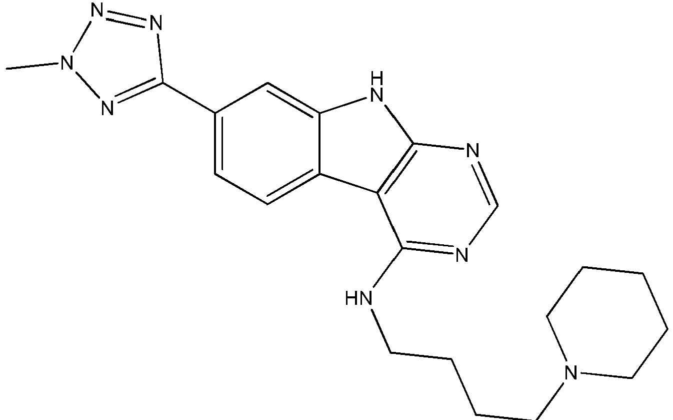 |
| 30 | 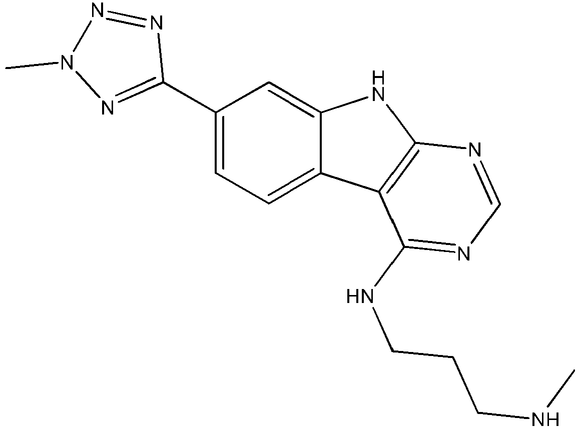 |
| 31 | 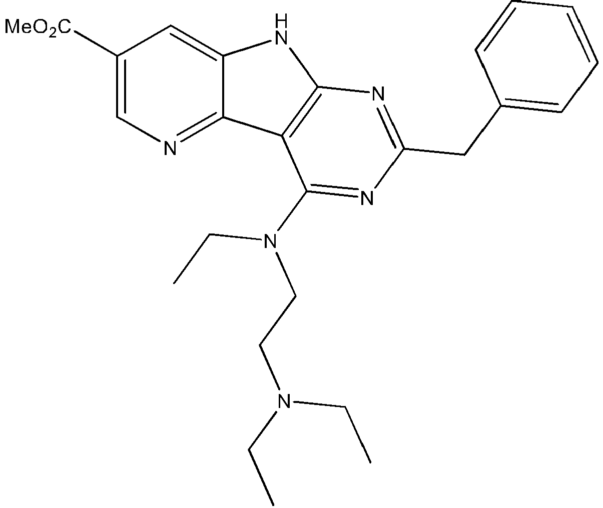 |
| 32 | 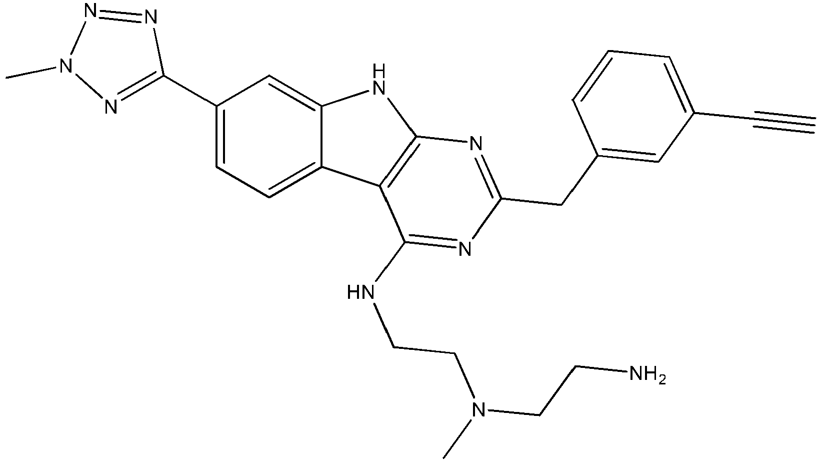 |
| 33 | 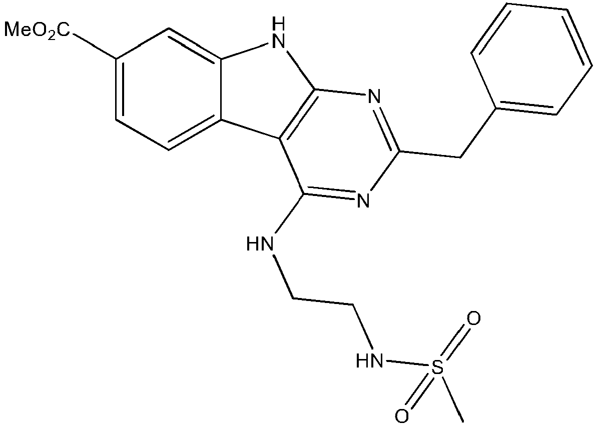 |
| 34 | 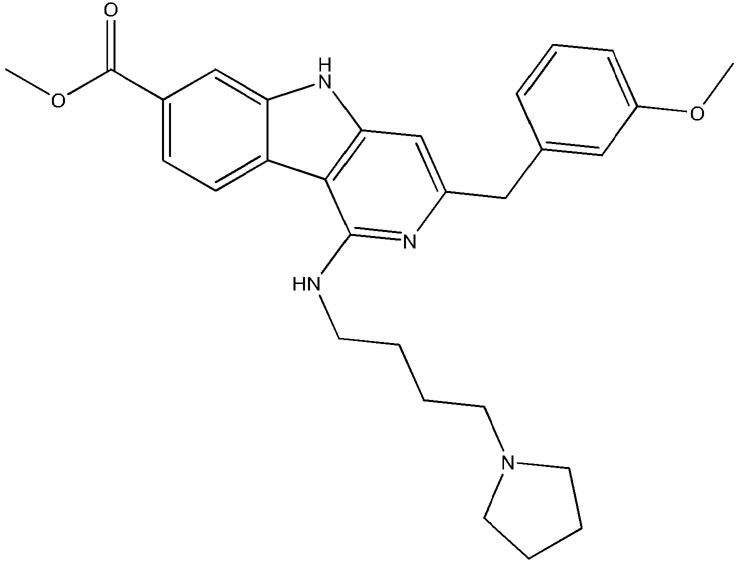 |
| 35 | 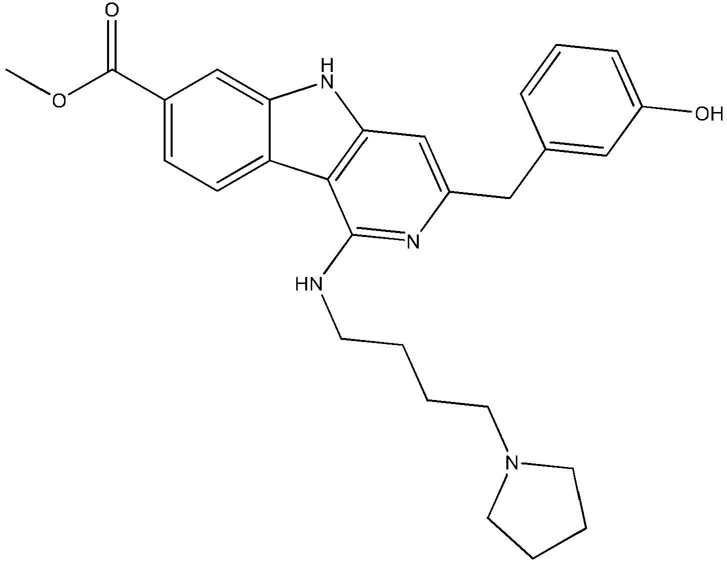 |
| 36 | 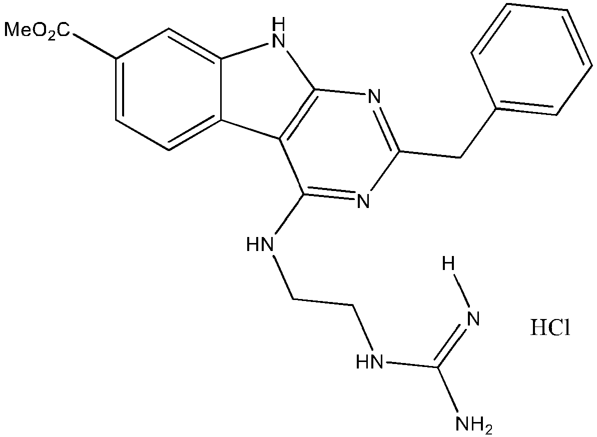 |
| 37 | 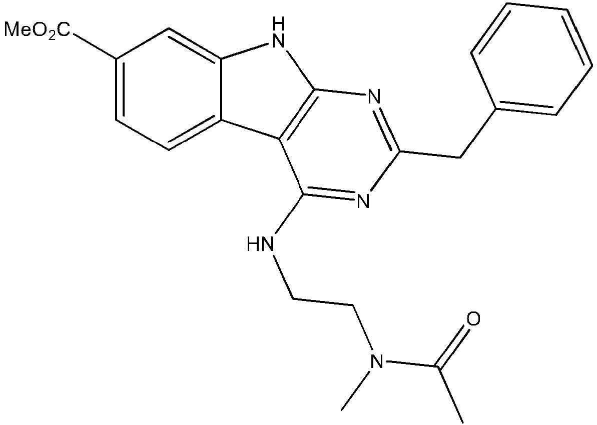 |
| 38 | 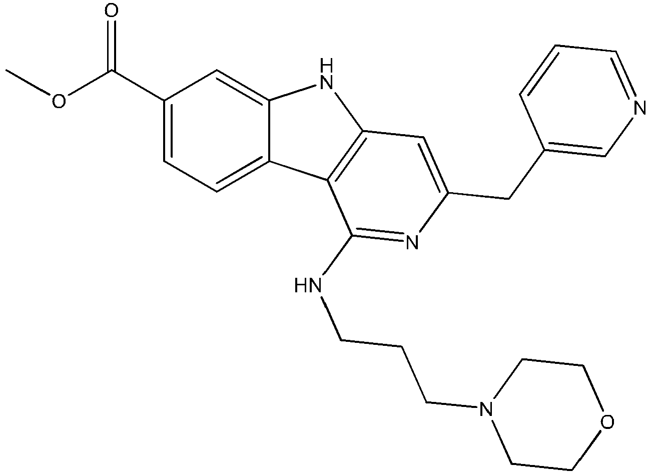 |
| 39 | 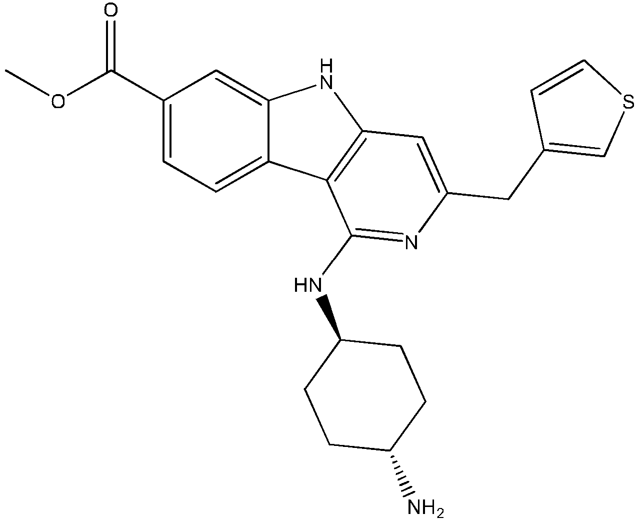 |
| 40 | 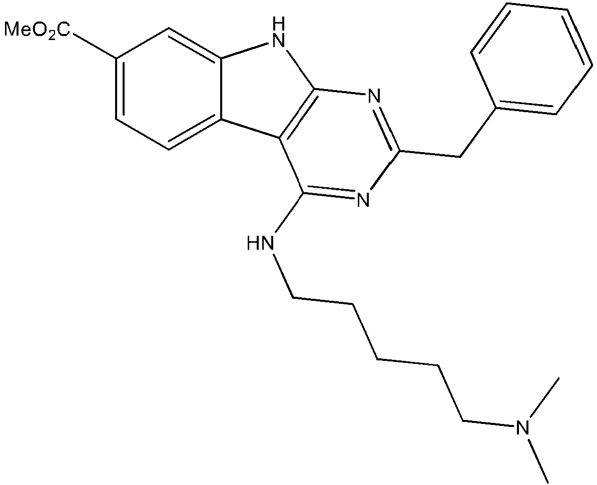 |
| 41 | 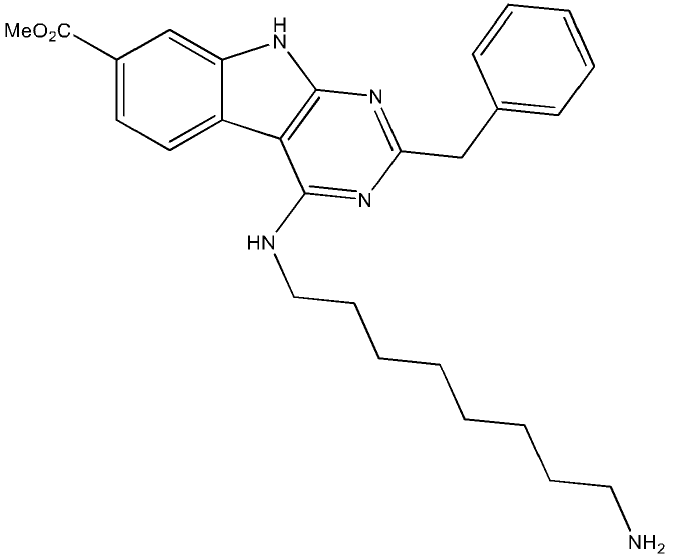 |
| 42 | 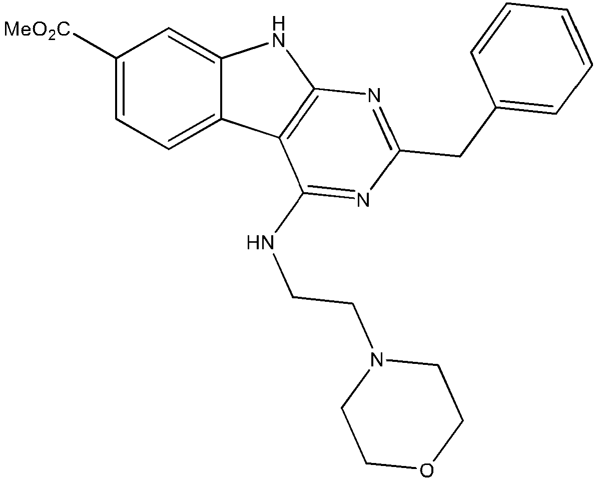 |
| 43 | 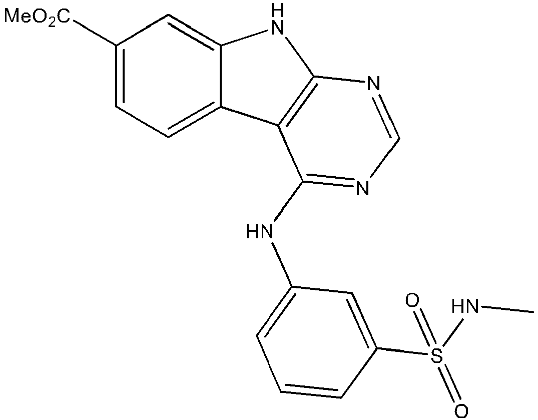 |
| 44 | 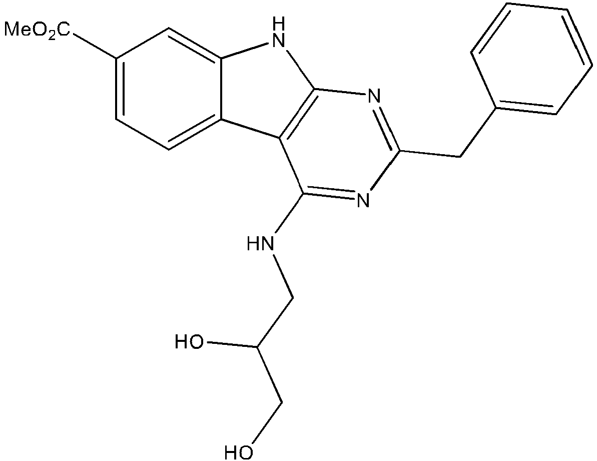 |
| 45 | 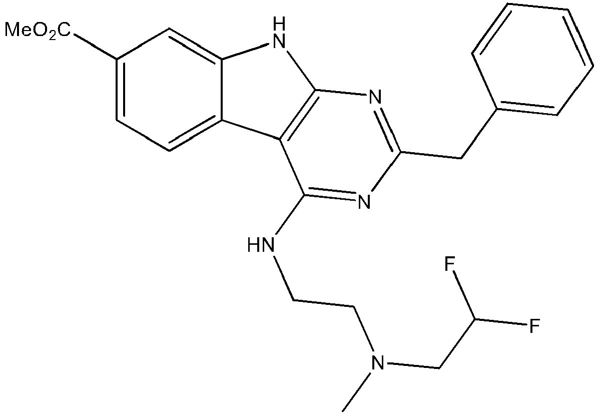 |
| 46 | 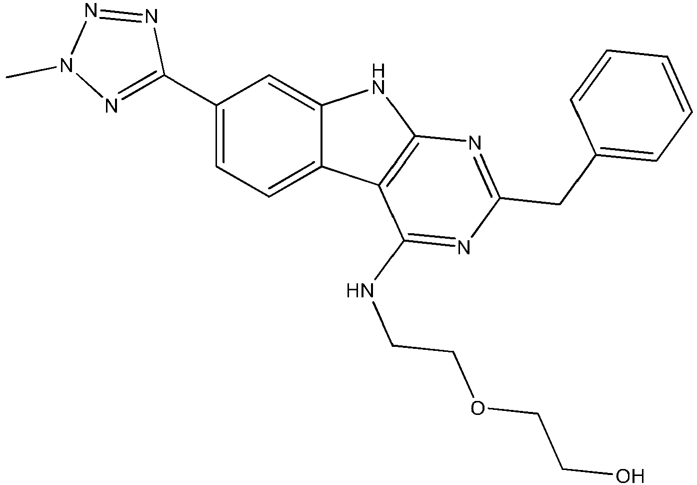 |
| 47 | 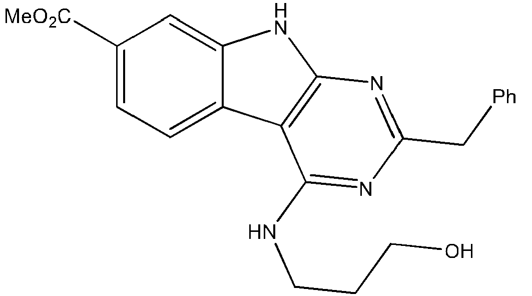 |
| 48 | 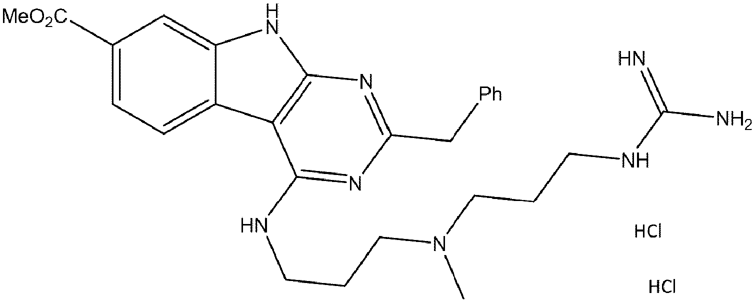 |
| 49 | 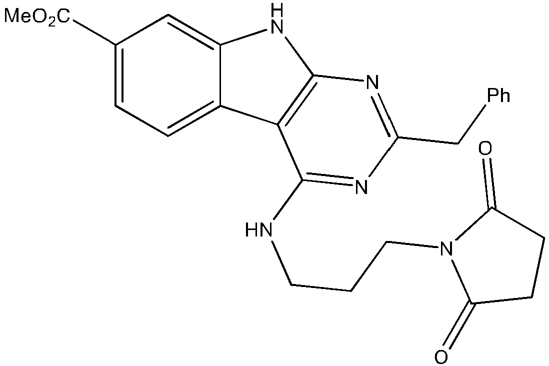 |
| 50 | 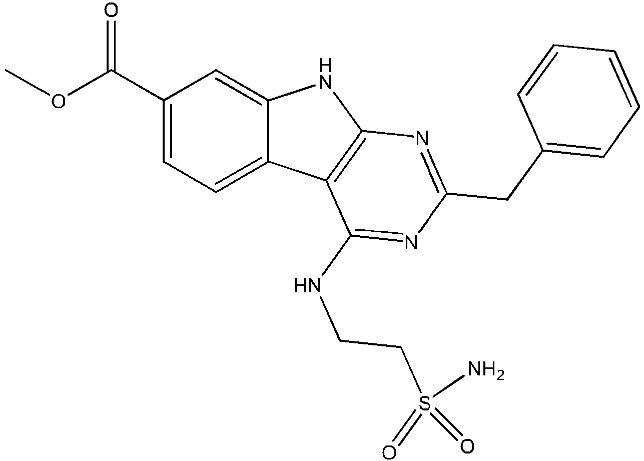 |
| 51 | 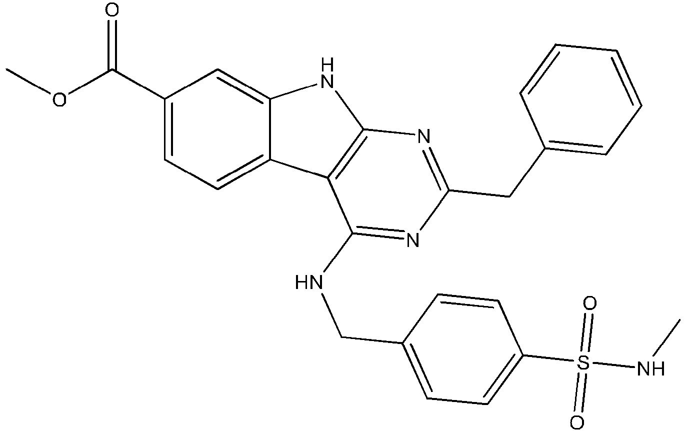 |
| 52 | 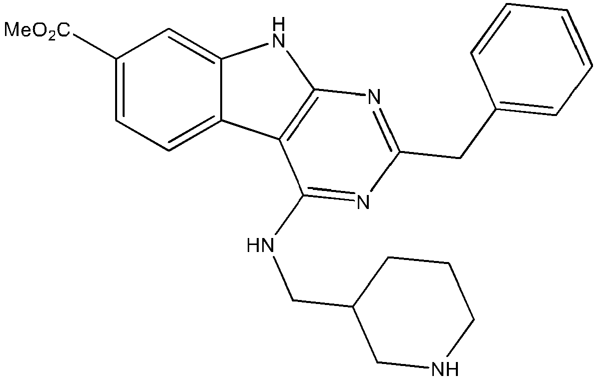 |
| 53 | 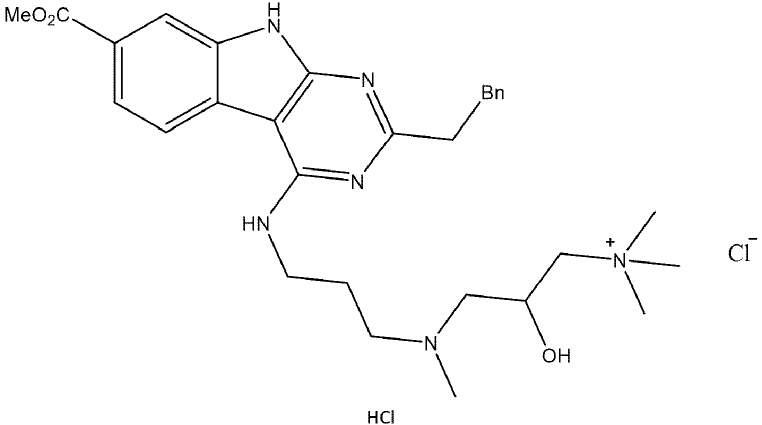 |
| 54 | 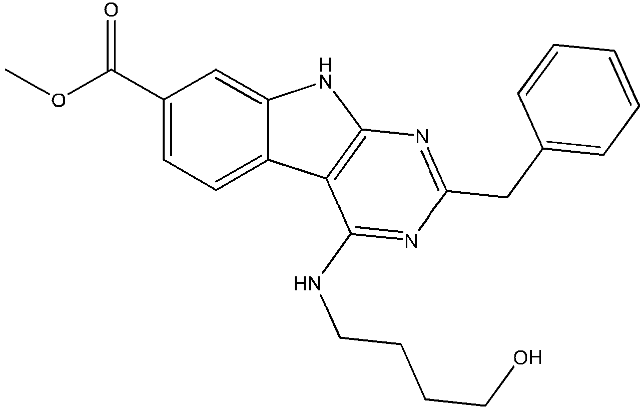 |
| 55 | 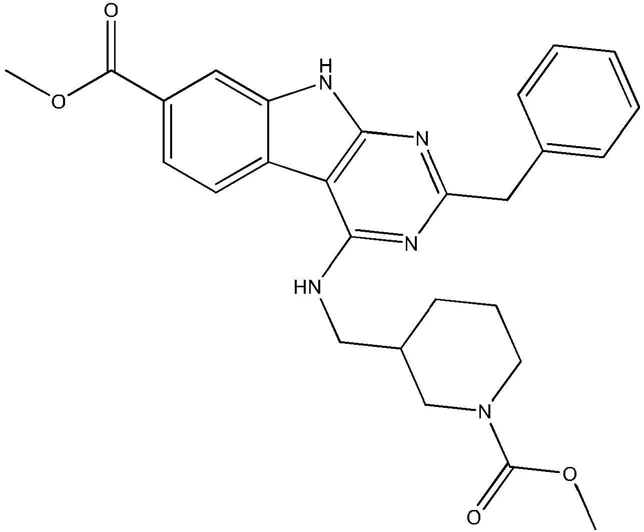 |
| 56 | 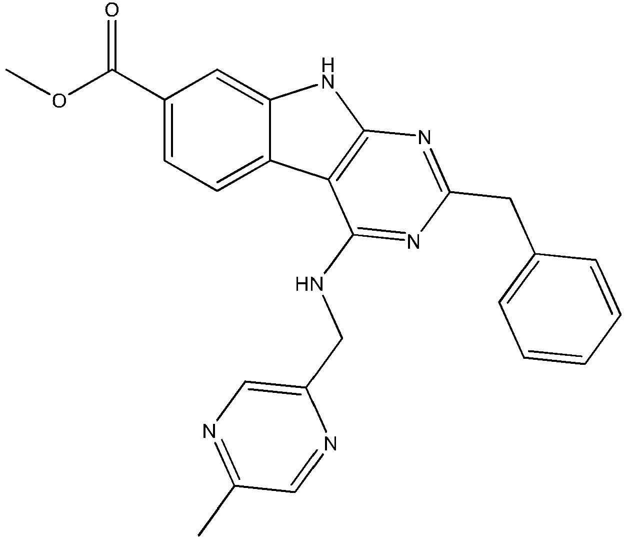 |
| 57 | 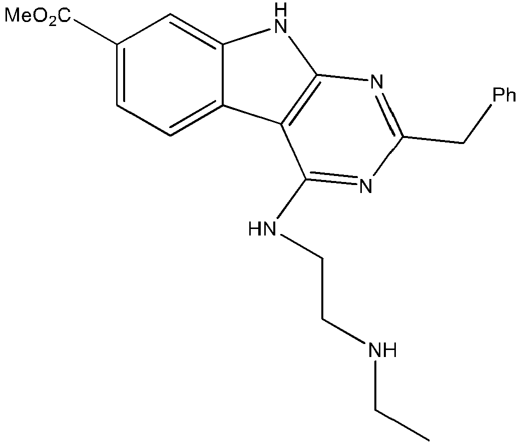 |
| 58 | 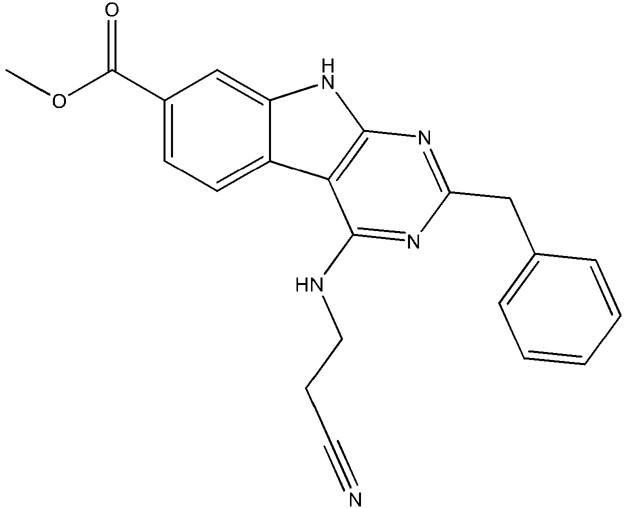 |
| 59 | 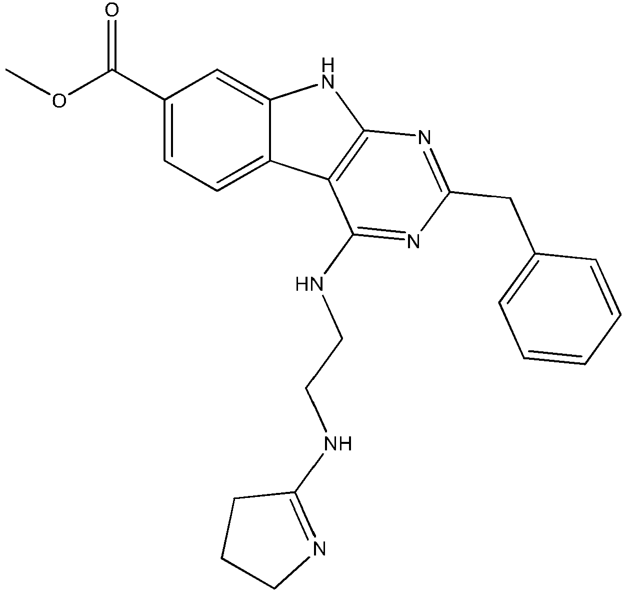 |
| 60 | 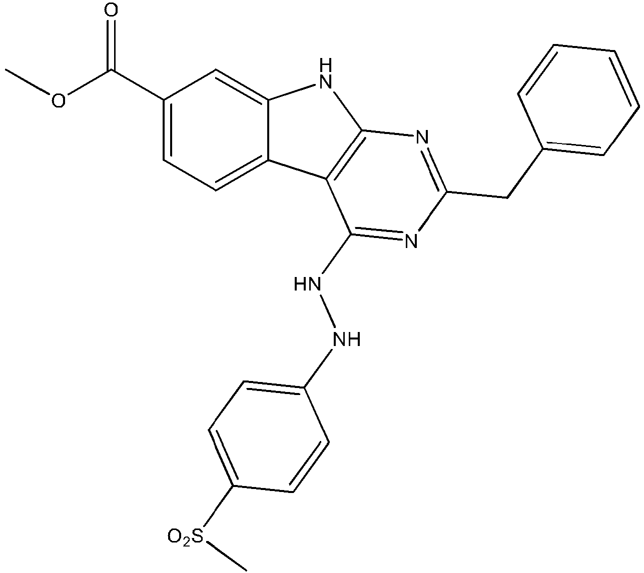 |
| 61 | 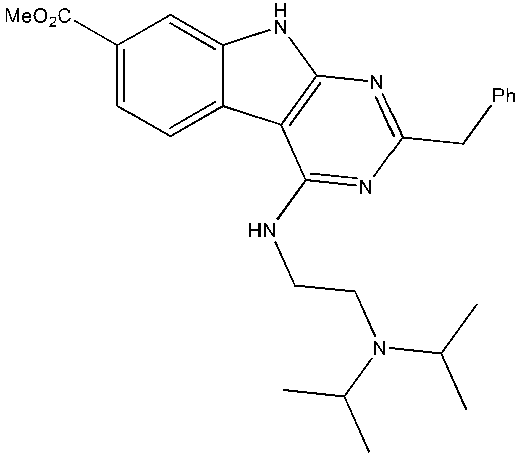 |
or a pharmaceutically acceptable salt thereof.

10. The graft of expanded stem and/or progenitor cells cultured with at least one compound, said at least one compound having formula I, for use of any one of claims 1-9, wherein the inflammatory state is controlled in said patient.

11. The graft of expanded stem and/or progenitor cells cultured with at least one compound, said at least one compound having formula I, for use of any one of claims 1-10, wherein the graft comprises a dendritic cell population and mast cells,

12. The graft of expanded stem and/or progenitor cells cultured with at least one compound, said at least one compound having formula I, for use of any one of claims 1-11, wherein the graft comprises FCER1⁺CD34⁺ cells, CD34⁺CD45RA⁺ cells, CD34⁺CD86⁺ cells, CD34⁺CD45RA⁻ cells and CD34⁻ cells.

## Patentansprüche

1. Transplantat expandierter Stamm- und/oder Progenitorzellen, kultiviert mit wenigstens einer Verbindung, zur Verwendung in einem Verfahren zum Reduzieren schwerer Graft-versus-Host-Krankheit (graft-versus-host disease; GVHD) und/oder schwerer Virusinfektionen, wobei das Transplantat zu einer Expansion dendritischer Zellen (dendritic cells; DC), natürlicher Killer (NK)-Zellen oder einer Kombination davon *in vivo* in einem Patienten führt, wobei die Expansion von DC-Zellen, NK-Zellen oder der Kombination davon eine Immunreaktion in dem Patienten stimuliert und wobei die wenigstens eine Verbindung Formel I hat: 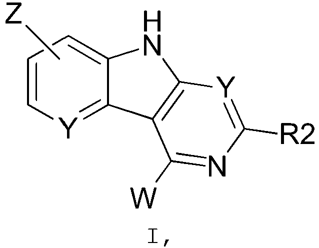 oder ein Salz davon,
wobei:
jedes Y unabhängig ausgewählt ist aus N und CH;
Z ist
- CN
- C(O)OR1,
- C(O)N(R1)R3,
- C(O)R1 oder
- Heteroaryl, optional substituiert mit einem oder mehreren RA- oder R4-Substituenten,
wobei, wenn (R1) und R3 an ein Stickstoffatom angeheftet sind, sie sich optional mit dem Stickstoffatom verbinden unter Bildung eines 3- bis 7-gliedrigen Rings, welcher optional ein oder mehrere Heteroatome einschließt, ausgewählt aus N, O und S, optional der Ring mit einem oder mehreren RA oder R4 substituiert ist;
W ist
- CN,
- N(R1)R3,
- C(O)OR1,
- C(O)N(R1)R3,
- NR1C(0)R1,
- NR1C(O)OR1,
- OC(O)N(R1)R3,
- OC(O)R1,
- C(0)R1,
- NR1C(O)N(R1)R3,
- NR1S(O)₂R1,
- Benzyl, optional substituiert mit 1, 2 oder 3 RA- oder R1-Substituenten,
- X-L-(X-L)n - N(R1)R3,
- X-L-(X-L)n - Heteroaryl, optional substituiert mit einem oder mehreren RA- oder R4-Substituenten, angeheftet an eine oder beide der L- und Heteroarylgruppen,
- X-L-(X-L)n - Heterocyclyl, optional substituiert mit einem oder mehreren RA- oder R4-Substituenten, angeheftet an eine oder beide der L- und Heterocyclylgruppen,
- X-L-(X-L)n- Aryl, optional substituiert mit einem oder mehreren RA- oder R4-Substituenten,
- X-L-(X-L)ₙ-NR1RA oder
- (N(R1)-L)ₙ - N⁺R1R3R5 R6⁻
wobei n eine ganze Zahl gleich entweder 0, 1, 2, 3, 4, oder 5 ist,
und wobei, wenn R1 und R3 an ein Stickstoffatom angeheftet sind, sie sich optional mit dem Stickstoffatom verbinden unter Bildung eines 3- bis 7-gliedrigen Rings, welcher optional ein oder mehrere Heteroatome einschließt, ausgewählt aus N, O und S, optional der Ring mit einem oder mehreren RA oder R4 substituiert ist;
jedes X unabhängig ausgewählt ist aus C, O, S und NR1;
jedes L unabhängig ist
- C₁₋₆-Alkylen,
- C₂₋₆-Alkenylen,
- C₂₋₆-Alkynylen,
- C₃₋₇-Cycloalkylen, welches optional ein oder mehrere Heteroatome einschließt, ausgewählt aus N, O und S oder
- C₃₋₇-Cycloalkenylen, welches optional ein oder mehrere Heteroatome einschließt, ausgewählt aus N, O und S
wobei die Alkylen-, die Alkenylen-, die Alkynylen- die Cycloalkylen- und die Cycloalkenylengruppen jeweils unabhängig optional substituiert sind mit einem oder zwei R4- oder RA-Substituenten;
R1 jeweils unabhängig ist
- H,
- C₁₋₆-Alkyl,
- C₂₋₆-Alkenyl,
- C₂₋₆-Alkynyl,
- C₃₋₇-Cycloalkyl,
- C₃₋₇-Cycloalkenyl,
- C₁₋₅ perfluoriert,
- Heterocyclyl,
- Aryl,
- Heteroaryl oder
- Benzyl,
wobei die Alkyl-, die Alkenyl-, die Alkynyl-, die Cycloalkenyl-, die perfluorierten Alkyl-, die Heterocyclyl-, die Aryl-, die Heteroaryl- und die Benzylgruppen jeweils unabhängig optional substituiert sind mit 1, 2 oder 3 RA- oder Rd-Substituenten;
R2 ist
- H,
- C₁₋₆-Alkyl, optional substituiert mit einem oder mehreren RA-Substituenten
- C(O)R4,
- L-Heteroaryl, optional substituiert mit einem oder mehreren RA- oder R4-Substituenten
- L-Heterocyclyl, optional substituiert mit einem oder mehreren RA oder R4, oder
- L-Aryl, optional substituiert mit einem oder mehreren RA- oder R4-Substituenten;
R3 jeweils unabhängig ist
- H,
- C₁₋₆-Alkyl,
- C₂₋₆-Alkenyl,
- C₂₋₆-Alkynyl,
- C₃₋₇-Cycloalkyl,
- C₃₋₇-Cycloalkenyl,
- C₁₋₅ perfluoriert,
- Heterocyclyl,
- Aryl,
- Heteroaryl oder
- Benzyl,
wobei die Alkyl-, die Alkenyl-, die Alkynyl-, die Cycloalkyl-, die Cycloalkenyl-, die perfluorierten Alkyl-, die Heterocyclyl-, die Aryl-, die Heteroaryl- und die Benzylgruppen jeweils unabhängig optional substituiert sind mit 1, 2 oder 3 RA- oder Rd-Substituenten;
R4 jeweils unabhängig ist
- H,
- C₁₋₆-Alkyl,
- C₂₋₆-Alkenyl,
- C₂₋₆-Alkynyl,
- C₃₋₇-Cycloalkyl,
- C₃₋₇-Cycloalkenyl,
- C₁₋₅ perfluoriert,
- Heterocyclyl,
- Aryl,
- Heteroaryl oder
- Benzyl,
wobei die Alkyl-, die Alkenyl-, die Alkynyl-, die Cycloalkyl-, die Cycloalkenyl-, die perfluorierten Alkyl-, die Heterocyclyl-, die Aryl-, die Heteroaryl- und die Benzylgruppen jeweils unabhängig optional substituiert sind mit 1, 2 oder 3 RA- oder Rd-Substituenten;
R5 jeweils unabhängig ist
- C₁₋₆-Alkyl,
- C₁₋₆-Alkylen-C₂₋₆-alkenyl, welches optional ein oder mehrere Heteroatome einschließt, ausgewählt aus N, O und S
- C₁₋₆-Alkylen-C₂₋₆-alkynyl, welches optional ein oder mehrere Heteroatome einschließt, ausgewählt aus N, O und S
- L-Aryl, welches optional einen oder mehrere RA- oder R4-Substituenten einschließt
- L-Heteroaryl, welches optional einen oder mehrere RA- oder R4-Substituenten einschließt
- C₁₋₆-Alkylen-C(O)O-
- C₁₋₆-Alkylen-C(O)OR1
- C₁₋₆-Alkylen-CN
- C₁₋₆-Alkylen-C(O)NR1R3, wobei R1 und R3 sich optional mit dem Stickstoffatom verbinden unter Bildung eines 3-bis 7-gliedrigen Rings, welcher optional ein oder mehrere Heteroatome einschließt, ausgewählt aus N, O und S; oder
- C₁₋₆-Alkylen-OH;
R6 ist
- Halogen
- OC(O)CF₃ oder
- OC(O)R1;
RA jeweils unabhängig ist
- Halogen,
- CF₃,
- OR1,
- L-OR1,
- OCF₃,
- SR1,
- CN,
- NO₂,
- NR1R3,
- L-NR1R1,
- C(O)OR1,
- S(O)₂R4
- C(O)N(R1)R3,
- NR1C(0)R1,
- NR1C(O)OR1,
- OC(O)N(R1)R3,
- OC(O)R1,
- C(O)R4,
- NHC(O)N(R1)R3,
- NR1C(O)N(R1)R3 oder
- N₃; und
Rd jeweils unabhängig ist
- H,
- C₁₋₆-Alkyl,
- C₂₋₆-Alkenyl,
- C₂₋₆-Alkynyl,
- C₃₋₇-Cycloalkyl,
- C₃₋₇-Cycloalkenyl,
- C₁₋₅ perfluoriert
- Benzyl oder
- Heterocyclyl.

2. Transplantat expandierter Stamm- und/oder Progenitorzellen, kultiviert mit wenigstens einer Verbindung, wobei die wenigstens eine Verbindung Formel I hat, zur Verwendung nach Anspruch 1, wobei die Stamm- und/oder Progenitorzellen menschliche hämatopoetische Stammzellen (hematopoietic stem cells; HSC) sind.

3. Transplantat expandierter Stamm- und/oder Progenitorzellen, kultiviert mit wenigstens einer Verbindung, wobei die wenigstens eine Verbindung Formel I hat, zur Verwendung nach Anspruch 2, wobei die hämatopoetischen Stammzellen von Nabelschnurblutzellen, mobilisierten Zellen des peripheren Blutes oder Knochenmarkzellen sind.

4. Transplantat expandierter Stamm- und/oder Progenitorzellen, kultiviert mit wenigstens einer Verbindung, wobei die wenigstens eine Verbindung Formel I hat, zur Verwendung nach irgendeinem der Ansprüche 1-3, wobei die NK-Zellen CD56⁺- oder NKG2A⁺-Zellen sind und die DC-Zellen CD11c⁺-Zellen sind.

5. Transplantat expandierter Stamm- und/oder Progenitorzellen, kultiviert mit wenigstens einer Verbindung, wobei die wenigstens eine Verbindung Formel I hat, zur Verwendung nach irgendeinem der Ansprüche 1-4, wobei die Stamm- und Progenitorzellen ferner mit einem Arylkohlenwasserstoffrezeptor (aryl hydrocarbon receptor; AHR)-Antagonisten kultiviert werden, wobei der AHR-Antagonist bevorzugt Stem Regenin 1 (SR1) oder CH223191 ist.

6. Transplantat expandierter Stamm- und/oder Progenitorzellen, kultiviert mit wenigstens einer Verbindung, wobei die wenigstens eine Verbindung Formel I hat, zur Verwendung nach irgendeinem der Ansprüche 1-5, wobei die Verbindung der Formel I UM171 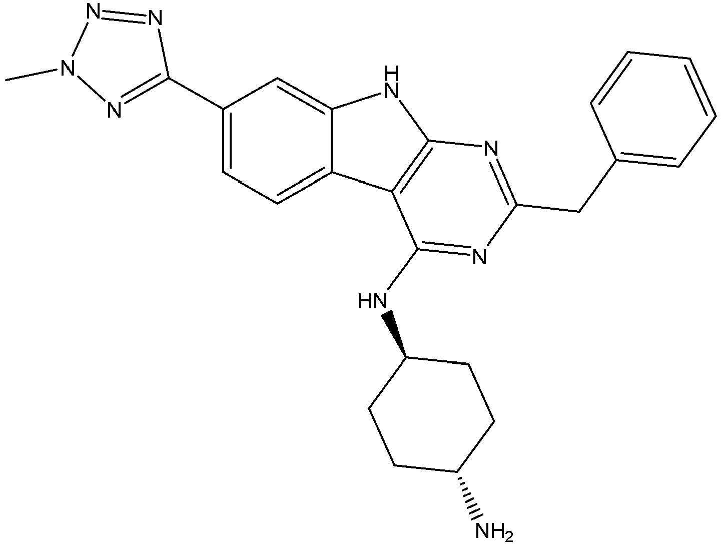 oder ein pharmazeutisch akzeptables Salz davon ist.

7. Transplantat expandierter Stamm- und/oder Progenitorzellen, kultiviert mit wenigstens einer Verbindung, wobei die wenigstens eine Verbindung Formel I hat, zur Verwendung nach irgendeinem der Ansprüche 1-5, wobei die Verbindung der Formel I ein Bromwasserstoffsalz ist von

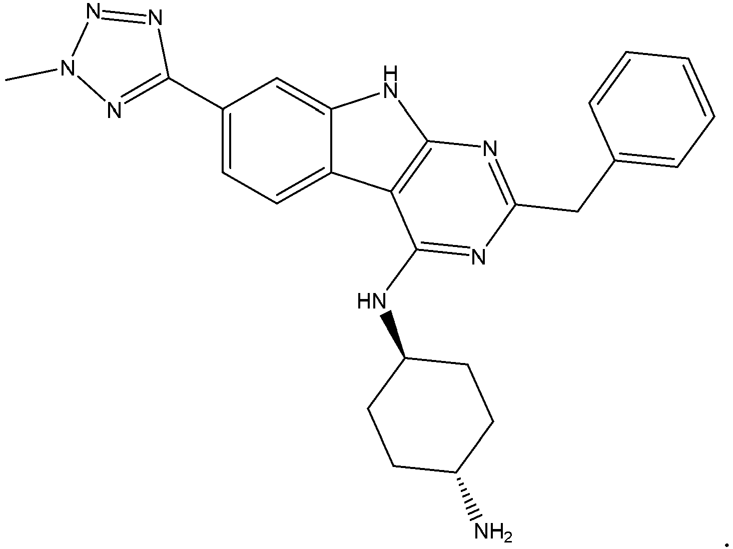

8. Transplantat expandierter Stamm- und/oder Progenitorzellen, kultiviert mit wenigstens einer Verbindung, wobei die wenigstens eine Verbindung Formel I hat, zur Verwendung nach irgendeinem der Ansprüche 1-5, wobei die Verbindung der Formel I 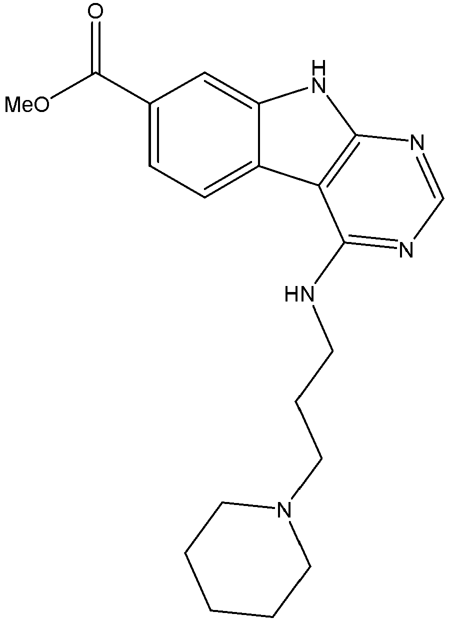 oder ein pharmazeutisch akzeptables Salz davon ist.

9. Transplantat expandierter Stamm- und/oder Progenitorzellen, kultiviert mit wenigstens einer Verbindung, wobei die wenigstens eine Verbindung Formel I hat, zur Verwendung nach irgendeinem der Ansprüche 1-5, wobei die Verbindung der Formel I ist:
| | |
|---|---|
| 1 | 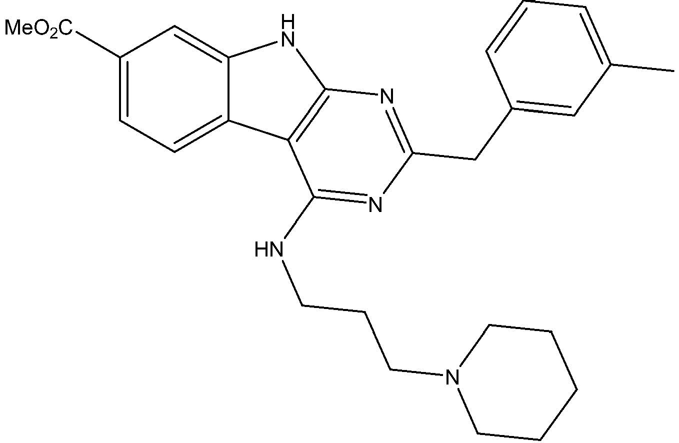 |
| 2 | 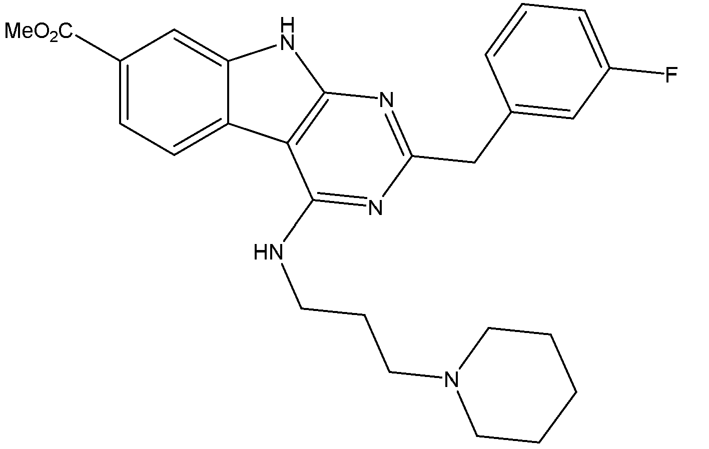 |
| 3 | 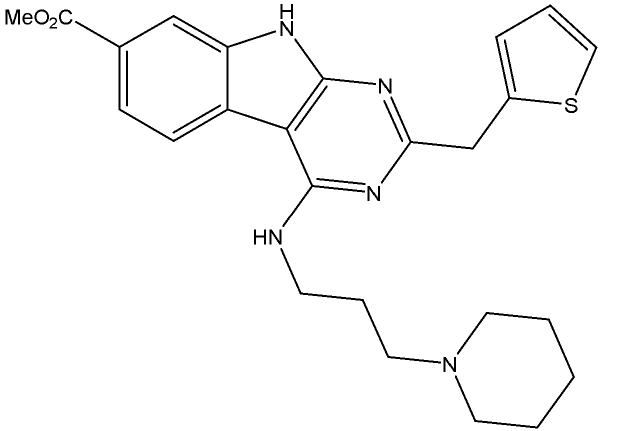 |
| 4 | 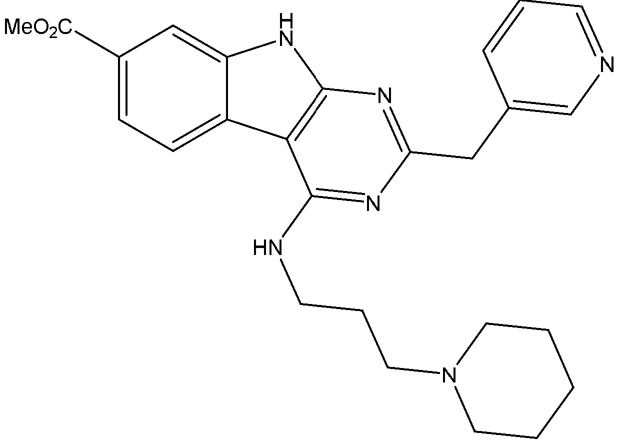 |
| 5 | 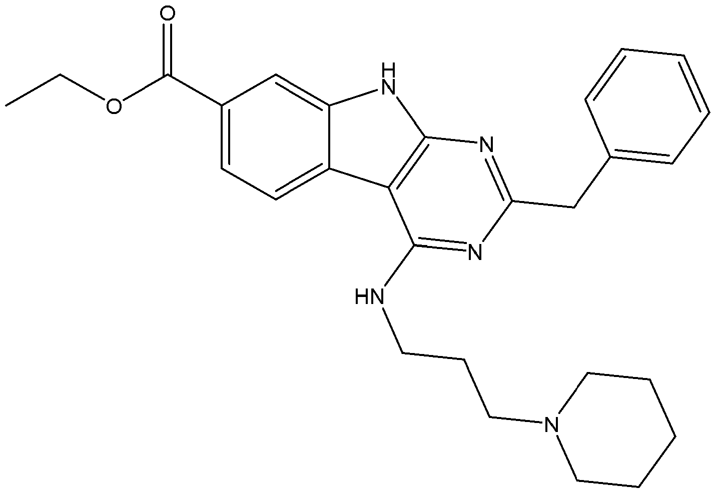 |
| 6 | 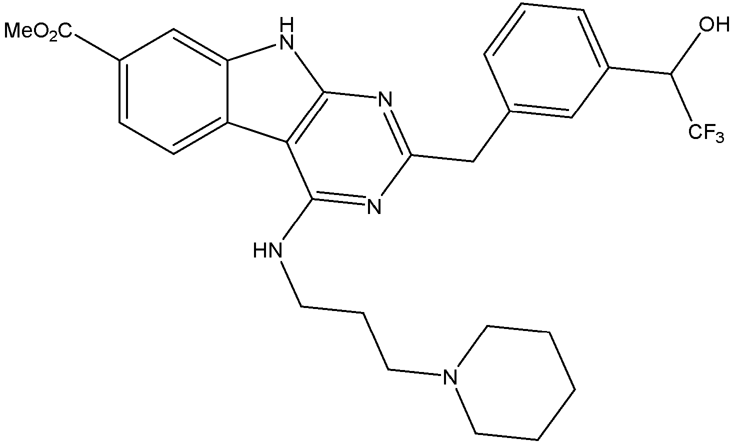 |
| 7 | 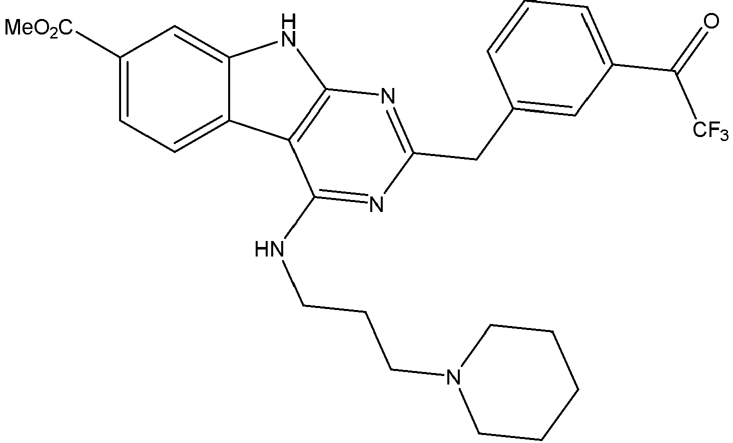 |
| 8 | 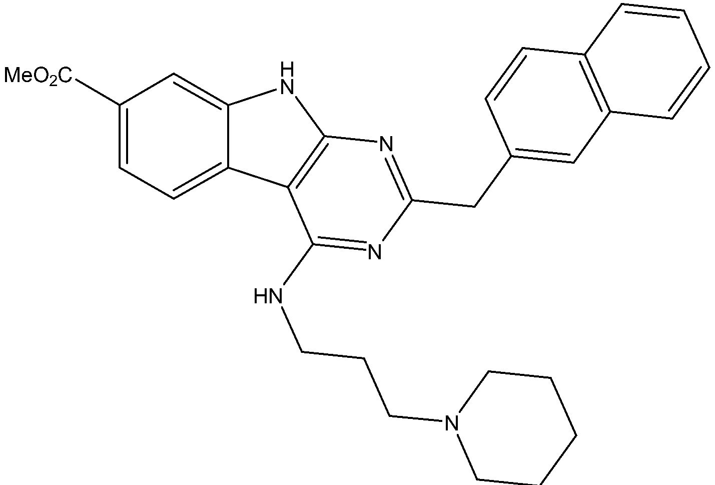 |
| 9 | 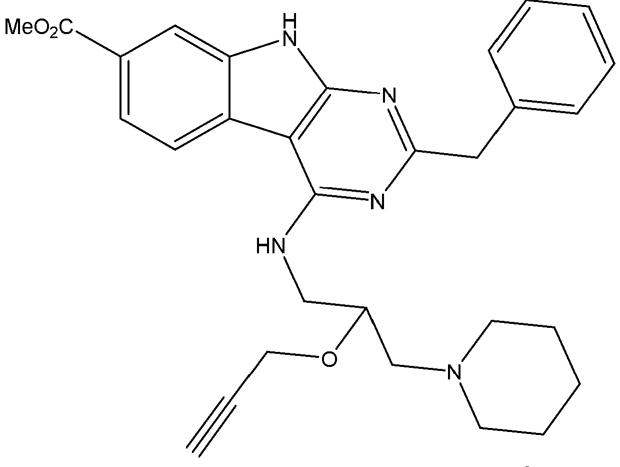 |
| 10 | 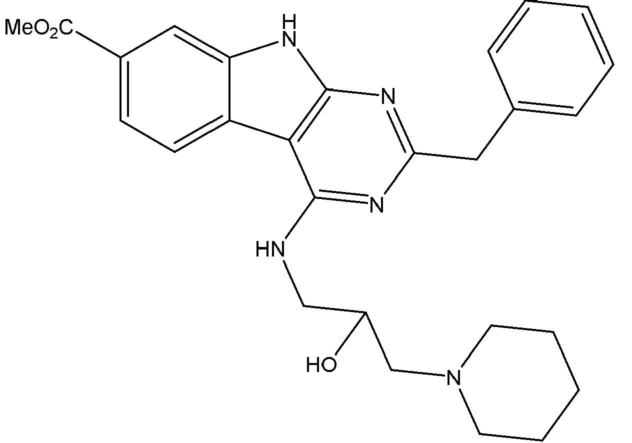 |
| 11 | 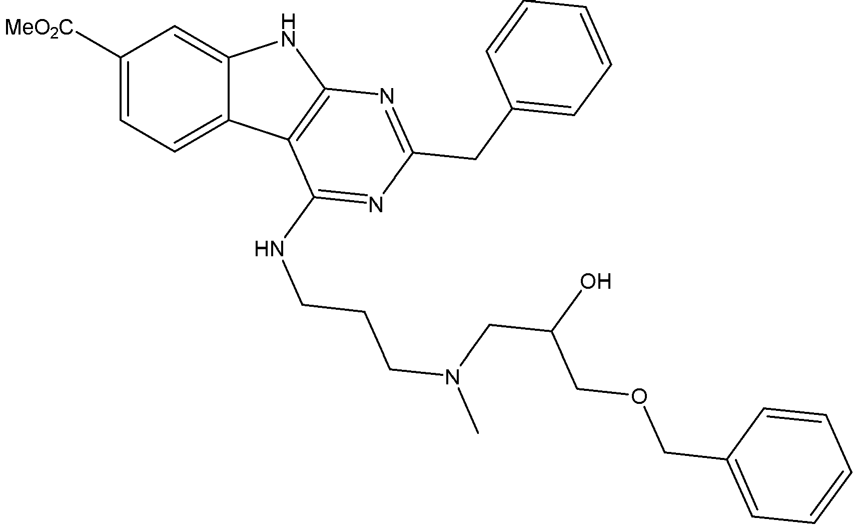 |
| 12 | 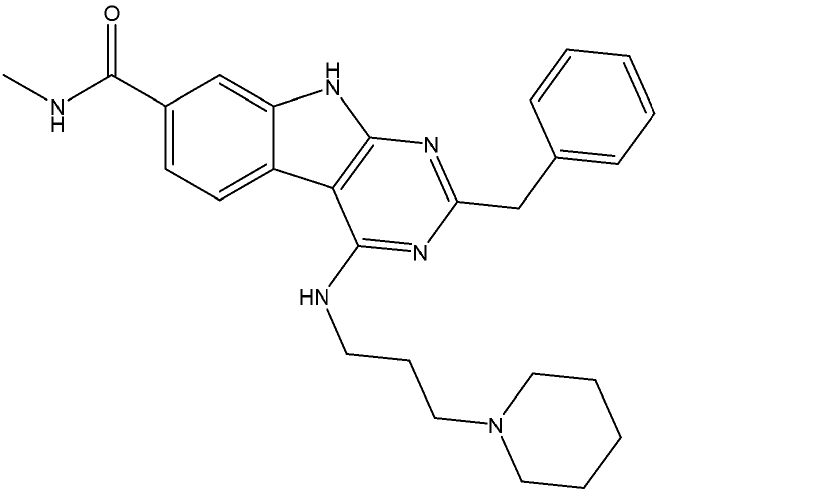 |
| 13 | 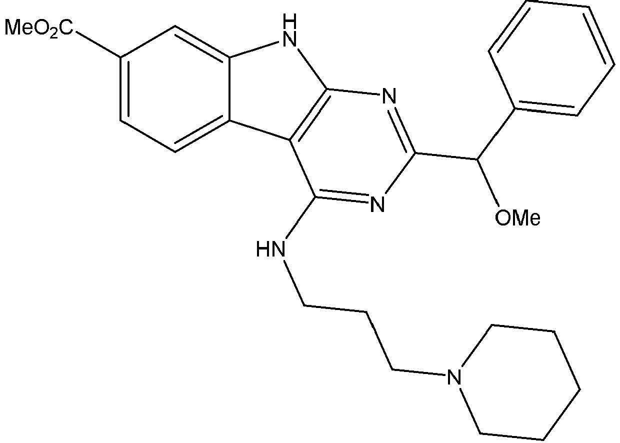 |
| 14 | 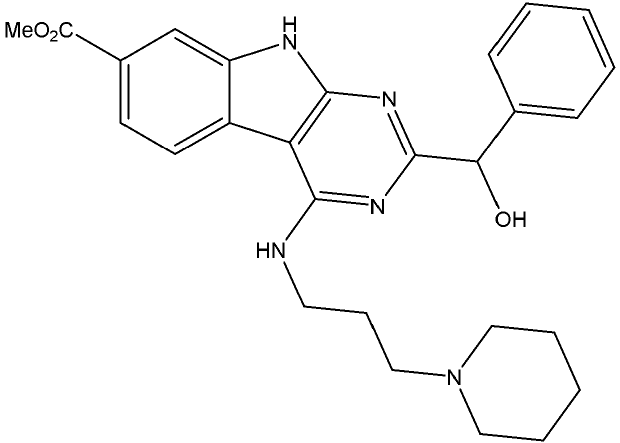 |
| 15 | 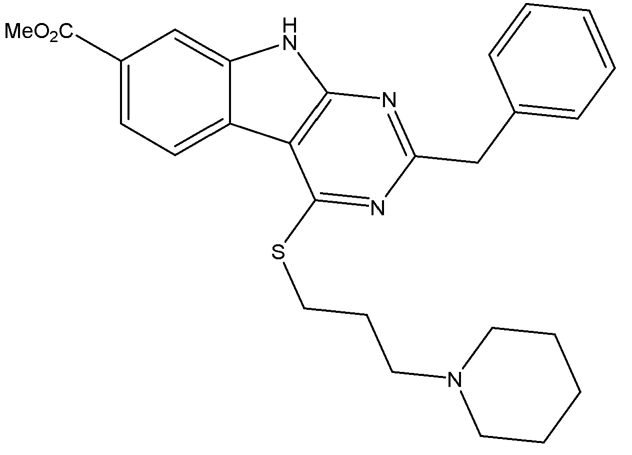 |
| 16 | 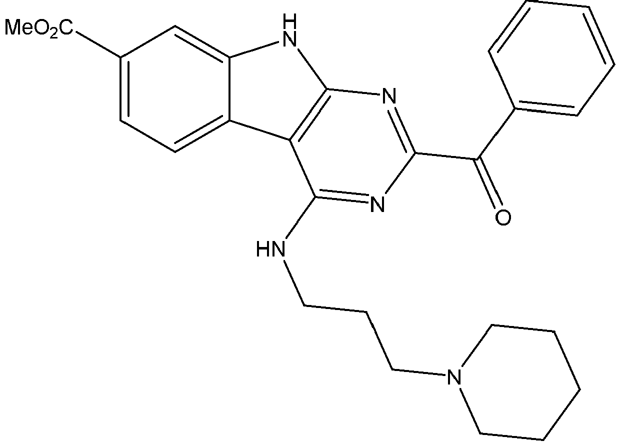 |
| 17 | 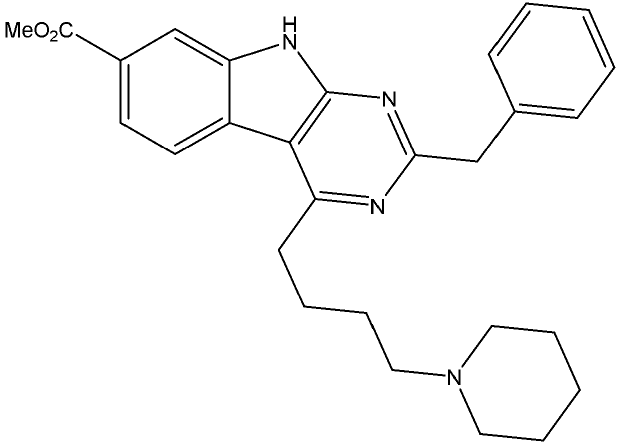 |
| 18 | 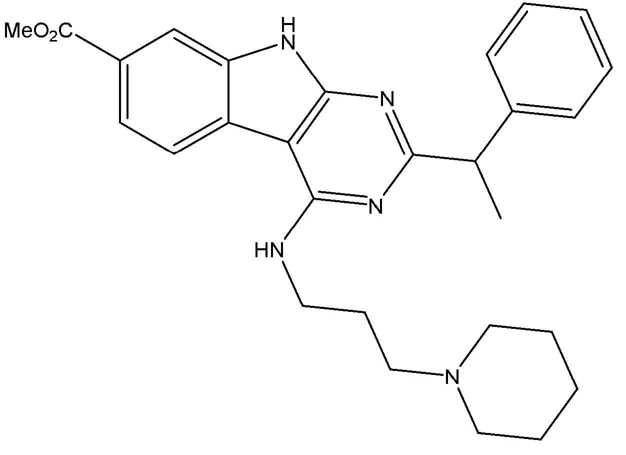 |
| 19 | 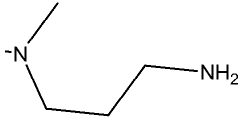 |
| 20 | 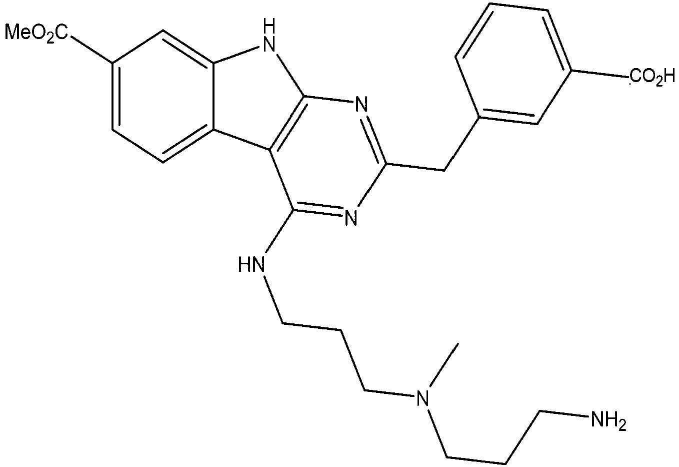 |
| 21 | 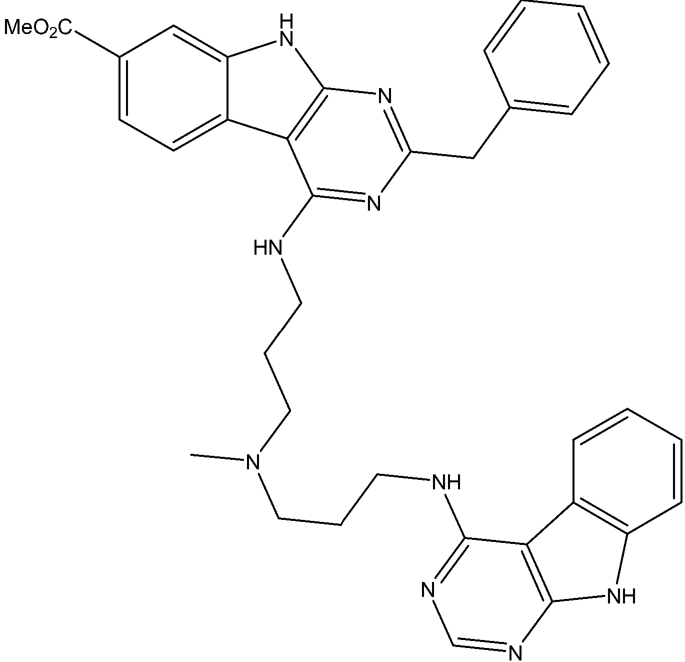 |
| 22 | 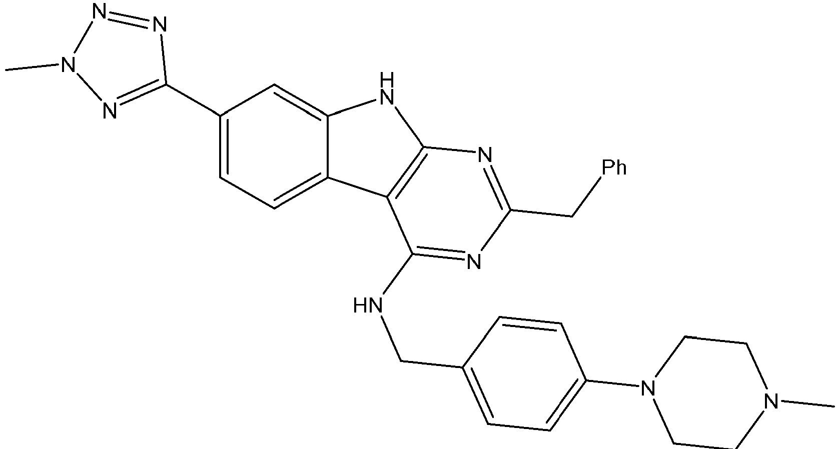 |
| 23 | 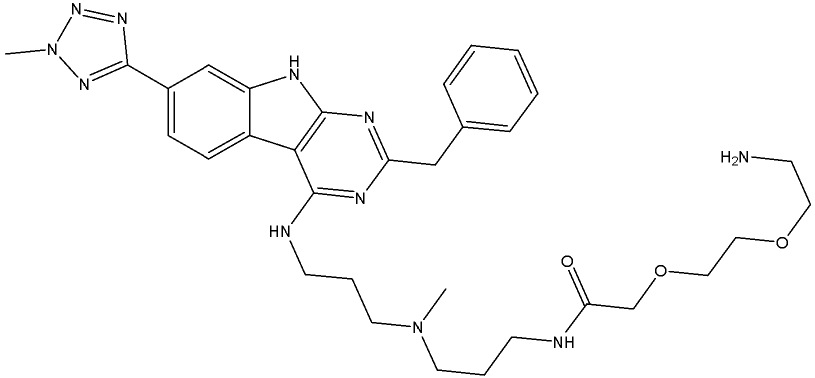 |
| 24 | 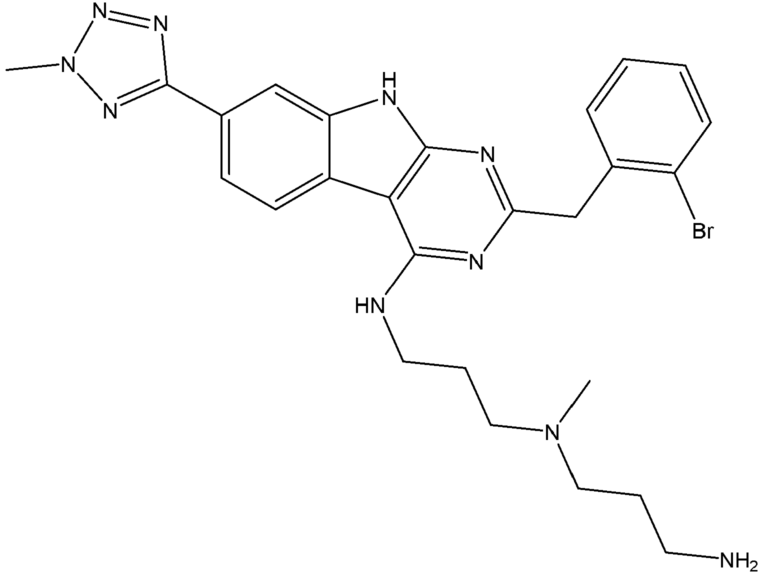 |
| 25 | 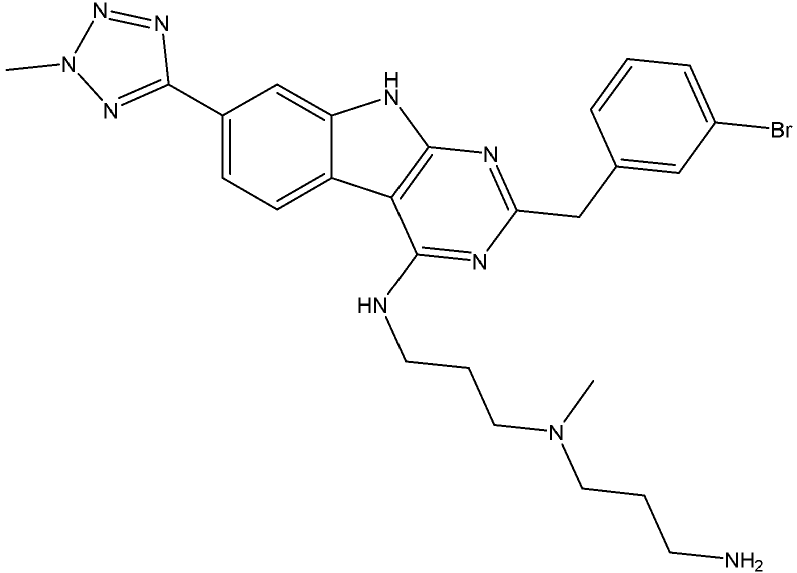 |
| 26 | 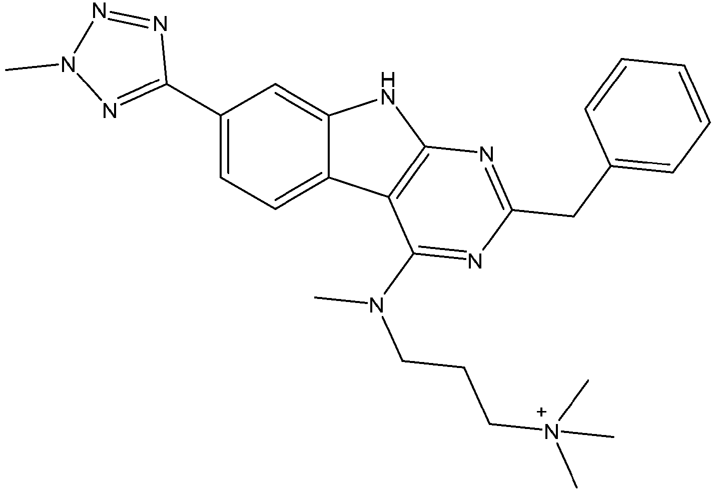 |
| 27 | 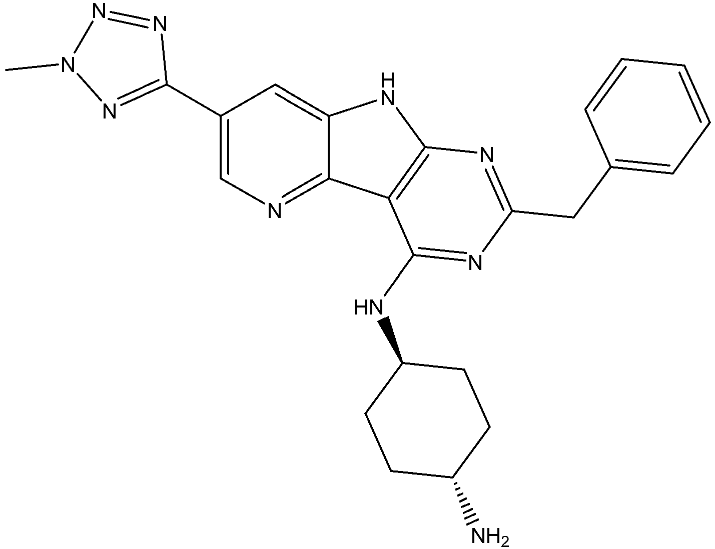 |
| 28 | 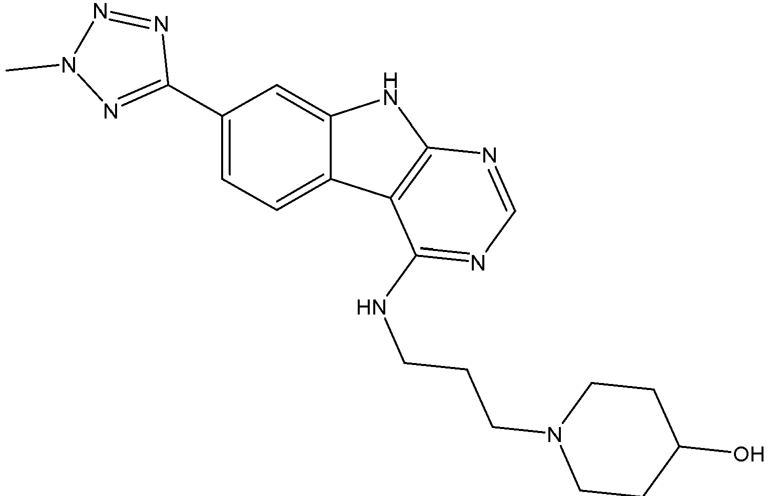 |
| 29 | 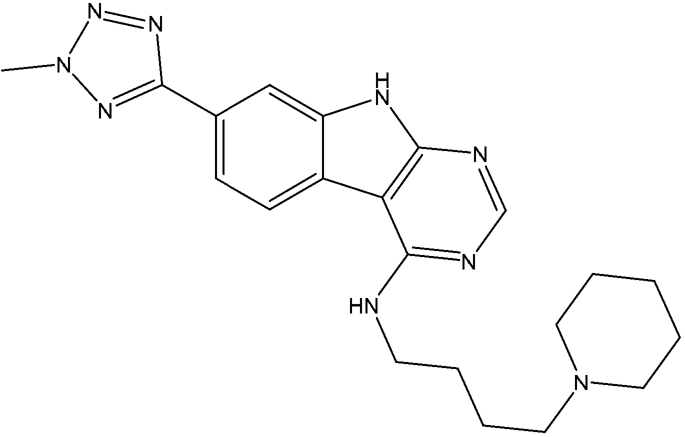 |
| 30 | 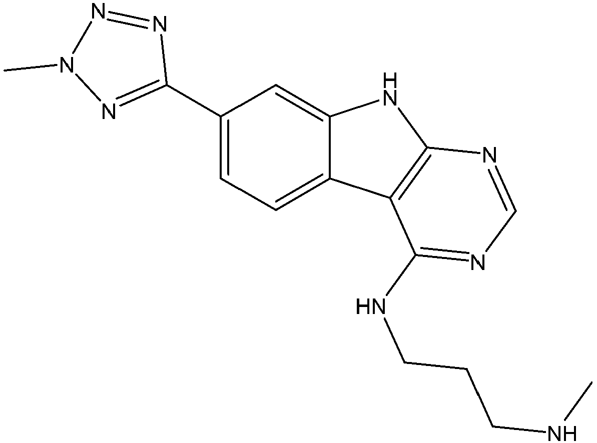 |
| 31 | 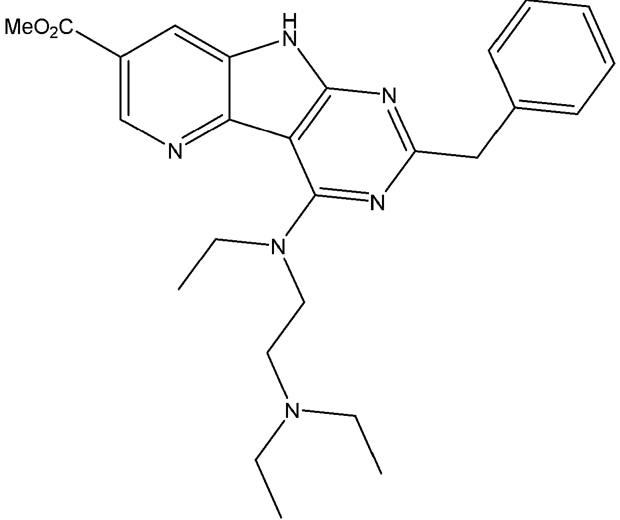 |
| 32 | 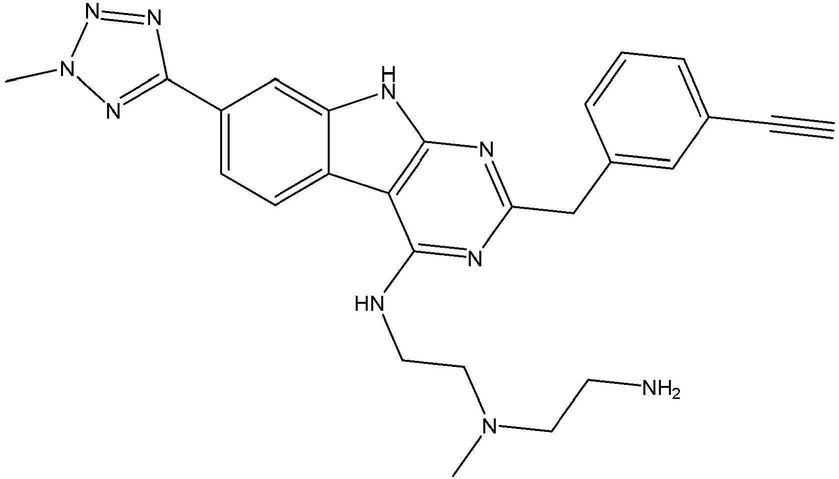 |
| 33 | 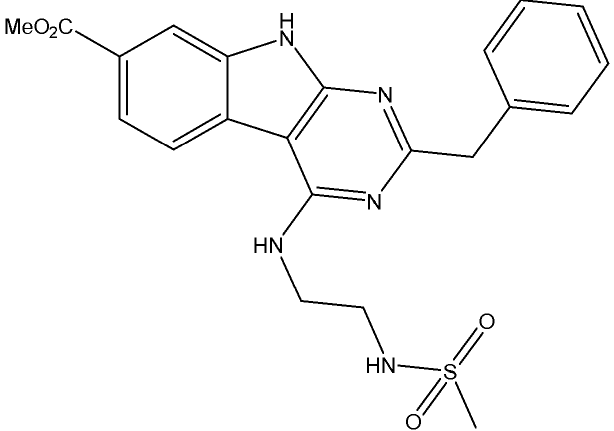 |
| 34 | 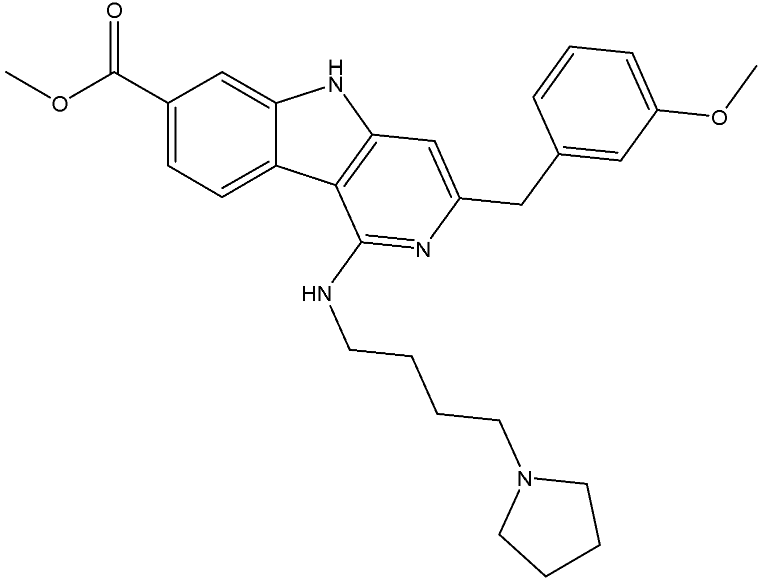 |
| 35 | 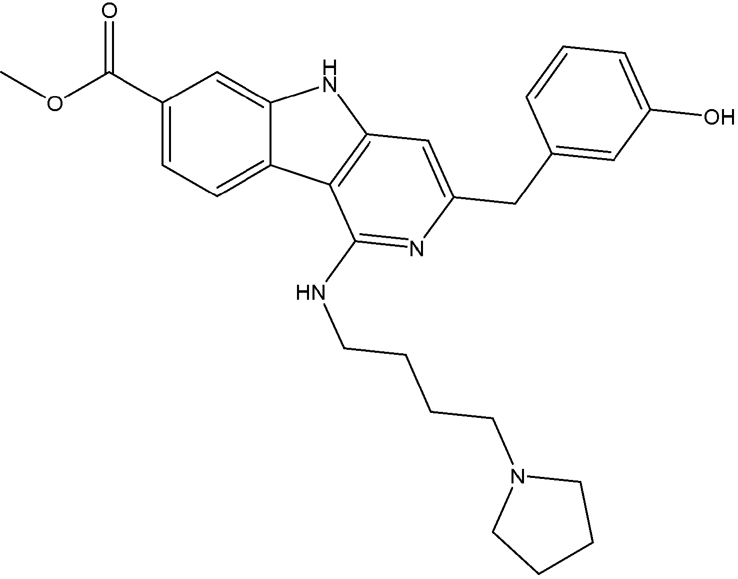 |
| 36 | 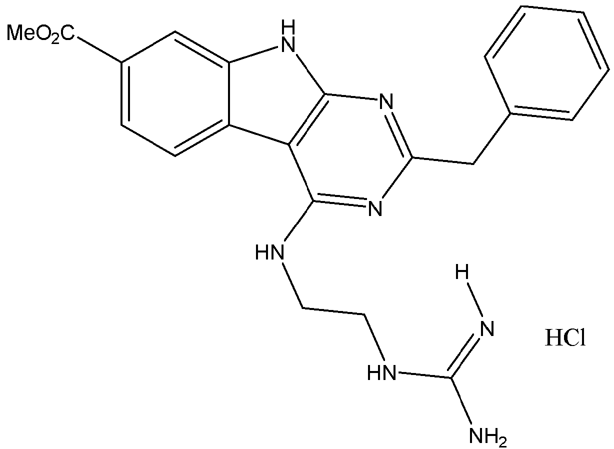 |
| 37 | 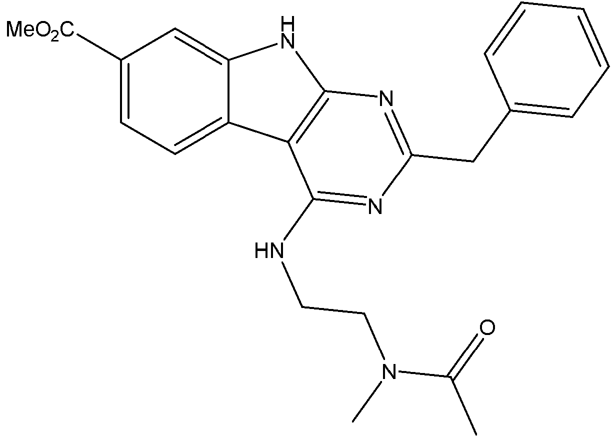 |
| 38 | 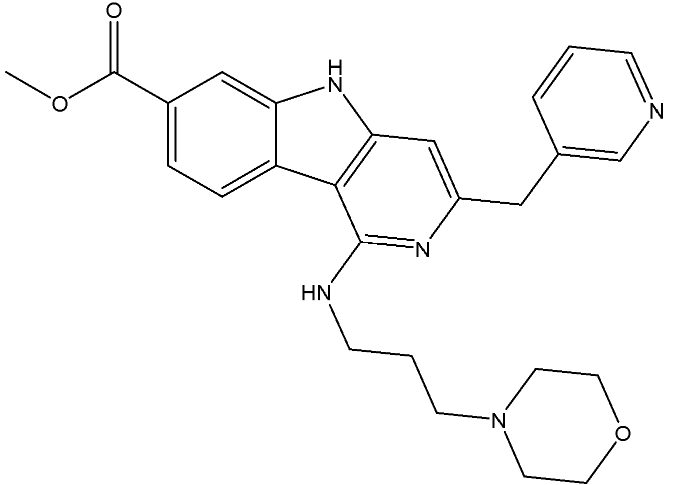 |
| 39 | 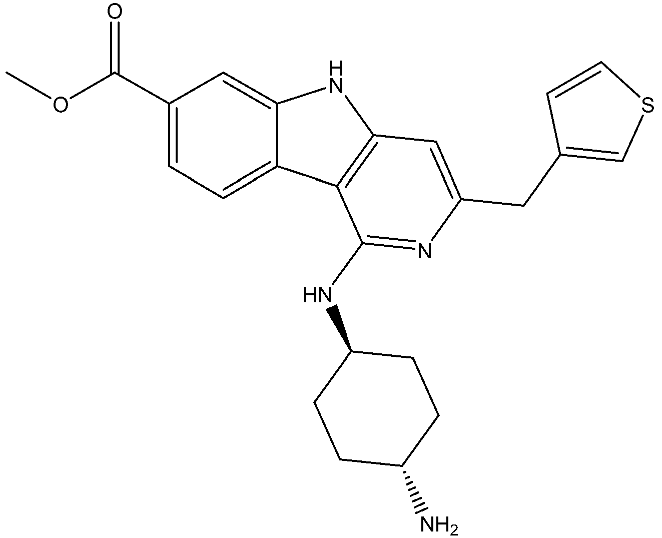 |
| 40 | 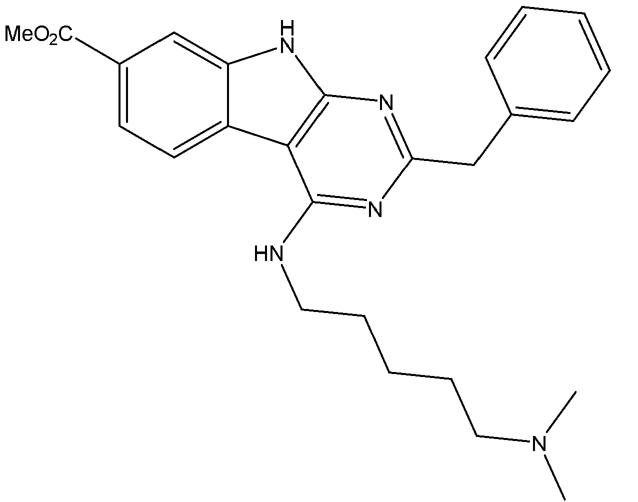 |
| 41 | 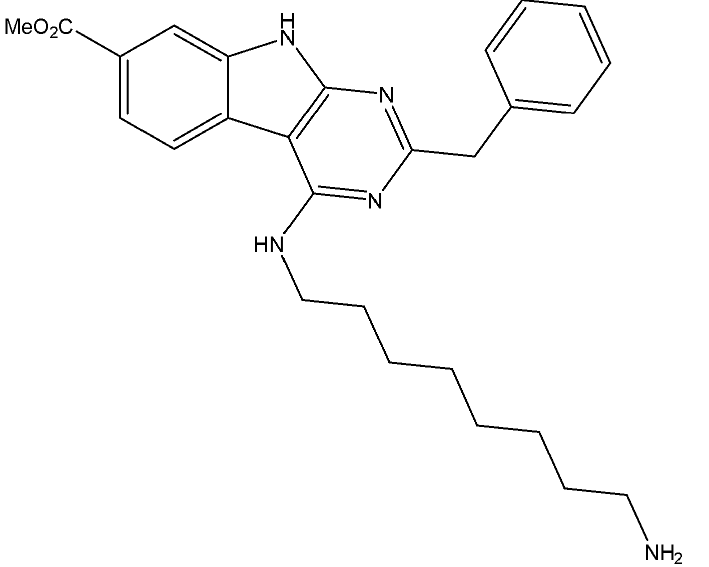 |
| 42 | 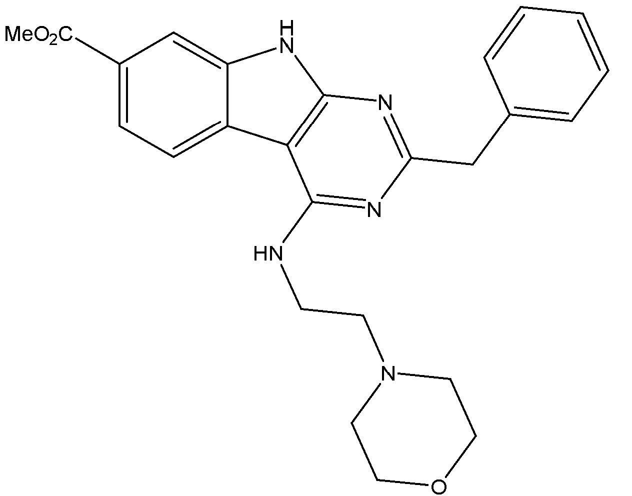 |
| 43 | 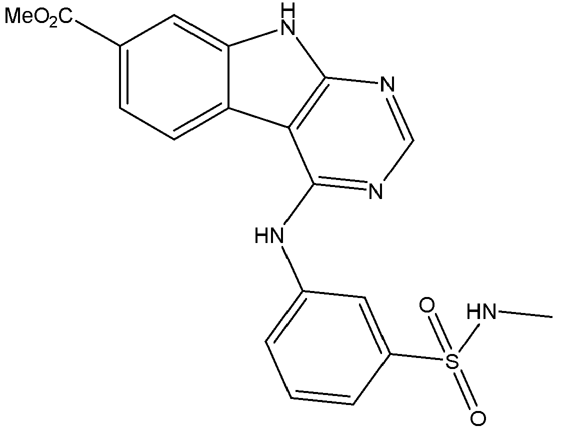 |
| 44 | 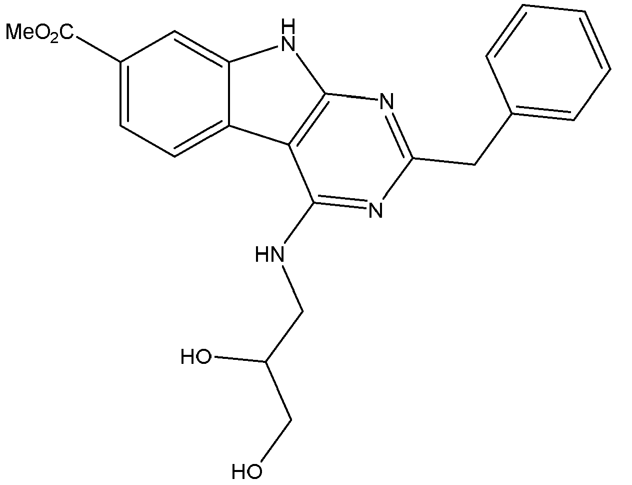 |
| 45 | 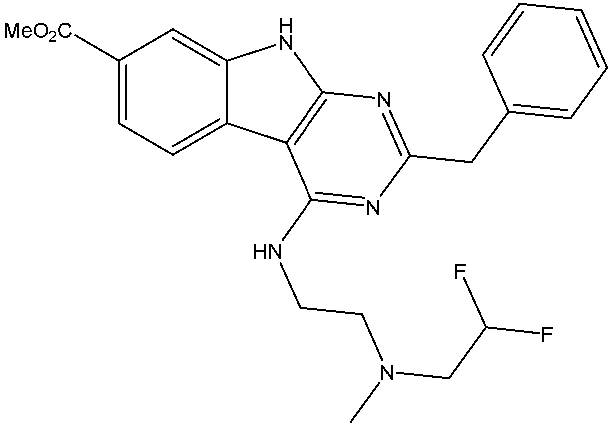 |
| 46 | 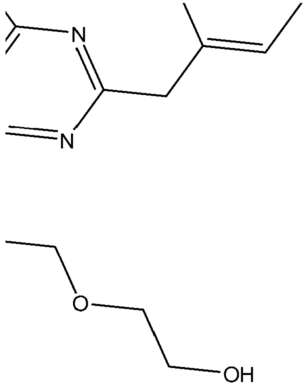 |
| 47 | |
| 48 | 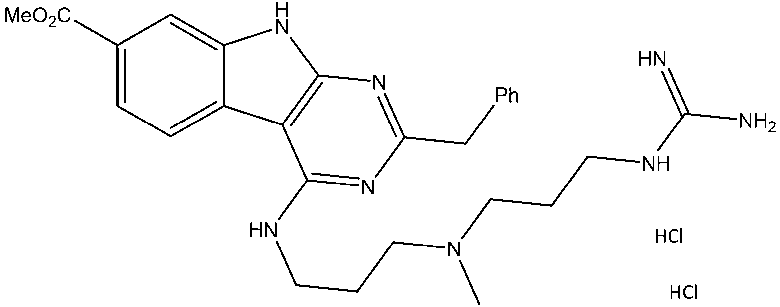 |
| 49 | 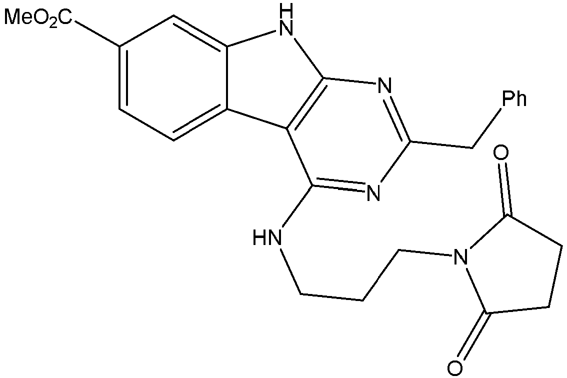 |
| 50 | 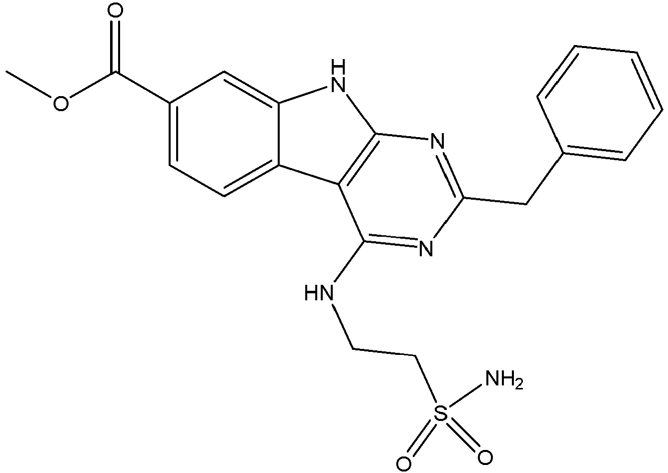 |
| 51 | 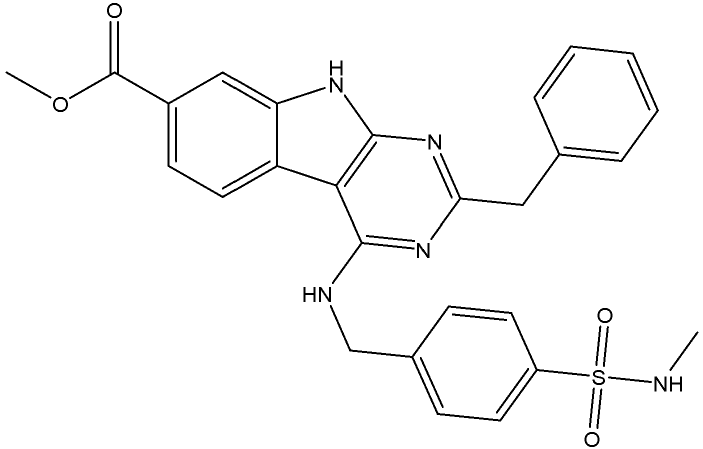 |
| 52 | 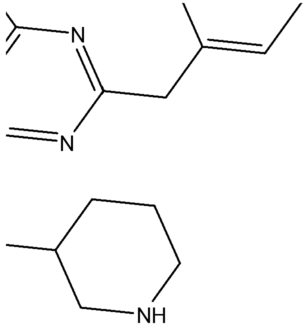 |
| 53 | 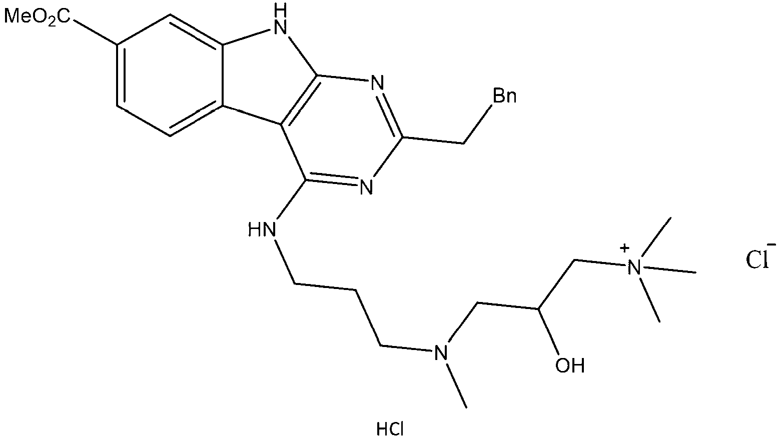 |
| 54 | |
| 55 | 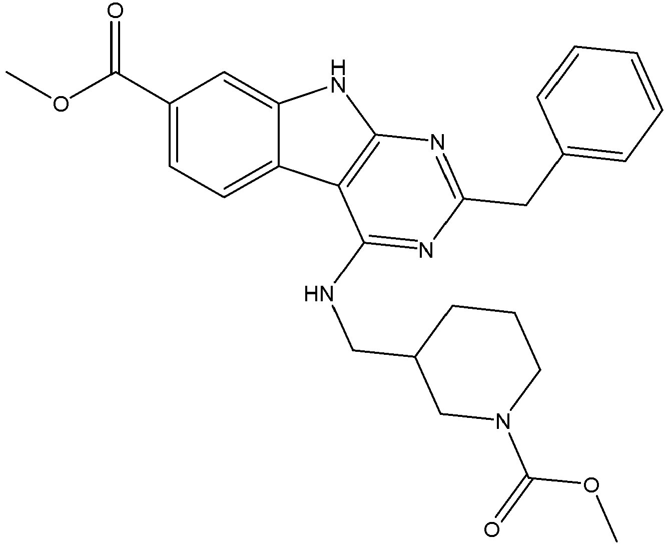 |
| 56 | 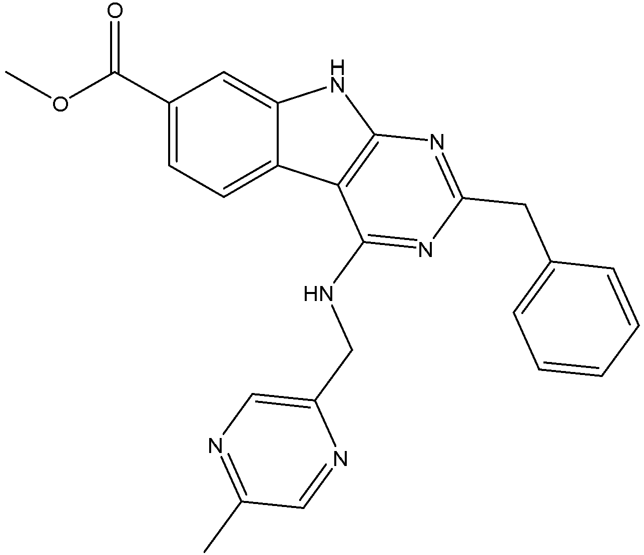 |
| 57 | 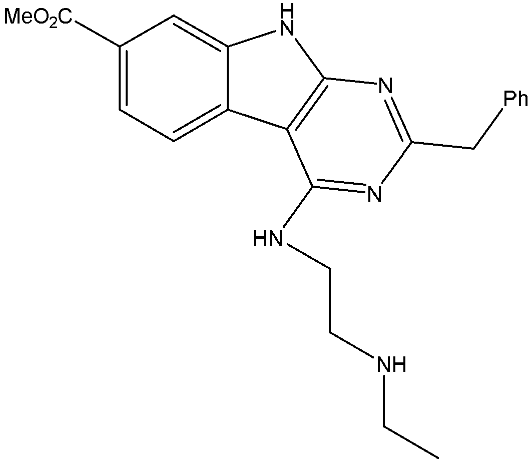 |
| 58 | 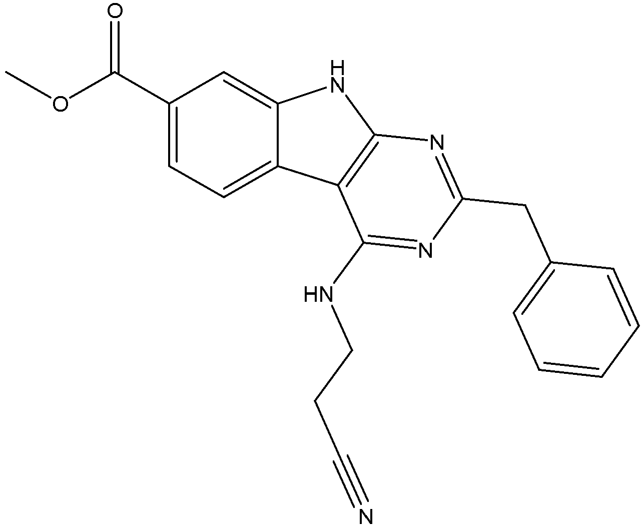 |
| 59 | 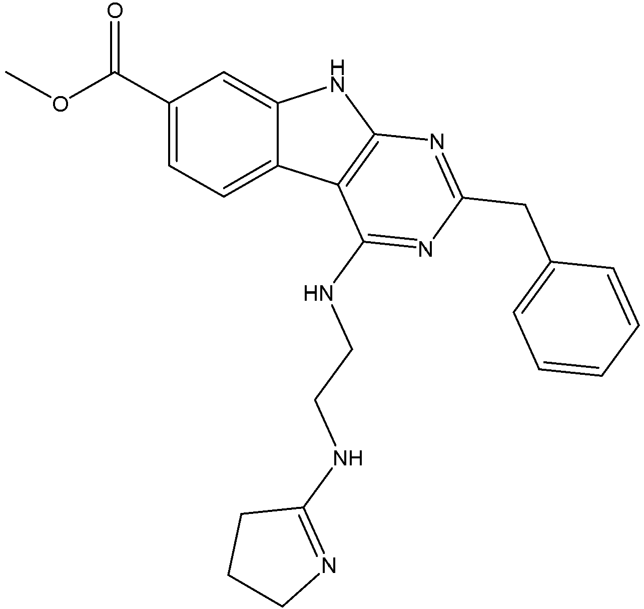 |
| 60 | 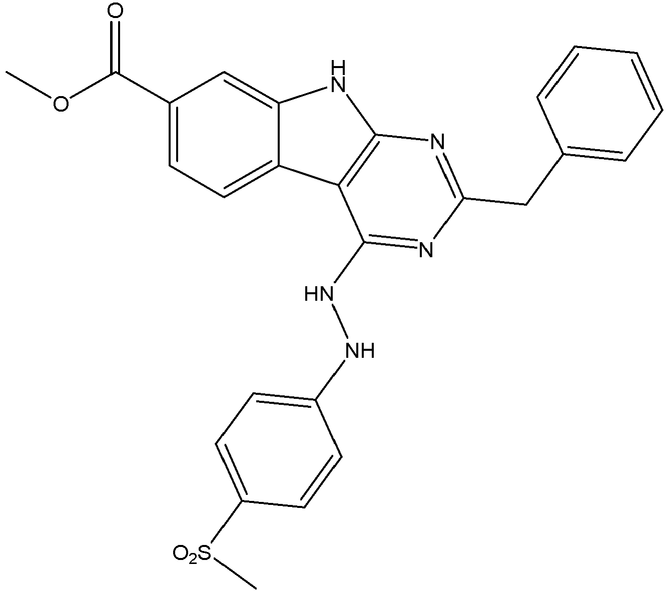 |
| 61 | 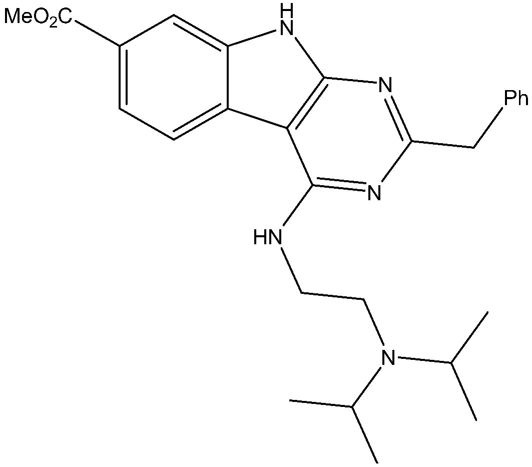 |
oder ein pharmazeutisch akzeptables Salz davon.

10. Transplantat expandierter Stamm- und/oder Progenitorzellen, kultiviert mit wenigstens einer Verbindung, wobei die wenigstens eine Verbindung Formel I hat, zur Verwendung nach irgendeinem der Ansprüche 1-9, wobei der inflammatorische Zustand in dem Patienten kontrolliert wird.

11. Transplantat expandierter Stamm- und/oder Progenitorzellen, kultiviert mit wenigstens einer Verbindung, wobei die wenigstens eine Verbindung Formel I hat, zur Verwendung nach irgendeinem der Ansprüche 1-10, wobei das Transplantat eine dendritische Zellpopulation und Mastzellen umfasst.

12. Transplantat expandierter Stamm- und/oder Progenitorzellen, kultiviert mit wenigstens einer Verbindung, wobei die wenigstens eine Verbindung Formel I hat, zur Verwendung nach irgendeinem der Ansprüche 1-11, wobei das Transplantat FCER1⁺CD34⁺-Zellen, CD34⁺CD45RA⁺-Zellen, CD34⁺CD86⁺-Zellen, CD34⁺CD45RA⁻-Zellen und CD34⁻-Zellen umfasst.

## Revendications

1. Greffon de cellules souches et/ou progénitrices expansées cultivées avec au moins un composé, pour une utilisation dans un procédé de réduction de la maladie du greffon contre l'hôte sévère (GVHD) et/ou d'infections virales sévères, dans lequel ledit greffon conduit à une expansion de cellules dendritiques (DC), de cellules tueuses naturelles (NK) ou d'une combinaison de celles-ci *in vivo* chez un patient, dans lequel ladite expansion de cellules DC, de cellules NK ou de la combinaison de celles-ci stimule une réponse immunitaire chez ledit patient et dans lequel ledit au moins un composé présente la formule I : 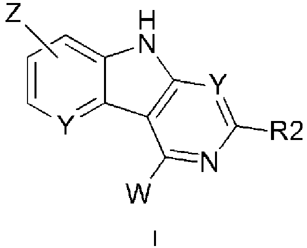 ou un sel de celui-ci,
dans lequel :
chaque Y est indépendamment sélectionné parmi N et CH ;
Z est
- CN
- C(O)OR1,
- C(O)N(R1)R3,
- C(O)R1, ou
- hétéroaryle facultativement substitué par un ou plusieurs substituants RA ou R4,
dans lequel, lorsque (R1) et R3 sont attachés à un atome d'azote, ils se joignent facultativement ensemble avec l'atome d'azote pour former un cycle à 3 à 7 chaînons qui inclut facultativement un ou plusieurs autres hétéroatomes sélectionnés parmi N, O et S, le cycle étant facultativement substitué par un ou plusieurs RA ou R4 ;
W est
- CN,
- N(R1)R3,
- C(O)OR1,
- C(O)N(R1)R3,
- NR1C(O)R1,
- NR1C(O)OR1,
- OC(O)N(R1)R3,
- OC(O)R1,
- C(O)R1,
- NR1C(O)N(R1)R3,
- NR1S(O)₂R1,
- benzyle facultativement substitué par 1, 2 ou 3 substituants RA ou R1,
- X-L-(X-L)n - N(R1)R3,
- X-L-(X-L)n - hétéroaryle facultativement substitué par un ou plusieurs substituants RA ou R4 attachés sur l'un ou l'autre ou les deux groupes L et hétéroaryle,
- X-L-(X-L)n - hétérocycloyle facultativement substitué par un ou plusieurs substituants RA ou R4 attachés sur l'un ou l'autre ou les deux groupes L et hétérocycloyle,
- X-L-(X-L)n- aryle facultativement substitué par un ou plusieurs substituants RA ou R4,
- X-L-(X-L)ₙ-NR1RA ou
- (N(R1)-L)ₙ - N⁺R1R3R5 R6⁻
dans lequel n est un nombre entier égal à soit 0, 1, 2, 3, 4, ou 5,
et dans lequel, lorsque R1 et R3 sont attachés à un atome d'azote, ils se joignent facultativement ensemble avec l'atome d'azote pour former un cycle à 3 à 7 chaînons qui inclut facultativement un ou plusieurs autres hétéroatomes sélectionnés parmi N, O et S, le cycle étant facultativement substitué par un ou plusieurs RA ou R4 ;
chaque X est indépendamment sélectionné parmi C, O, S, et NR1 ;
chaque L est indépendamment
- C₁₋₆ alkylène,
- C₂₋₆ alcénylène,
- C₂₋₆ alcynylène,
- C₃₋₇ cycloalkylène, qui inclut facultativement un ou plusieurs autres hétéroatomes sélectionnés parmi N, O et S ou
- C₃₋₇ cycloalcénylène, qui inclut facultativement un ou plusieurs autres hétéroatomes sélectionnés parmi N, O et S
dans lequel les groupes alkylène, alcénylène, alcynylène, cycloalkylène et cycloalcénylène sont chacun indépendamment facultativement substitués par un ou deux substituants R4 ou RA ; R1 est chacun indépendamment
- H,
- C₁₋₆ alkyle,
- C₂₋₆ alcényle,
- C₂₋₆ alcynyle,
- C₃₋₇ cycloalkyle,
- C₃₋₇ cycloalcényle,
- C₁₋₅ perfluoré,
- hétérocycloyle,
- aryle,
- hétéroaryle, ou
- benzyle,
dans lequel les groupes alkyle, alcényle, alcynyle, cycloalcényle, alkyle perfluoré, hétérocycloyle, aryle, hétéroaryle et benzyle sont chacun indépendamment facultativement substitués par 1, 2 ou 3 substituants RA ou Rd ;
R2 est
- H,
- C₁₋₆ alkyle, facultativement substitué par un ou plusieurs substituants RA
- C(O)R4,
- L-hétéroaryle facultativement substitué par un ou plusieurs substituants RA ou R4
- L-hétérocycloyle facultativement substitué par un ou plusieurs RA ou R4, ou
- L-aryle facultativement substitué par un ou plusieurs substituants RA ou R4 ;
R3 est chacun indépendamment
- H,
- C₁₋₆ alkyle,
- C₂₋₆ alcényle,
- C₂₋₆ alcynyle,
- C₃₋₇ cycloalkyle,
- C₃₋₇ cycloalcényle,
- C₁₋₅ perfluoré,
- hétérocycloyle,
- aryle,
- hétéroaryle, ou
- benzyle,
dans lequel les groupes alkyle, alcényle, alcynyle, cycloalkyle, cycloalcényle, alkyle perfluoré, hétérocycloyle, aryle, hétéroaryle et benzyle sont chacun indépendamment facultativement substitués par 1, 2 ou 3 substituants RA ou Rd ;
R4 est chacun indépendamment
- H,
- C₁₋₆ alkyle,
- C₂₋₆ alcényle,
- C₂₋₆ alcynyle,
- C₃₋₇ cycloalkyle,
- C₃₋₇ cycloalcényle,
- C₁₋₅ perfluoré,
- hétérocycloyle,
- aryle,
- hétéroaryle, ou
- benzyle,
dans lequel les groupes alkyle, alcényle, alcynyle, cycloalkyle, cycloalcényle, alkyle perfluoré, hétérocycloyle, aryle, hétéroaryle et benzyle sont chacun indépendamment facultativement substitués par 1, 2 ou 3 substituants RA ou Rd ;
R5 est chacun indépendamment
- C₁₋₆ alkyle,
- C₁₋₆ alkylène-C₂₋₆ alcényle qui inclut facultativement un ou plusieurs autres hétéroatomes sélectionnés parmi N, O et S
- C₁₋₆ alkylène-C₂₋₆ alcynyle qui inclut facultativement un ou plusieurs autres hétéroatomes sélectionnés parmi N, O et S
- L-aryle qui inclut facultativement un ou plusieurs substituants RA ou R4
- L-hétéroaryle qui inclut facultativement un ou plusieurs substituants RA ou R4
- C₁₋₆ alkylène-C(O)O-
- C₁₋₆ alkylène-C(O)OR1
- C₁₋₆ alkylène-CN
- C₁₋₆ alkylène-C(O)NR1R3, dans lequel R1 et R3 se joignent facultativement avec l'atome d'azote pour former un cycle à 3 à 7 chaînons qui inclut facultativement un ou plusieurs autres hétéroatomes sélectionnés parmi N, O et S ; ou
- C₁₋₆ alkylène-OH ;
R6 est
- Halogène
- OC(O)CF₃ ou
- OC(O)R1 ;
RA est chacun indépendamment
- halogène,
- CF₃,
- OR1,
- L-OR1,
- OCF₃,
- SR1,
- CN,
- NO₂,
- NR1R3,
- L-NR1R1,
- C(O)OR1,
- S(O)₂R4
- C(O)N(R1)R3,
- NR1C(O)R1,
- NR1C(O)OR1,
- OC(O)N(R1)R3,
- OC(O)R1,
- C(O)R4,
- NHC(O)N(R1)R3,
- NR1C(O)N(R1)R3, ou
- N₃ ; et
Rd est chacun indépendamment
- H,
- C₁₋₆ alkyle,
- C₂₋₆ alcényle,
- C₂₋₆ alcynyle,
- C₃₋₇ cycloalkyle,
- C₃₋₇ cycloalcényle,
- C₁₋₅ perfluoré
- benzyle ou
- hétérocycloyle.

2. Greffon de cellules souches et/ou progénitrices expansées cultivées avec au moins un composé, ledit au moins un composé présentant la formule I, pour l'utilisation selon la revendication 1, dans lequel les cellules souches et/ou progénitrices sont des cellules souches hématopoïétiques humaines (HSC).

3. Greffon de cellules souches et/ou progénitrices expansées cultivées avec au moins un composé, ledit au moins un composé présentant la formule I, pour l'utilisation selon la revendication 2, dans lequel les cellules souches hématopoïétiques proviennent de cellules de sang de cordon ombilical, de cellules de sang périphérique mobilisé, ou de cellules de moelle osseuse.

4. Greffon de cellules souches et/ou progénitrices expansées cultivées avec au moins un composé, ledit au moins un composé présentant la formule I, pour l'utilisation selon l'une quelconque des revendications 1-3, dans lequel lesdites cellules NK sont des cellules CD56⁺ ou NKG2A⁺ et lesdites cellules DC sont des cellules CD11c⁺.

5. Greffon de cellules souches et/ou progénitrices expansées cultivées avec au moins un composé, ledit au moins un composé présentant la formule I, pour l'utilisation selon l'une quelconque des revendications 1-4, dans lequel lesdites cellules souches et progénitrices sont en outre cultivées avec un antagoniste du récepteur des hydrocarbures aryliques (AHR) ; de préférence, l'antagoniste de l'AHR est Stem Regenin 1 (SR1) ou CH223191.

6. Greffon de cellules souches et/ou progénitrices expansées cultivées avec au moins un composé, ledit au moins un composé présentant la formule I, pour l'utilisation selon l'une quelconque des revendications 1-5, dans lequel le composé de formule I est UM171 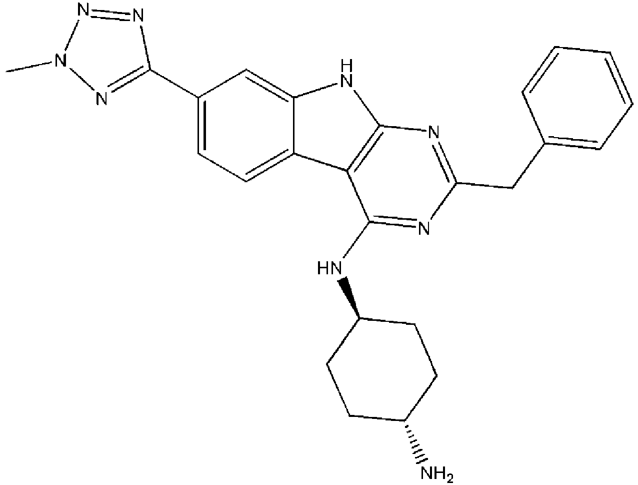 ou d'un sel pharmaceutiquement acceptable de celui-ci.

7. Greffon de cellules souches et/ou progénitrices expansées cultivées avec au moins un composé, ledit au moins un composé présentant la formule I, pour l'utilisation selon l'une quelconque des revendications 1-5, dans lequel le composé de formule I est un sel d'hydrobromure de

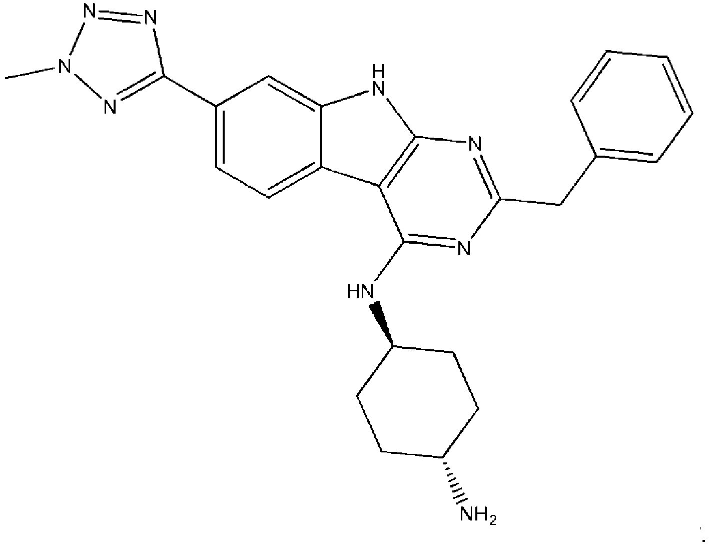

8. Greffon de cellules souches et/ou progénitrices expansées cultivées avec au moins un composé, ledit au moins un composé présentant la formule I, pour l'utilisation selon l'une quelconque des revendications 1-5, dans lequel le composé de formule I est 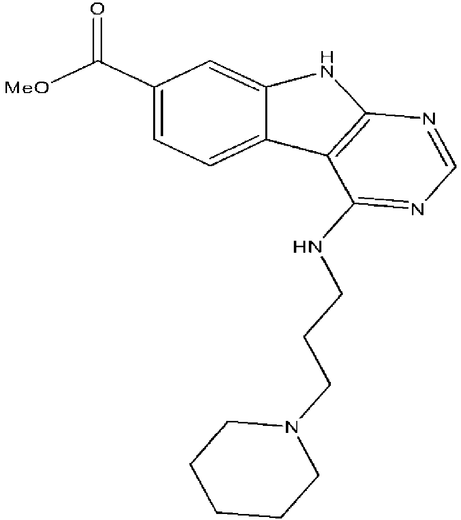 ou un sel pharmaceutiquement acceptable de celui-ci.

9. Greffon de cellules souches et/ou progénitrices expansées cultivées avec au moins un composé, ledit au moins un composé présentant la formule I, pour l'utilisation selon l'une quelconque des revendications 1-5, dans lequel le composé de formule I est :
| | |
|---|---|
| 1 | 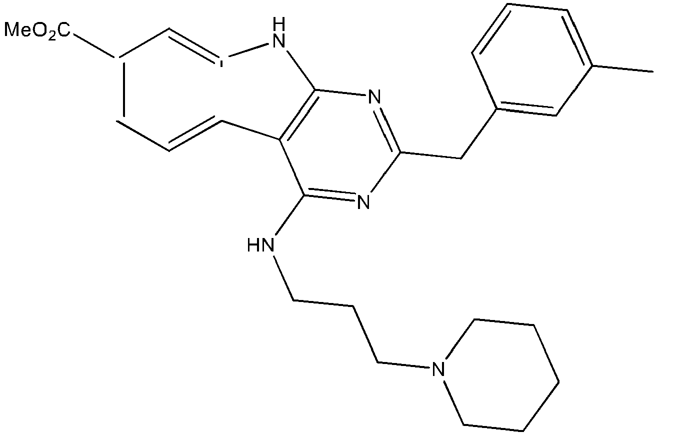 |
| 2 | 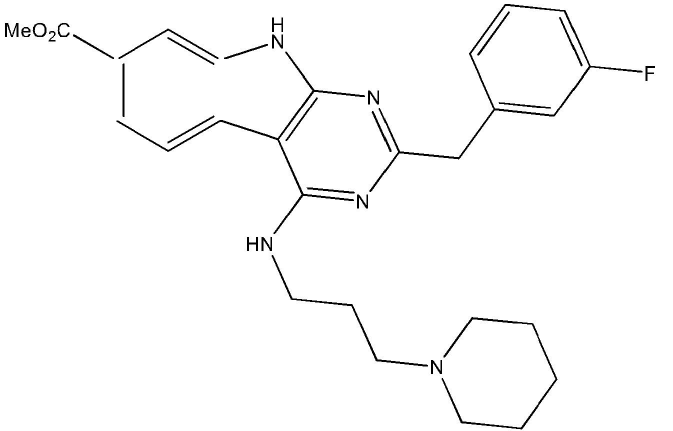 |
| 3 | 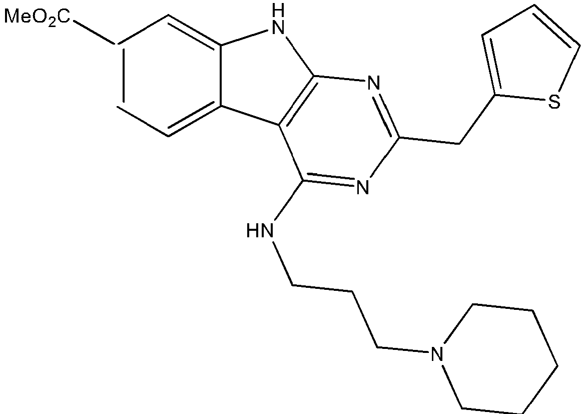 |
| 4 | 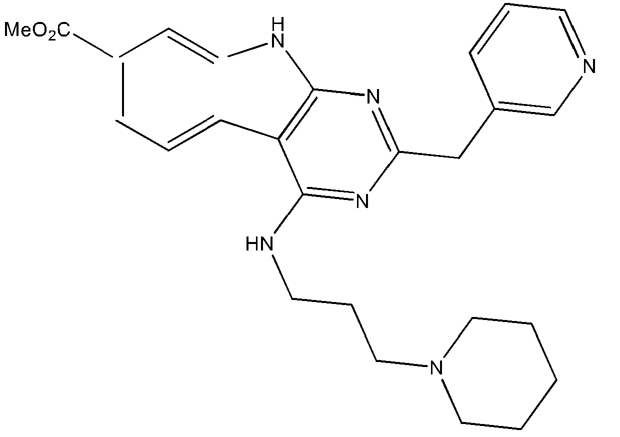 |
| 5 | 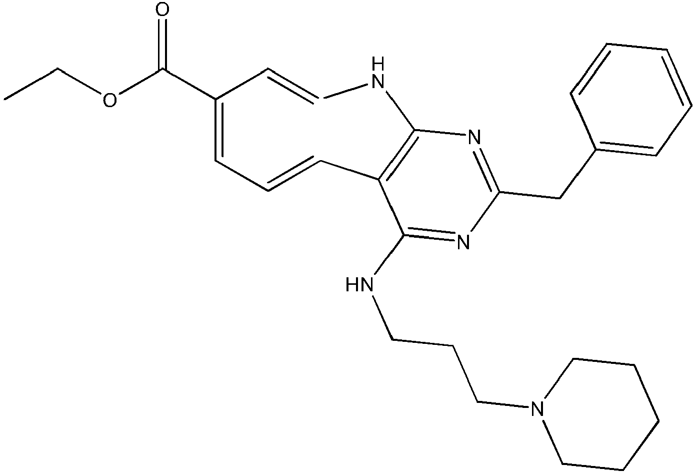 |
| 6 | 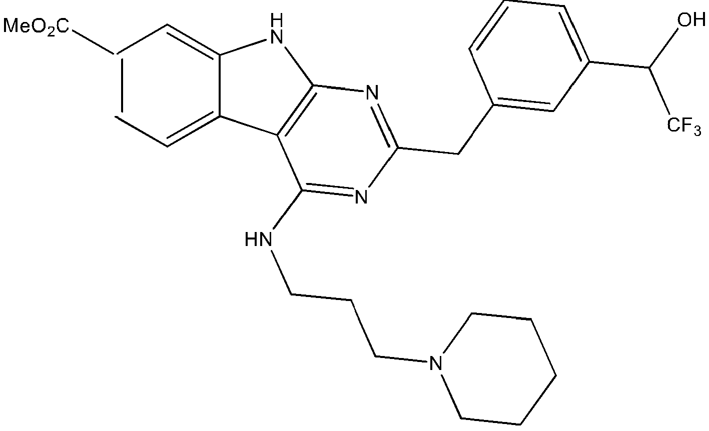 |
| 7 | 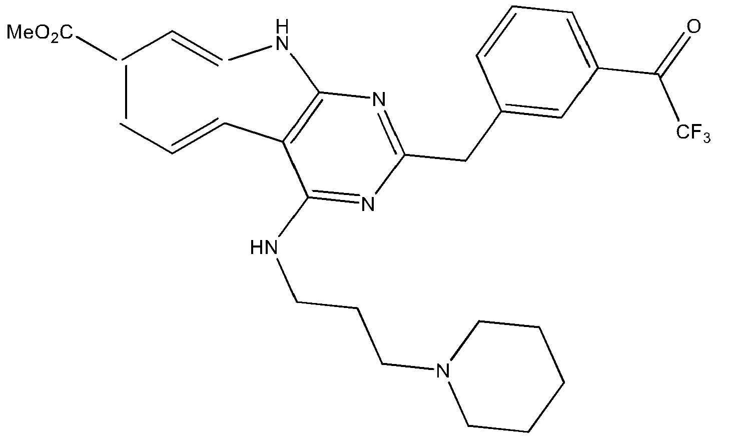 |
| 8 | 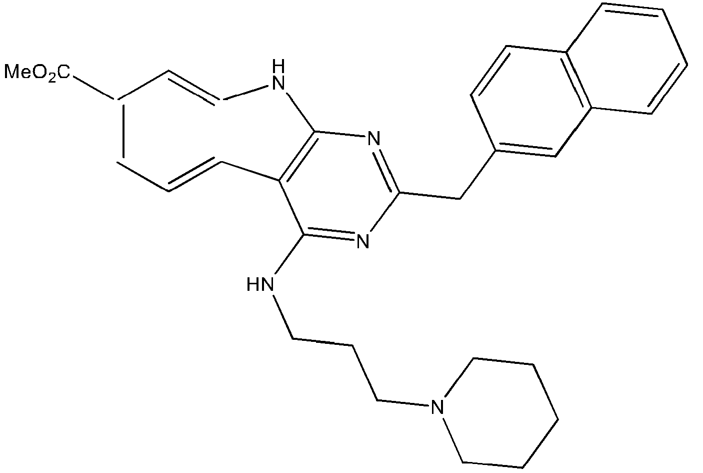 |
| 9 | 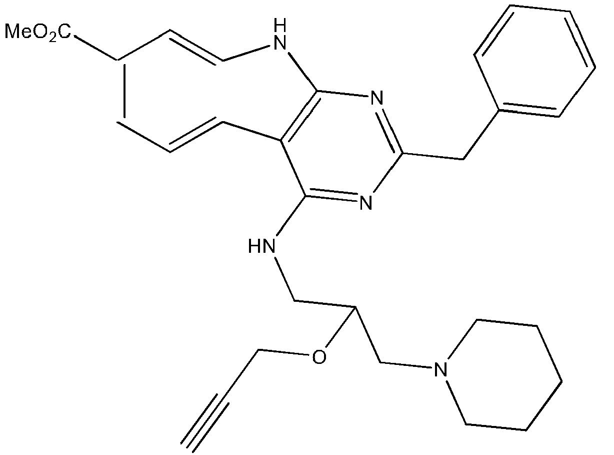 |
| 10 | 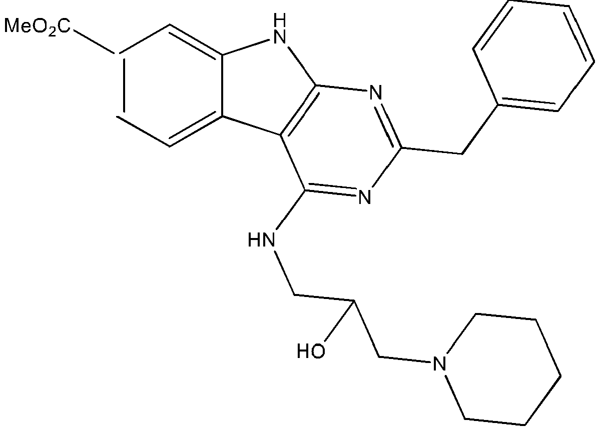 |
| 11 | 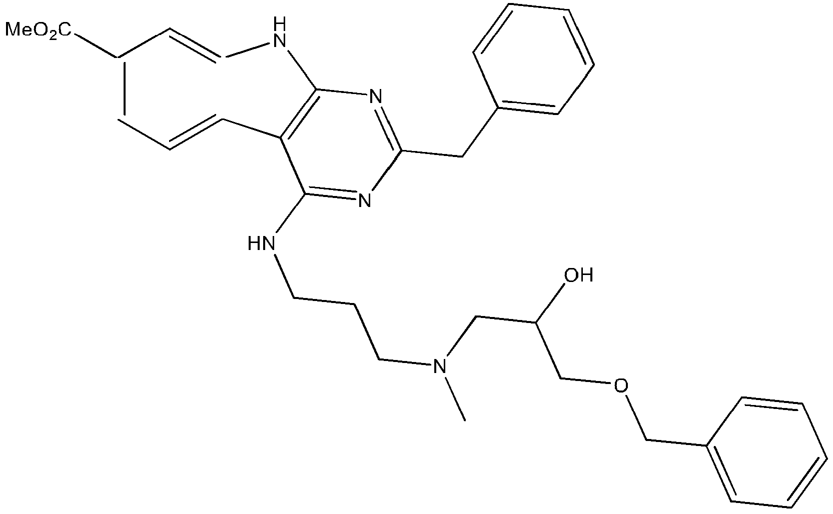 |
| 12 | 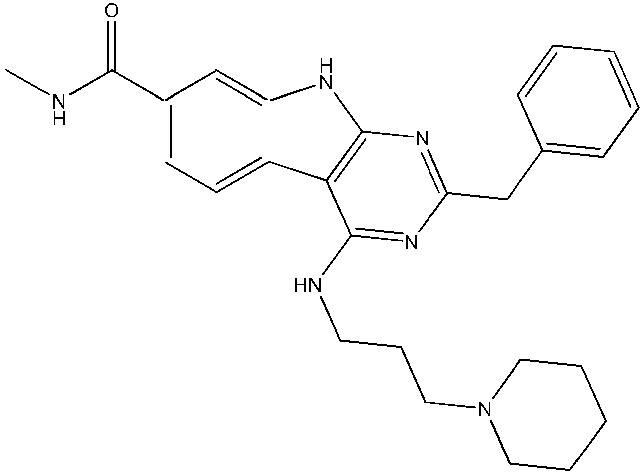 |
| 13 | 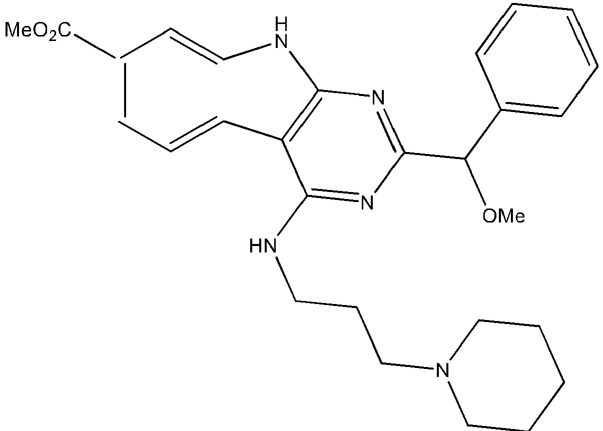 |
| 14 | 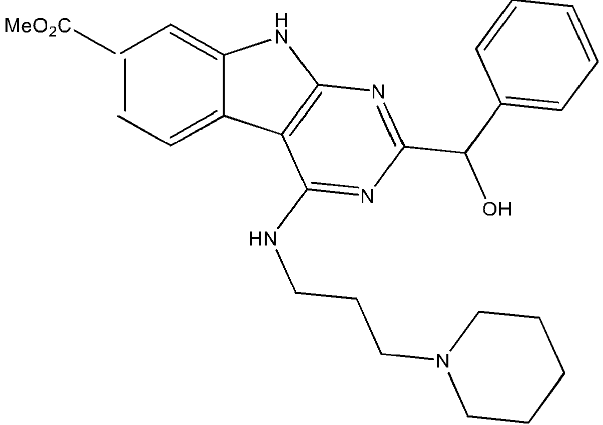 |
| 15 | 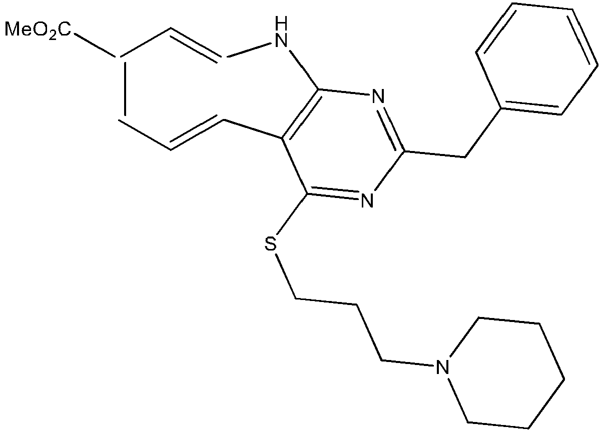 |
| 16 | 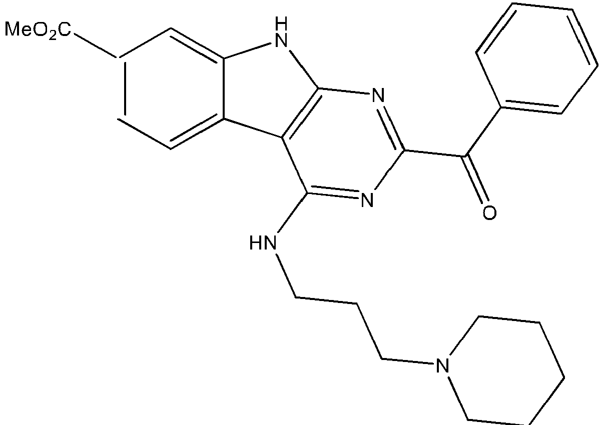 |
| 17 | 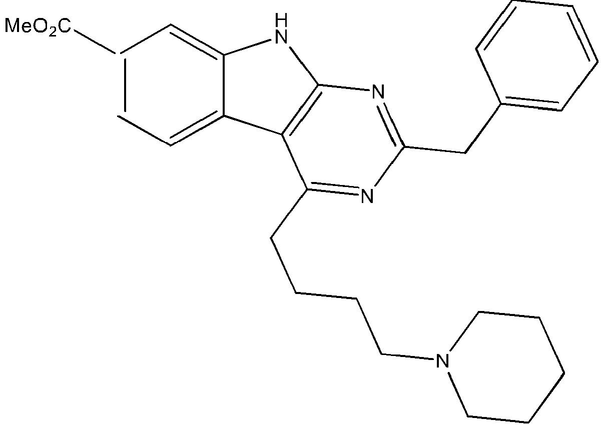 |
| 18 | 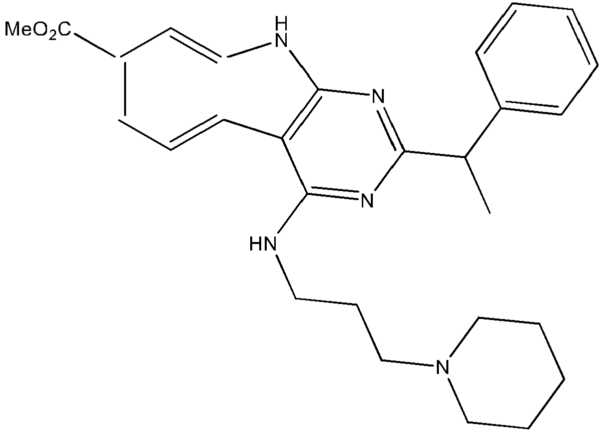 |
| 19 | 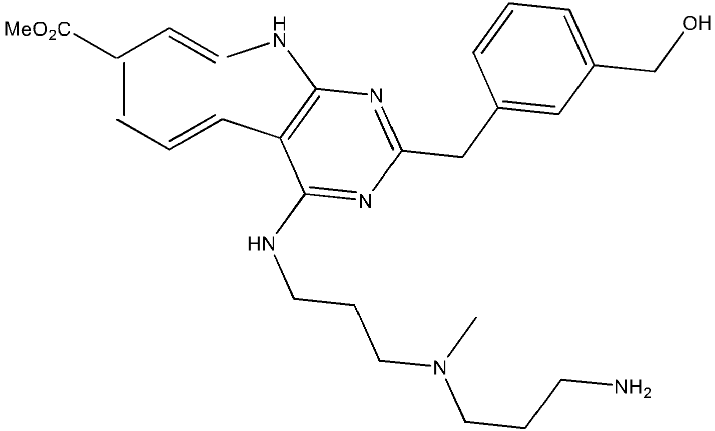 |
| 20 | 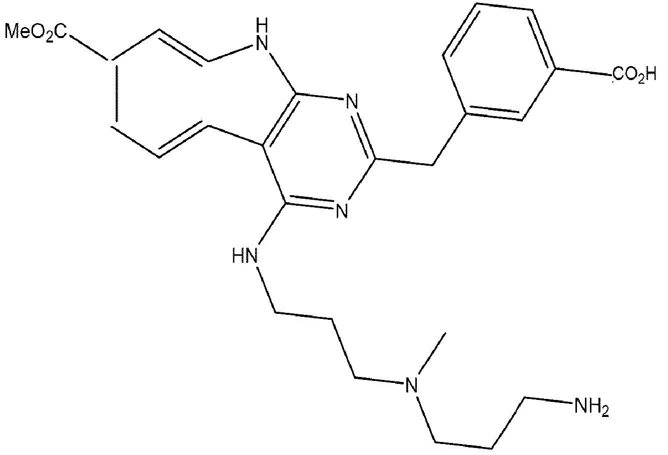 |
| 21 | 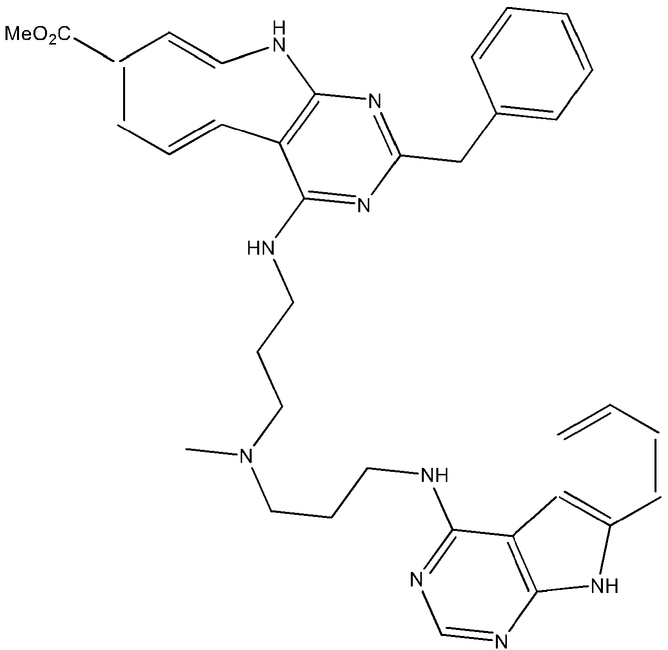 |
| 22 | 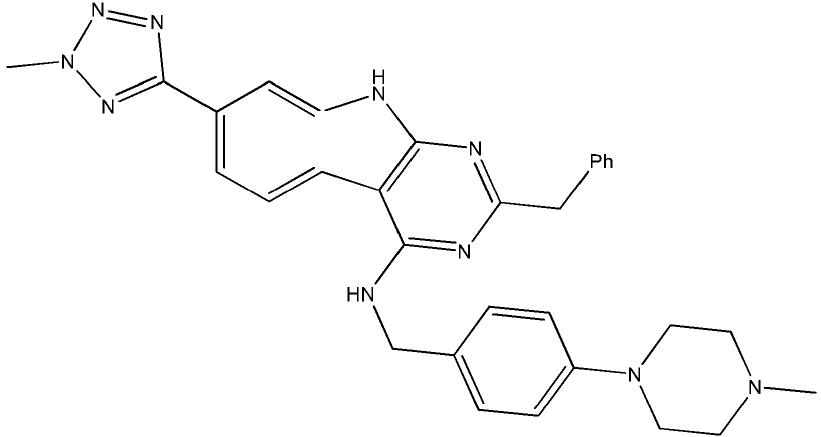 |
| 23 | 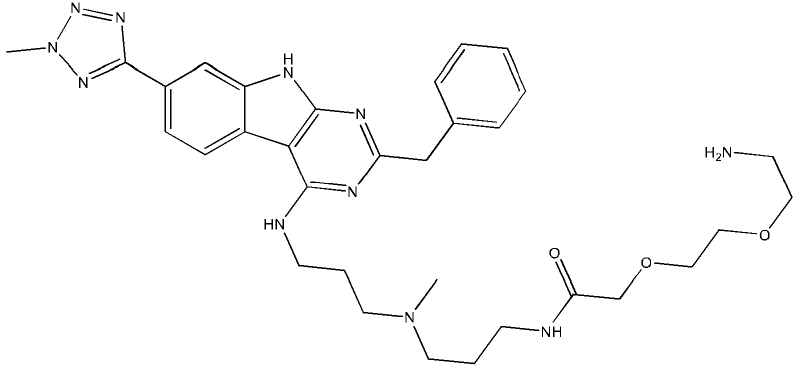 |
| 24 | 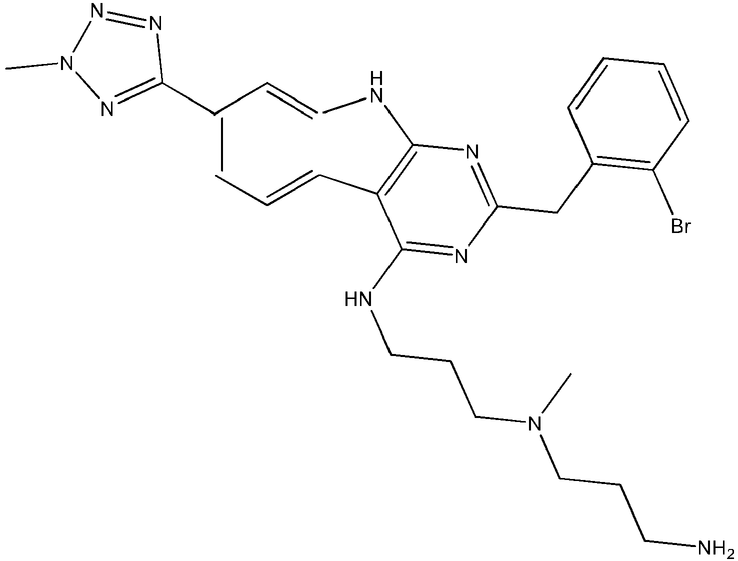 |
| 25 | 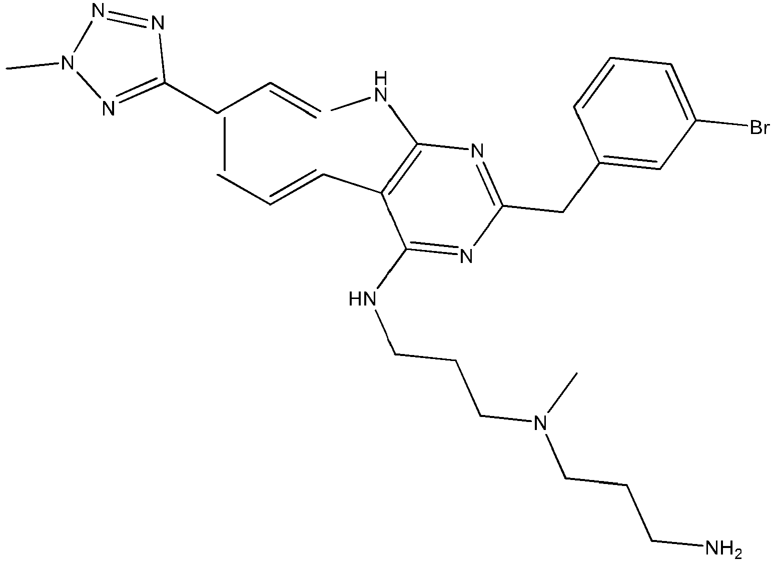 |
| 26 | 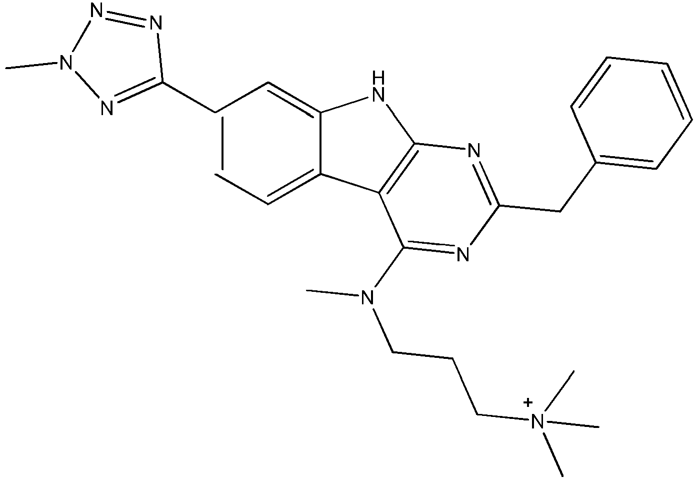 |
| 27 | 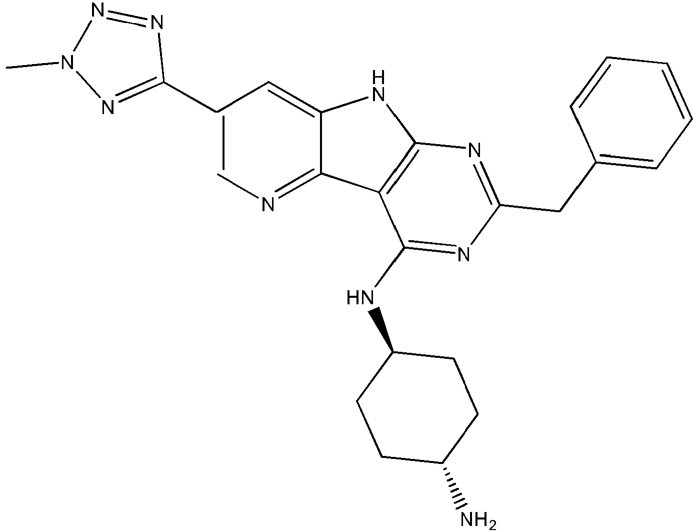 |
| 28 | 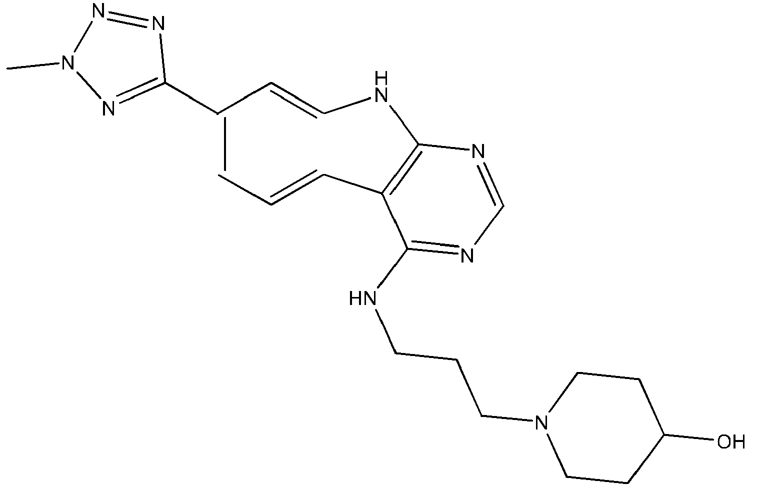 |
| 29 | 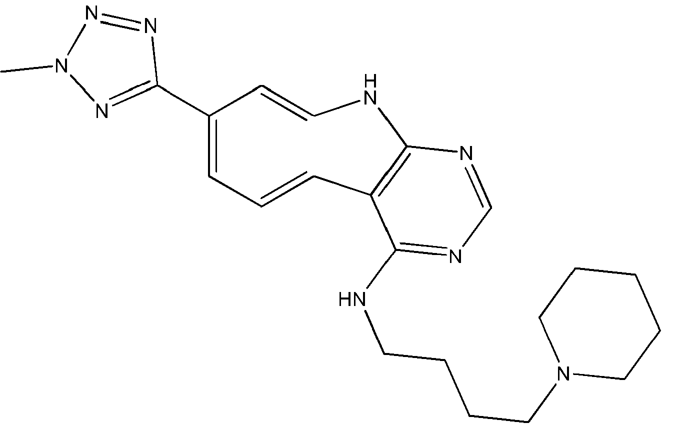 |
| 30 | 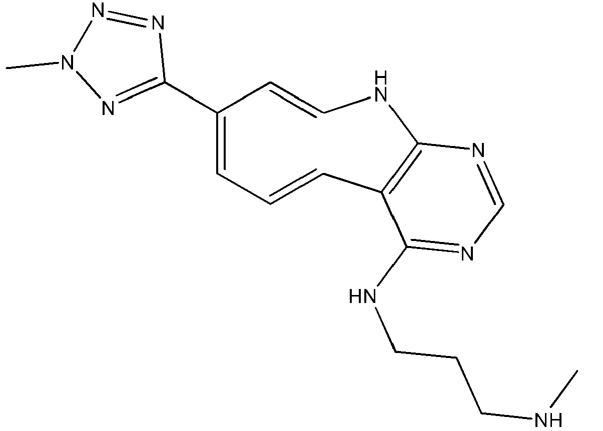 |
| 31 | 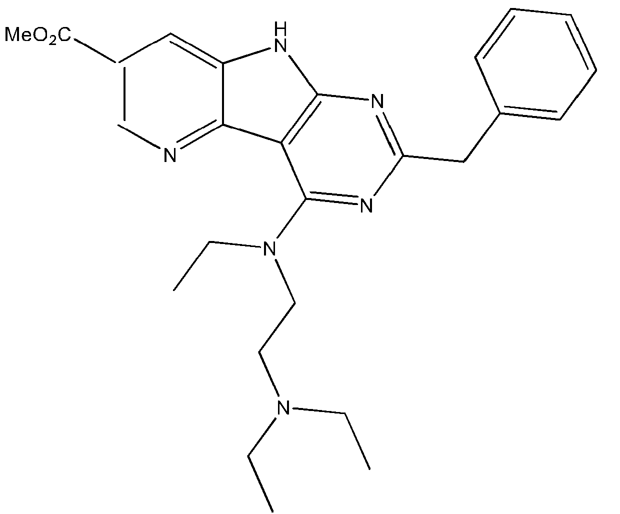 |
| 32 | 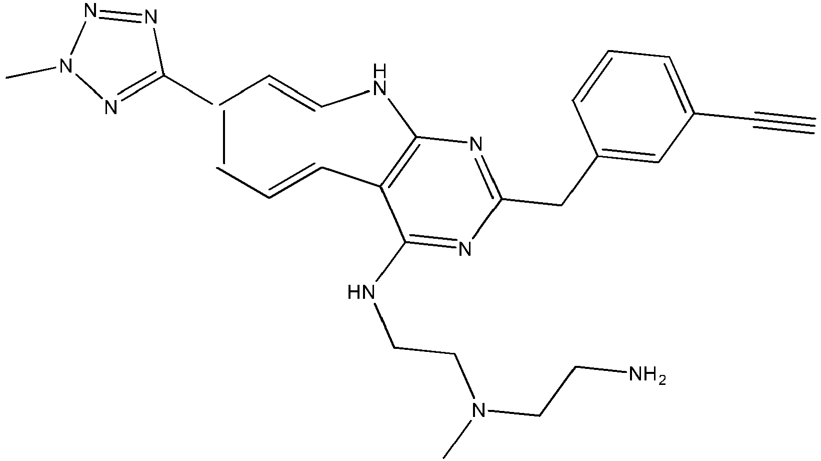 |
| 33 | 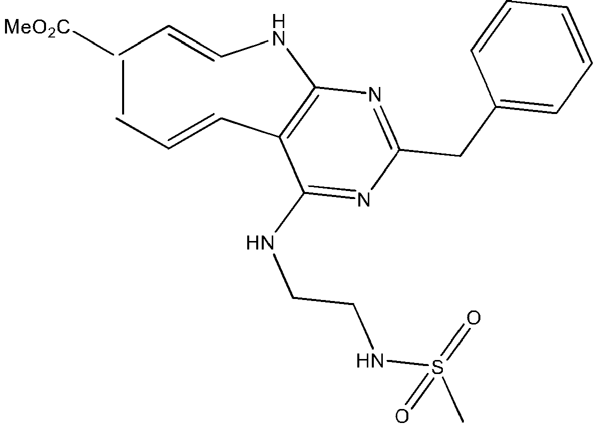 |
| 34 | 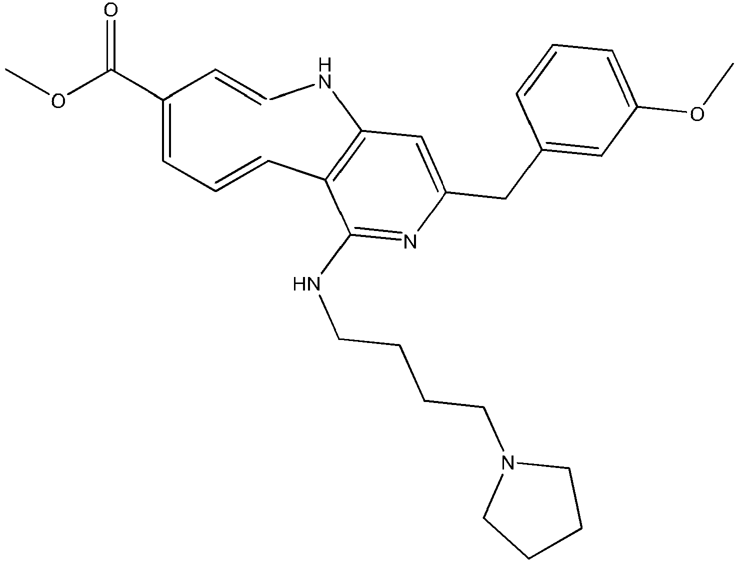 |
| 35 | 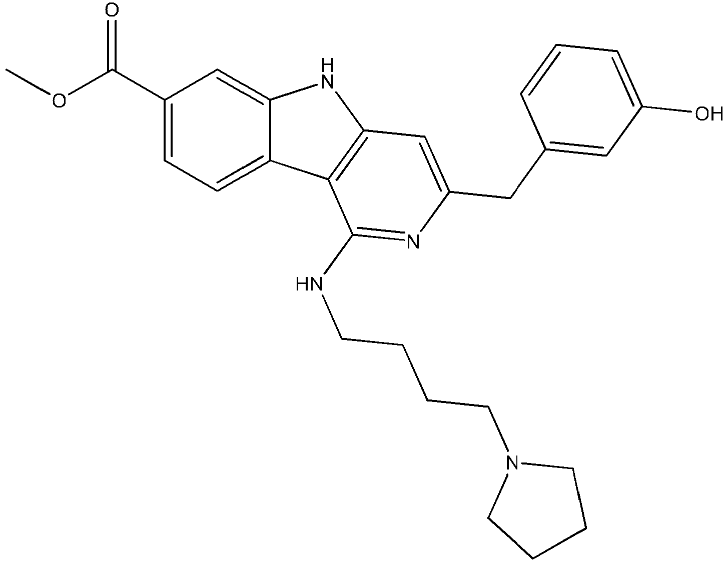 |
| 36 | 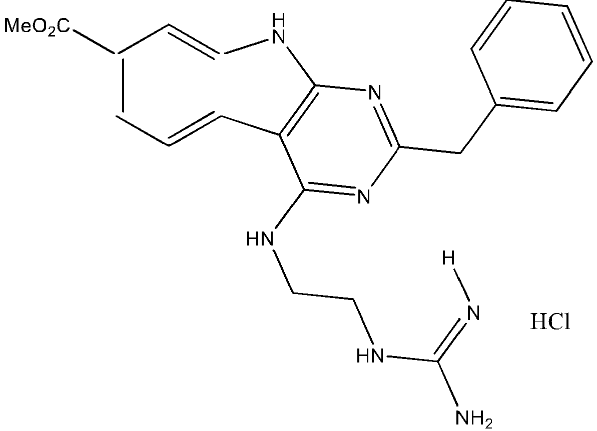 |
| 37 | 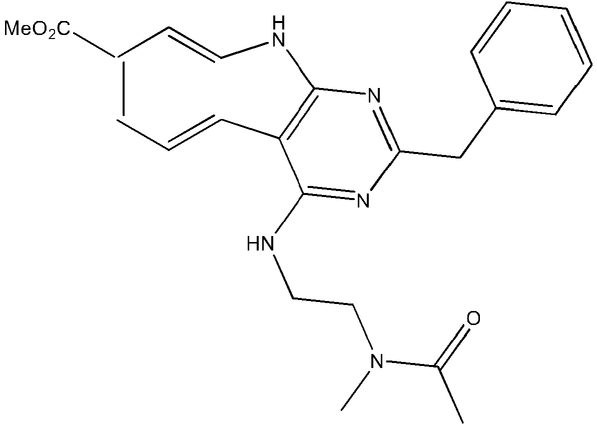 |
| 38 | 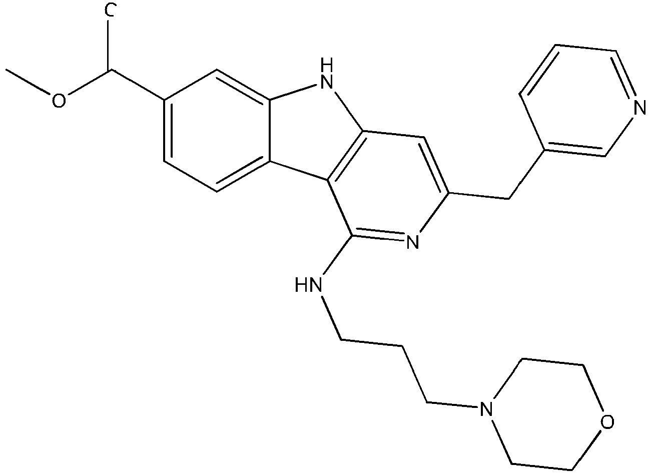 |
| 39 | 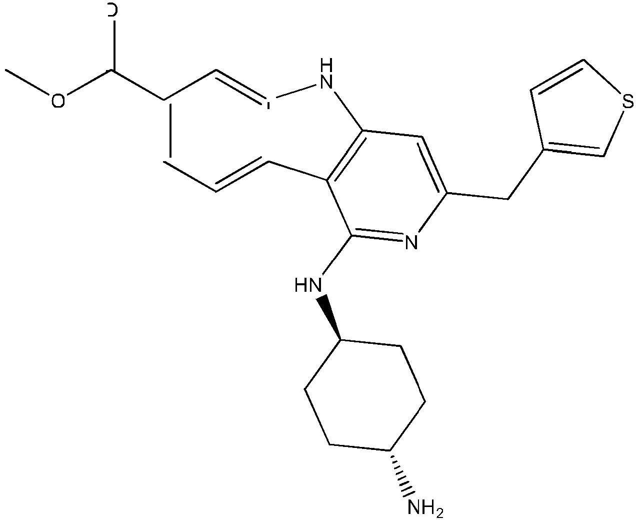 |
| 40 | 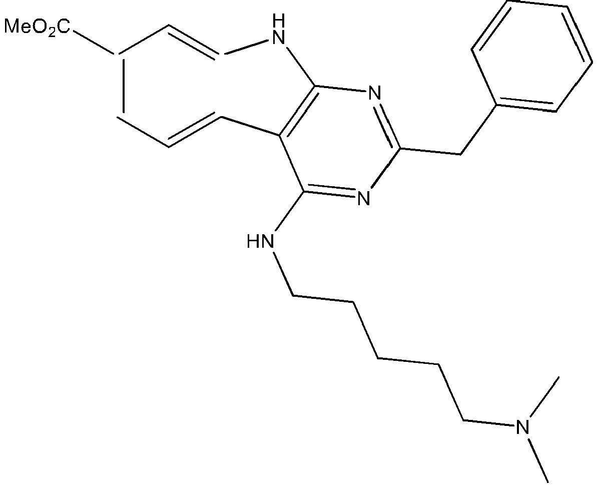 |
| 41 | 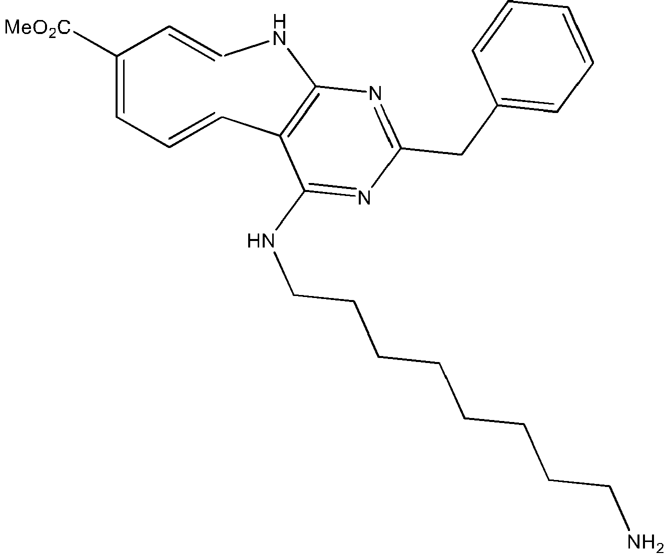 |
| 42 | 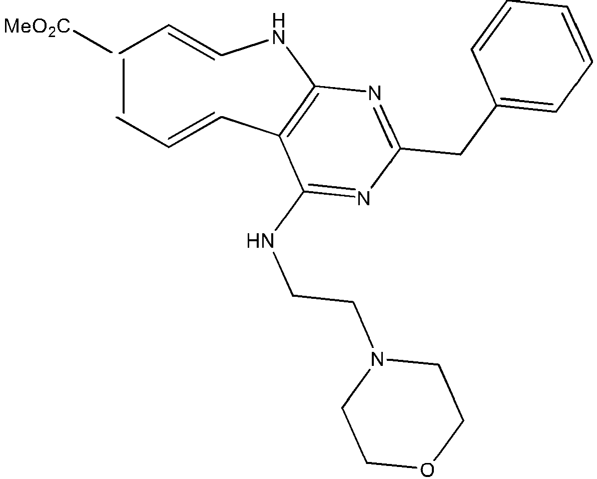 |
| 43 | 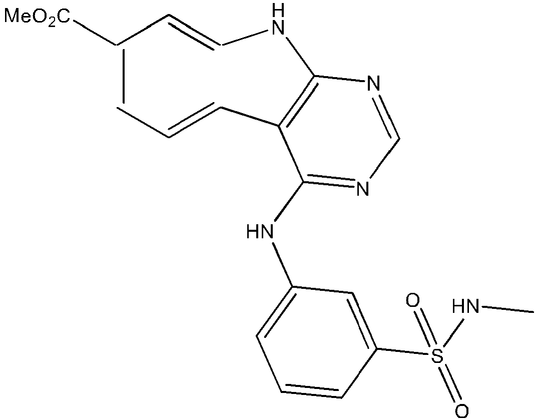 |
| 44 | 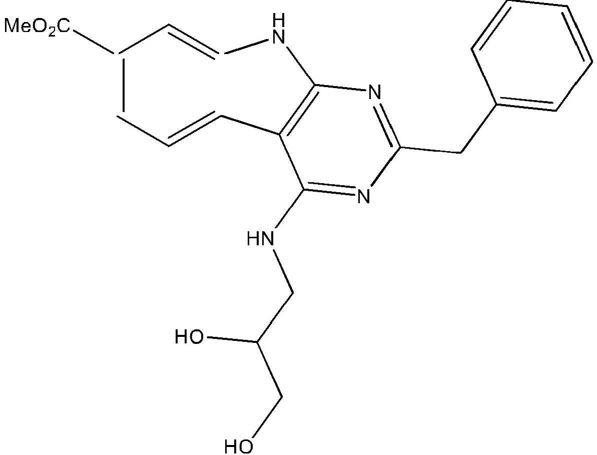 |
| 45 | 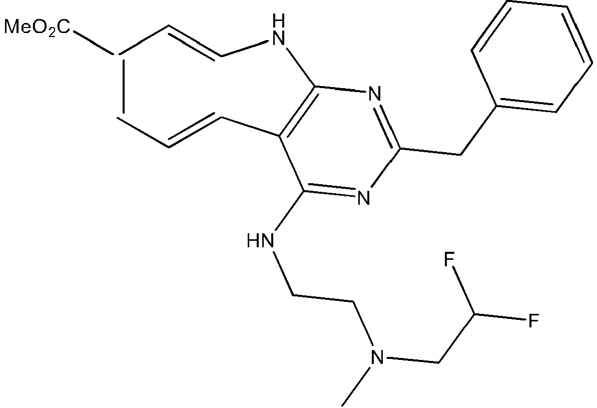 |
| 46 | 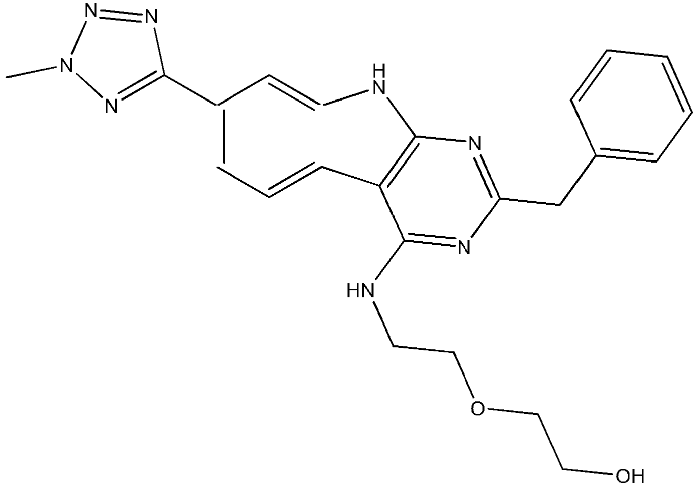 |
| 47 | 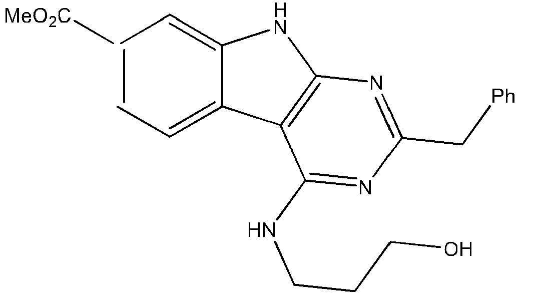 |
| 48 | 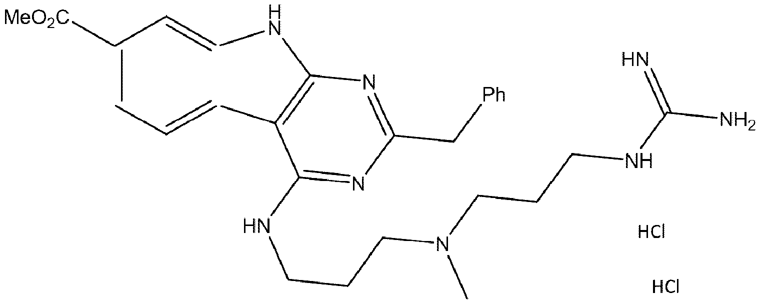 |
| 49 | 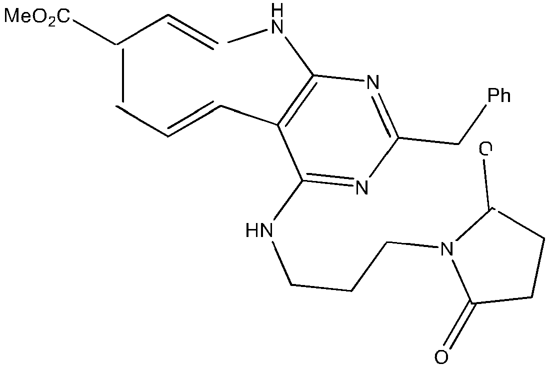 |
| 50 | 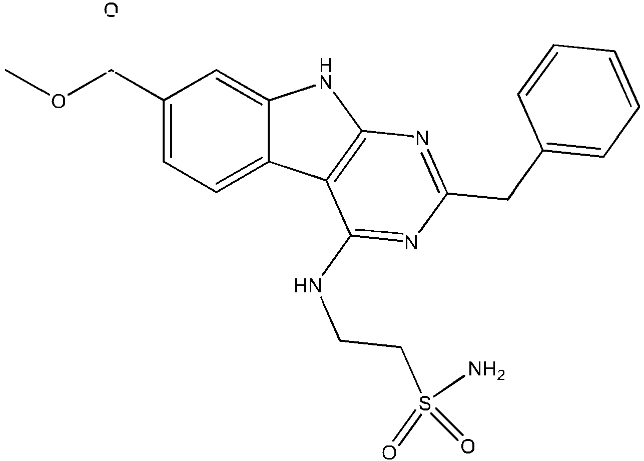 |
| 51 | 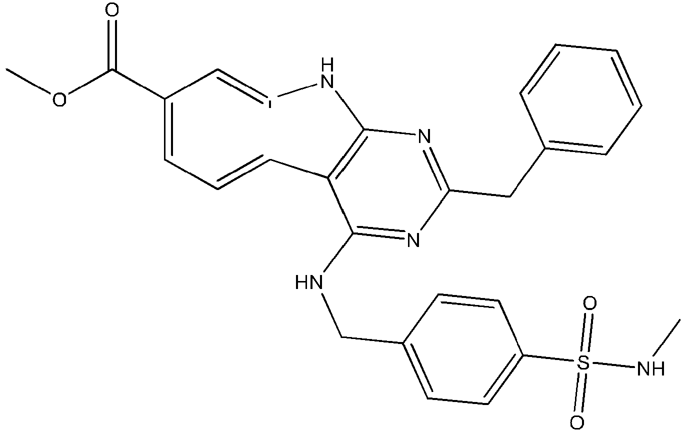 |
| 52 | 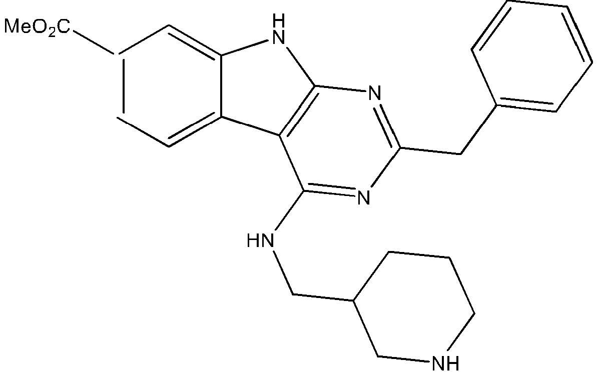 |
| 53 | 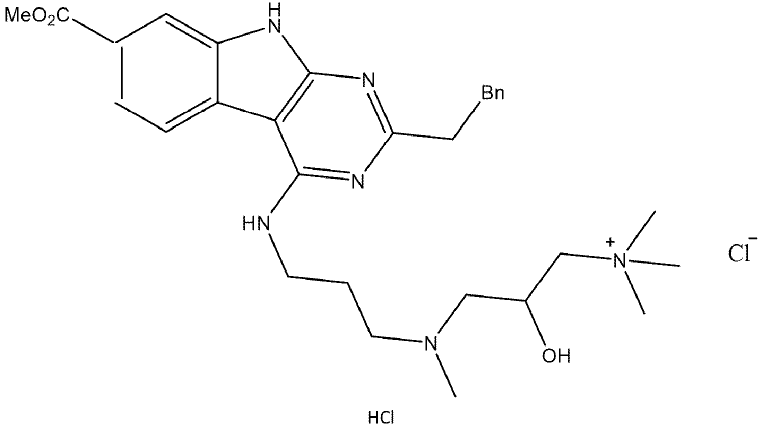 |
| 54 | 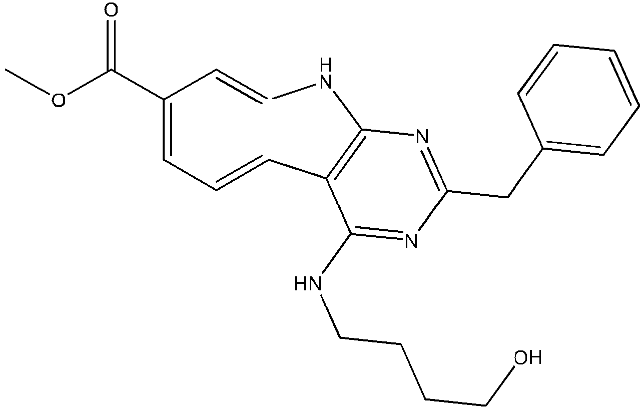 |
| 55 | 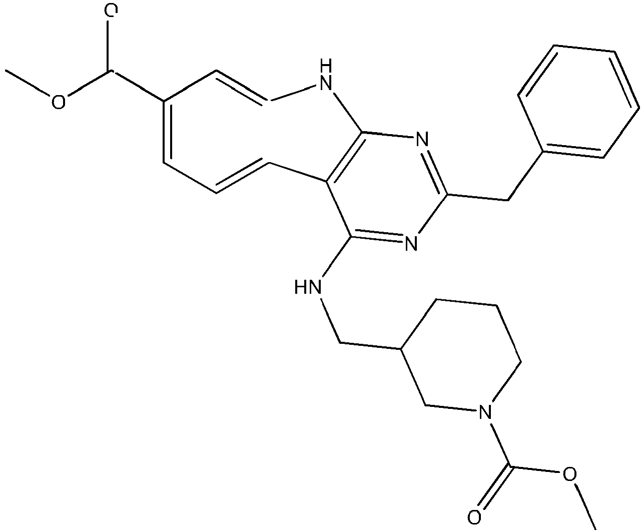 |
| 56 | 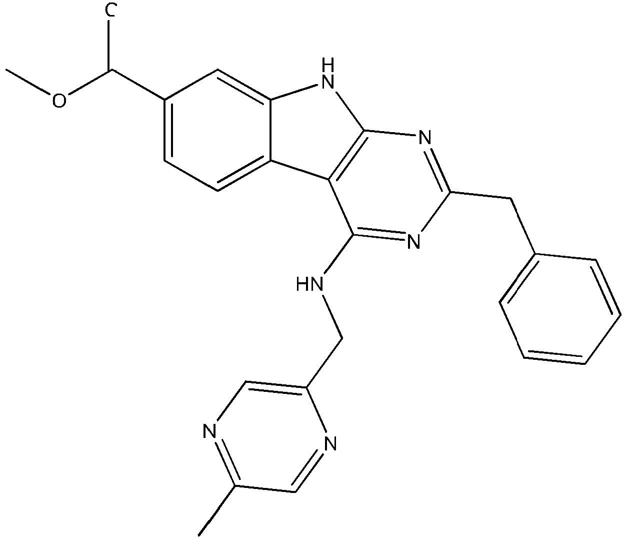 |
| 57 | 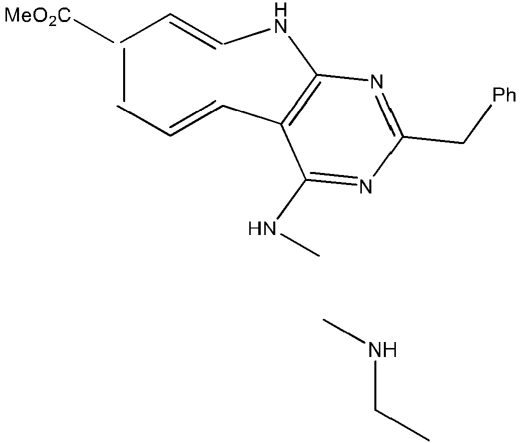 |
| 58 | 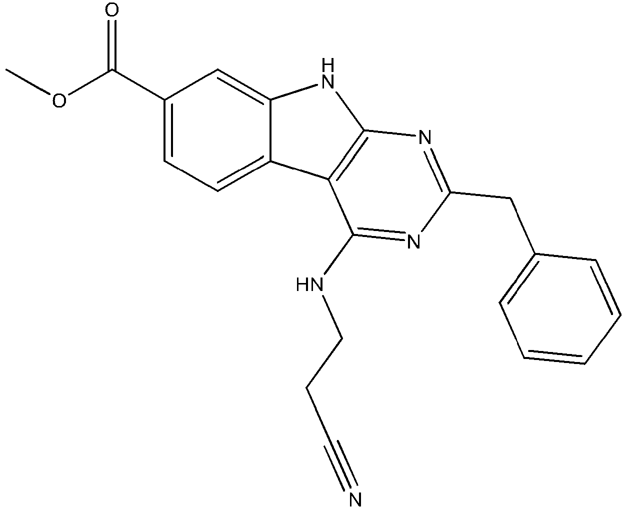 |
| 59 | 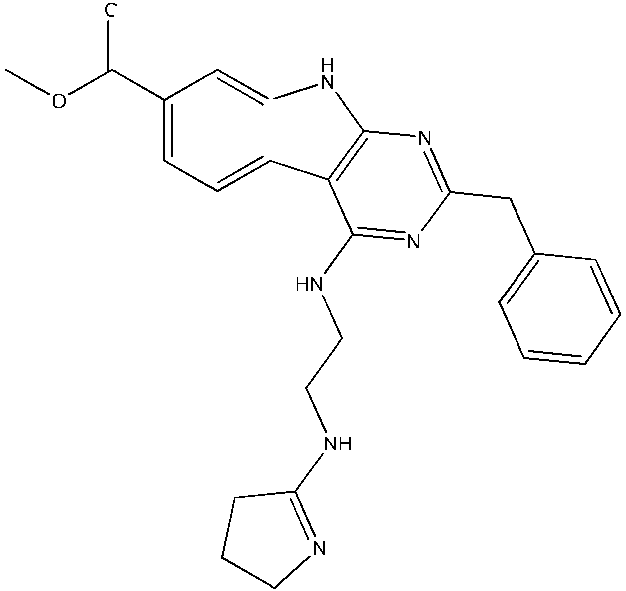 |
| 60 | 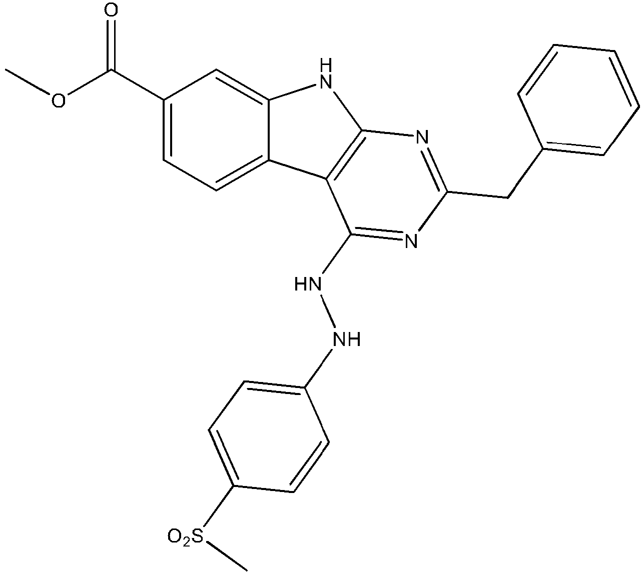 |
| 61 | 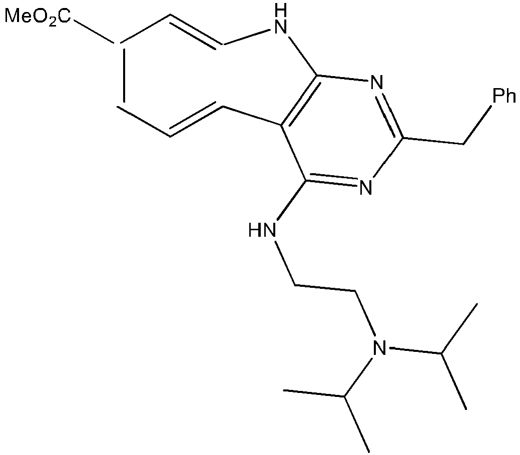 |
ou un sel pharmaceutiquement acceptable de celui-ci.

10. Greffon de cellules souches et/ou progénitrices expansées cultivées avec au moins un composé, ledit au moins un composé présentant la formule I, pour l'utilisation selon l'une quelconque des revendications 1-9, dans lequel l'état inflammatoire est contrôlé chez ledit patient.

11. Greffon de cellules souches et/ou progénitrices expansées cultivées avec au moins un composé, ledit au moins un composé présentant la formule I, pour l'utilisation selon l'une quelconque des revendications 1-10, dans lequel le greffon comprend une population de cellules dendritiques et des mastocytes,

12. Greffon de cellules souches et/ou progénitrices expansées cultivées avec au moins un composé, ledit au moins un composé présentant la formule I, pour l'utilisation selon l'une quelconque des revendications 1-11, dans lequel le greffon comprend des cellules FCER1⁺CD34⁺, des cellules CD34⁺CD45RA⁺, des cellules CD34⁺CD86⁺, des cellules CD34⁺CD45RA⁻ et des cellules CD34⁻.
